# DEMANDE DE BREVET EUROPEEN

(11) **EP 2 058 308 A1**
(43) Date de publication de la demande: **13.05.2009**
(21) Numéro de dépôt: 07291349.4
(22) Date de dépôt: 12.11.2007
(51) Int. Cl.: C07D 417/04, C07D 417/14, A61K 31/54

(54) **Dérivés de benzimidazoledihydrothiadiazinone comme inhibiteurs de fructose-1,6-biphosphatase et compositions pharmaceutiques les contenant.**

(71) Demandeur: Merck Sante, 69008 Lyon (FR)
(72) Inventeur: Botton Gérard, 78530 Buc (FR); Leriche Caroline, 75014 Paris (FR); Arbellot Annick, 91640 Fantenay les Briijs (FR); Audet Annick, 91640 Fontenay les Brlls (FR); Gleitz Johannes, 64289 Darmstadt (DE)
(74) Mandataire: Colombet, Alain André

(57) **Abrégé**

La présente invention concerne de nouveaux dérivés de benzimidazole-dihydrothiadiazinone comme inhibiteurs de fructose-1,6-biphosphatase, leurs procédés de préparation et leur utilisation en thérapie, notamment pour le traitement du diabète.

## Description

La présente invention concerne des dérivés de dihydrothiadiazinone inhibiteurs de la fructose-1,6-bisphosphatase, leur préparation et leur utilisation en thérapeutique dans le traitement de pathologies associées au syndrome d'insulinorésistance.

Le « *diabetes mellitus* » (ou diabète) est aujourd'hui l'une des maladies des plus répandues dans le monde. Les sujets atteints de diabète ont été divisés en deux classes, à savoir le type I ou *diabetes mellitus* insulinodépendant et type Il ou *diabetes mellitus* non insulinodépendant (NIDDM). Le NIDDM représente approximativement 90 % de tous les sujets diabétiques, et l'on estime qu'il affecte de 12 à 14 millions d'adultes seulement aux États-Unis (6,6 % de la population).

Le NIDDM est caractérisé à la fois par une hyperglycémie à jeun et également par des augmentations post-prandiales exagérées des taux de glucose plasmatique. Le NIDDM est associé à un grand nombre de complications à long terme, comprenant les maladies microvasculaires telles que la rétinopathie, la néphropathie et la neuropathie, ainsi que les maladies macrovasculaires telles que la cardiopathie coronaire.

De nombreuses études chez le modèle animal montrent une relation causale entre les complications à long terme et l'hyperglycémie. Les résultats récents obtenus par le Diabetes Control and Complications Trial (DCCT) et le Stockholm Prospective Study ont mis pour la première fois en évidence cette relation chez l'homme en montrant que les sujets atteints de diabète insulinodépendant présentent un risque substantiellement moins élevé de développement et de progression de ces complications lorsqu'ils sont soumis à un contrôle glycémique plus rigoureux. On s'attend également à ce qu'un contrôle plus rigoureux soit en faveur des patients NIDDM.

Les thérapies actuelles utilisées pour traiter les patients NIDDM impliquent à la fois un contrôle des facteurs de risque liés au style de vie et une intervention pharmaceutique. La thérapie première pour les patients NIDDM est habituellement un aliment de régime et des exercices strictement contrôlés puisqu'un nombre extraordinaire de patients NIDDM sont en surcharge pondérale ou obèses (≈ 67 %) et puisqu'une perte de poids peut améliorer la sécrétion d'insuline, la sensibilité à l'insuline et conduire à une normoglycémie.

La normalisation du glucose sanguin a lieu chez moins de 30 % de ces patients en raison d'une faible compatibilité et d'une faible réponse. Les patients atteints d'hyperglycémie non contrôlée seulement par un régime sont par la suite traités avec de l'insuline ou des hypoglycémiants oraux. Actuellement, les insulino-secréteurs (sulfonylurées, glinides), les biguanides (metformine) et les sensibilisateurs à l'insuline (glitazones) sont les seules classes d'agents hypoglycémiques oraux mis à la disposition des NIDDM. Le traitement par les sulfonylurées conduit à une réduction efficace du glucose sanguin chez seulement 70 % des patients et seulement 40 % après dix années de thérapie. Les patients pour lesquels le régime et les sulfonylurées sont sans effet sont traités ensuite avec des injections quotidiennes d'insuline afin d'établir un contrôle adéquat de la glycémie.

Bien que les sulfonylurées représentent une thérapie majeure pour les patients NIDDM, quatre facteurs limitent leur succès général. En premier lieu, comme indiqué ci-dessus, une grande partie de la population NIDDM ne répond pas de manière adéquate à la thérapie aux sulfonylurées (c'est-à-dire défaillance première ou « primary failures » en langue anglaise) ou devient résistante (c'est-à-dire défaillance secondaire ou « secondary failures » en langue anglaise). Ceci est particulièrement vrai chez les patients NIDDM présentant une NIDDM avancée, en raison du fait que ces patients souffrent d'une sévère défaillance de sécrétion d'insuline. En second lieu, la thérapie aux sulfonylurées est associée à un risque élevé de graves périodes d'hypoglycémie. En troisième lieu, l'hyperinsulinémie chronique est associée à une augmentation des maladies cardiovasculaires bien que l'on considère cette relation comme controversée et non prouvée. Enfin, les sulfonylurées sont associées à un gain de poids, ce qui conduit à une aggravation de la sensibilité périphérique à l'insuline et par conséquent peut accélérer la progression de la maladie.

De récents résultats du U.K. Diabetes Prospective Study montrent également que les patients soumis à une thérapie maximale d'une sulfonylurée, de metformine ou d'une combinaison des deux, étaient incapables de maintenir une glycémie normale à jeun pendant la période des six années de l'étude U.K. Prospective Diabetes Study, 16. Diabetes, 44, 1249-158 (1995). Ces résultats illustrent en outre le grand besoin de thérapies alternatives. Trois stratégies thérapeutiques, qui pourraient fournir des bénéfices supplémentaires sur le plan de la santé de patients NIDDM au-delà des thérapies disponibles actuellement, comprennent des médicaments qui pourraient : (i) éviter l'apparition du NIDDM ; (ii) éviter les complications diabétiques en bloquant les événements nuisibles occasionnés par l'hyperglycémie chronique ; ou (iii) normaliser les niveaux de glucose ou au moins diminuer les niveaux de glucose en dessous du seuil déclaré pour les maladies microvasculaires et macrovasculaires.

L'hyperglycémie chez les NIDDM est associée à deux anomalies biochimiques, à savoir une résistance à l'insuline et une insuffisance de sécrétion d'insuline. Les rôles relatifs de ces anomalies métaboliques dans la pathogenèse des NIDDM ont fait l'objet de nombreuses études durant les dernières décennies. Des études réalisées parmi la descendance et la fratrie de patients NIDDM, parmi les jumeaux mono- et di-zygotes et les populations ethniques présentant une grande incidence sur le NIDDM (par exemple les indiens Pima), supportent très fortement la nature héréditaire de la maladie.

En dépit de la présence de résistance à l'insuline et d'une défaillance de sécrétion d'insuline, les niveaux de glucose à jeun (FBG, « Fasted Blood Glucose Levels » en langue anglaise) restent normaux chez les patients pré-diabétiques en raison d'un état d'hyperinsulinémie compensatrice. Cependant, la sécrétion d'insuline est finalement inadéquate et conduit à une hyperglycémie à jeun. Avec le temps, les niveaux d'insuline décroissent. La progression de la maladie est caractérisée par des niveaux croissants de FBG et des niveaux d'insuline en baisse.

De nombreuses études cliniques ont tenté de définir le défaut primaire mis en jeu lors de l'augmentation progressive des FBG. Les résultats de ces études montrent qu'une production excessive de glucose hépatique (HGO) est la première raison de l'élévation des FBG, avec une corrélation significative trouvée pour HGO et FBG, dès lors que les FBG dépassent 140 mg/dL (Kolterman et coll., J. Clin. Invest., 68, 957, (1981) ; DeFronzo, Diabetes, 37, 667, (1988)).

Le HGO comprend du glucose dérivé de la dégradation du glycogène hépatique (glycogénolyse) et du glucose synthétisé à partir de précurseurs carbone-3 (gluconéogenèse). Un grand nombre d'études radio-isotopiques, ainsi que plusieurs études utilisant la spectroscopie ¹³C-NMR montrent que la gluconéogenèse contribue pour 50% à 100 % du glucose produit par le foie dans l'état de post-absorption et que le flux de gluconéogenèse se trouve en excès (2 à 3 fois) chez les patients NIDDM (Magnusson et coll., J. Clin. Invest., 90, 1323-1327, (1992*) ;* Rothmann et coll., Science, 254, 573-76, (1991); Consoli et coll., Diabetes, 38, 550-557, (1989)).

La gluconéogenèse à partir du pyruvate est une voie biosynthétique hautement régulée qui requiert onze enzymes. Sept enzymes catalysent des réactions réversibles et sont communes à la fois à la gluconéogenèse et à la glycolyse. Quatre enzymes catalysent des réactions spécifiques à la gluconéogenèse, à savoir la pyruvate carboxylase, la phosphoénolpyruvate carboxykinase, la fructose-1,6-bisphosphatase et la glucose-6-phosphatase. Le flux total est contrôlé tout au long de la voie biosynthétique par les activités spécifiques de ces enzymes, les enzymes qui catalysent les étapes correspondantes dans la direction glycolytique, et par la disponibilité du substrat. Les facteurs alimentaires (glucose, graisses) et des hormones (insuline, glucagon, glucocorticoïdes, épinéphrine) régulent de manière coordonnée les activités enzymatiques dans les processus de gluconéogenèse et de glycolyse au moyen d'expression de gènes et de mécanismes post-translationnels.

Parmi les quatre enzymes spécifiques à la gluconéogenèse, la fructose-1,6-bisphosphatase (notée par la suite « FBPase ») est une cible très appropriée pour un inhibiteur de gluconéogenèse du point de vue de l'efficacité et de la sécurité. Des études montrent que la nature utilise le cycle FBPase/PFK en tant que point de contrôle principal (interrupteur métabolique) permettant de déterminer si le flux métabolique se déroule dans la direction de la glycolyse ou dans la direction de la gluconéogenèse (Claus et coll., Mechanisms of Insulin Action, Belfrage, P. Editor, pp. 305-321, Elsevier Science, (1992) ; Regen et coll., J. Theor. Bio., 635-658, (1984) ; Pilkis et coll., Annu. Rev. Biochem., 57, 755-783, (1988)). La FBPase est inhibée par le fructose-2,6-bisphosphate dans la cellule. Le fructose-2,6-bisphosphate se lie au site de substrat de l'enzyme. L'AMP se lie à un site allostérique sur l'enzyme.

On a également présenté des inhibiteurs synthétiques de la FBPase. Maryanoff a signalé que des analogues de fructose-2,6-bisphosphate inhibent la FBPase en se liant au substrat (J. Med. Chem., 106, 7851, (1984) ; Brevet U.S. n° 4 968 790. Cependant, ces composés présentent une activité relativement faible et n'inhibent pas la production de glucose dans les hépatocytes, sans doute en raison d'une faible pénétration cellulaire.

On a signalé de nombreux inhibiteurs de fructose-1,6-bisphosphatase utiles dans le traitement du diabète :
- Gruber indique que certains nucléosides peuvent réduire le glucose sanguin au niveau de l'animal tout entier par inhibition de la FBPase (EP 0 427 799 B1) ; ces composés exercent leur activité tout d'abord en opérant une phosphorylation du monophosphate correspondant ;
- Gruber et coll. (brevet U.S. n° 5 658 889) ont décrit l'utilisation d'inhibiteurs du site de l'AMP de la FBPase pour traiter le diabète ;
- Dan et coll. (WO 98/39344, WO 00/014095) ont décrit de nouvelles purines et des composés hétéroaromatiques en tant qu'inhibiteurs de la FBPase ;
- Kasibhatla et coll. (WO 98/39343) ont décrit de nouveaux benzimidazolyl-phosphonates comme inhibiteurs de la FBPase ;
- Reddy et coll. (WO 98/39342) ont décrit de nouveaux indoles et aza-indoles comme inhibiteurs de la FBPase ;
- Jaing et coll. (WO 01/047935) ont décrit des bisamidate-phosphonates en tant qu'inhibiteurs de la FBPase pour traiter le diabète;
- Bookser et coll. (WO 01/066553) ont décrit des phosphates d'hétérocycles en tant qu'inhibiteurs spécifiques de la FBPase pour traiter le diabète.
- Bauer et coll. (US2003/0144308) ont décrit des composés quinazoline en tant qu'inhibiteurs de la FBPase pour traiter le diabète et ses complications.
- Tsukuda et coll. (WO2006104030) ont décrit des composés contenant un groupement thiazole en tant qu'inhibiteurs de la FBPase pour traiter le diabète, l'obésité, l'hyperlipidémie, l'hypertension, l'artériosclérose.
- Dang et coll. (WO2006023515) ont décrit des derivés thiazoles en tant qu'inhibiteurs de la FBPase pour traiter le diabète, l'obésité.

La présente invention concerne des nouveaux dérivés benzimidazoledihydrothiadiazinones comme inhibiteurs de la fructose-1,6-bisphosphatase utilisables dans le traitement du diabète et des pathologies associées.

Plus particulièrement, l'invention se rapporte à des dérivés de benzimidazoledihydrothiadiazinones, utiles dans le traitement de pathologies associées au syndrome d'insulinorésistance.

Les composés de l'invention sont de formule générale (I) suivante : 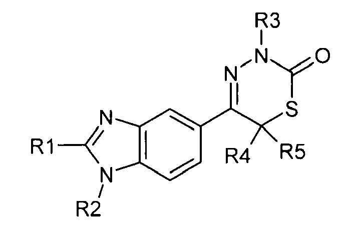
R1 représente un groupe choisi indifférement parmi :
   - Alkyle-, alcoxyalkyle-, alcényle-, alcynyle-, chacun de ces groupes pouvant être éventuellement substitué par un ou plusieurs groupes choisis parmi Y,
   - Aryle-, arylalkyle-, aryloxyalkyle-, arylalkyloxyalkyle-, arylthioalkyle-, arylalkylthioalkyle-,
   - Hétéroaryle-, hétéroarylalkyle-, hétéroaryloxyalkyle-, hétéroarylalkyloxyalkyle-, hétéroarylthioalkyle-, hétéroarylalkylthioalkyle-,
   - Cycloalkyle-, cycloalkylalkyle-, cycloalkyloxyalkyle-, cycloalkylalkyloxyalkyle-, cycloalkylthioalkyle-, cycloalkylalkylthioalkyle-,
   - Hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
   chacun des groupes aryle-, hétéroaryle-, cycloalkyle-, hétérocycloalkyle- pouvant être éventuellement substitué par un ou plusieurs groupes choisis indifféremment parmi Y.
R1 peut également représenter un groupe A-B- constitué de deux cycles A et B reliés par une liaison et dans lequel A et B représentent indépendamment un groupe choisi indifférement parmi :
   - Aryle-, hétéroaryle-, cycloalkyle-, hétérocycloalkyle-,
   chacun des groupes aryle-, hétéroaryle-, cycloalkyle-, hétérocycloalkyle- pouvant être éventuellement substitués par un ou plusieurs groupes choisis indifférement parmi Y.
R2 représente un groupe choisi indifféremment parmi :
   - H,
   - Alkyle-, alcoxyalkyle-, alcényle-, alcynyle-, chacun de ces groupes pouvant être éventuellement substitué par un ou plusieurs groupes choisis parmi Y,
   - Aryle-, arylalkyle-, aryloxyalkyle-, arylalkyloxyalkyle-, arylthioalkyle-, arylalkylthioalkyle-,
   - Hétéroaryle-, hétéroarylalkyle-, hétéroaryloxyalkyle-, hétéroarylalkyloxyalkyle-, hétéroarylthioalkyle-, hétéroarylalkylthioalkyle-,
   - Cycloalkyle-, cycloalkylalkyle-, cycloalkyloxyalkyle-, cycloalkylalkyloxyalkyle-, cycloalkylthioalkyle-, cycloalkylalkylthioalkyle-,
   - Hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
   chacun des groupes aryle-, hétéroaryle-, cycloalkyle-, hétérocycloalkyle- pouvant être éventuellement substitués par un ou plusieurs groupes choisis indifféremment parmi Y.
R3 représente un groupe choisi indifféremment parmi :
   - H,
   - Alkyle-, alcoxyalkyle-, hydroxyalkyl-, alcényle-, alcynyle-, chacun de ces groupes pouvant être éventuellement substitué par un ou plusieurs groupes choisis parmi
   - Y,
   - Aryle-, arylalkyle-, aryloxyalkyle-, arylalkyloxyalkyle-, arylthioalkyle-, arylalkylthioalkyle-,
   - Hétéroaryle-, hétéroarylalkyle-, hétéroaryloxyalkyle-, hétéroarylalkyloxyalkyle-, hétéroarylthioalkyle-, hétéroarylalkylthioalkyle-,
   - Cycloalkyle-, cycloalkylalkyle-, cycloalkyloxyalkyle-, cycloalkylalkyloxyalkyle-, cycloalkylthioalkyle-, cycloalkylalkylthioalkyle-,
   - Hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
   chacun des groupes aryle-, hétéroaryle-, cycloalkyle-, hétérocycloalkyle- pouvant être éventuellement substitués par un ou plusieurs groupes choisis indifférement parmi Y.
R4 et R5 indépendamment représentent un groupe choisi indifférement parmi:
   - H,
   - W;
Y représente un groupe choisi indifféremment parmi :
   Hydroxy- ; thio- ; halogène- ; trifluorométhoxy- ; trifluorométhyle- ; alkyloxy- ; carboxyle- ; alcoxycarbonyle- ; carbamoyle- ; sulfamoyle- ; nitro- ; guanidino- ; amidino-; aryle- ; hétéroaryle- ; amino- ; 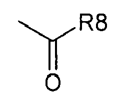
   dans lequel R8 représente un groupe choisi indifféremment parmi W; 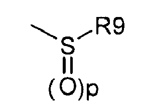 dans lequel R9 représente un groupe choisi indifférement parmi W et p est choisi parmi 0, 1 ou 2 ;
   - (CH₂)n-O-R10
   dans lequel R10 représente un groupe choisi indifférement parmi
   - H,
   - W,
   et n est un entier compris entre 0 à 8 ; 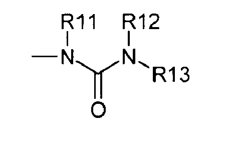 dans lequel R11, R12, R13 représentent indépendamment un groupe choisi indifféremment parmi :
   - H,
   - W,
   étant entendu que R12 et R13 peuvent former un système mono- ou bicyclique saturé ou non de 3 à 10 atomes comportant de 1 à 3 hétéroatomes, par exemple et de façon non exhaustive, une piperidine, une morpholine, une piperazine substituée ou non, une pyrrolidine ; 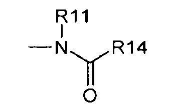 dans lequel R11 représente un groupe choisi indifféremment parmi :
   - H,
   - W,
   et dans lequel R14 représente un groupe choisi indifféremment parmi W ; 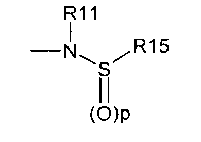 dans lequel R11 représente un groupe choisi indifféremment parmi :
   - H,
   - W,
   et dans lequel R15 représente un groupe choisi indifféremment parmi W et p est choisi parmi 0, 1 ou 2 ;
   où :
   Amino désigne un groupe 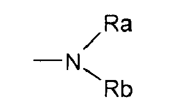
   dans lequel Ra et Rb sont choisis indifféremment parmi
   - H,
   - W,
   étant entendu que Ra et Rb peuvent former un système mono- ou bicyclique saturé ou non de 3 à 10 atomes comportant de 1 à 3 hétéroatomes, par exemple et de façon non exhaustive, une pipéridine, une morpholine, une pipérazine substituée ou non, une pyrrolidine.
W représente un groupe choisi indifféremment parmi :
   - Alkyle-, alcényle-, alcynyle-, aryle-, arylalkyle-, aryloxyalkyle-, arylalkyloxyalkyle-, arylthioalkyle-, arylalkylthioalkyle-, hétéroaryle-, hétéroarylalkyle-, hétéroaryloxyalkyle-, hétéroarylalkyloxyalkyle-, hétéroarylthioalkyle-, hétéroarylalkylthioalkyle-, cycloalkyle-, cycloalkylalkyle-, cycloalkyloxyalkyle-, cycloalkylalkyloxyalkyle-, cycloalkylthioalkyle-, cycloalkylalkylthioalkyle-, hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
   chacun de ces groupes pouvant être éventuellement substitué par un ou plusieurs groupes choisis indifférement parmi
   hydroxy-, thio-, halogène-, trifluorométhoxy-, trifluorométhyle-, alkyloxy-, carboxyle-, alcoxycarbonyle-, carbamoyle-, sulfamoyle-, nitro-, guanidino-, amidino-, aryle-, hétéroaryle-, amino- qui a la même signification que ci dessus, acétyle- ;
   sous forme de tautomères, énantiomères, diastéréoisomères, épimères, ainsi que les sels pharmaceutiquement acceptables, et les formes cristallines, ou leurs mélanges.

La présente invention concerne également les modes de réalisation particuliers de la formule générale (I) ci-dessous pris isolément ainsi que chacune de leurs combinaisons :
Dans la formule générale (I) présentée ci-dessus :
   - R1 représente un groupe aryle éventuellement substitué par un à quatre groupes identiques ou différents indépendamment choisis parmi halogène, -OR, perhalogénoalkyle-, -S(O)ₚ-R, -NRR', -S-CH₂-CN ou hétéroaryle; ou un groupe -hétéroaryle, éventuellement substitué par un ou plusieurs groupes -alkyle ; et/ou
   - R4 et R5, identiques ou différents, sont indépendamment choisis parmi un atome d'hydrogène et un groupe -alkyle.

De préférence, la présente invention concerne les composés de formule générale (I) représentée ci-dessus dans laquelle :
R1 représente un groupe aryle éventuellement substitué par un à quatre groupes identiques ou différents indépendamment choisis parmi halogène, -OR, perhalogénoalkyle-, -S(O)ₚ-R, -NRR', -S-CH₂-CN ou hétéroaryle; ou un groupe -hétéroaryle, éventuellement substitué par un ou plusieurs groupes -alkyle ;
R2 représente un atome d'hydrogène ; un groupe -cycloalkyle ; un groupe -alkyle éventuellement substitué par un groupe -cycloalkyle ou -aryle ;
R4 et R5, identiques ou différents, sont indépendamment choisis parmi un atome d'hydrogène et un groupe -alkyle ;
R3 représente :
   - un atome d'hydrogène ;
   - un groupe -alkyle, éventuellement substitué par un ou plusieurs groupes choisis parmi:
      -OR, -OHétérocycloalkyle,
      -cycloalkyle,
      -hétérocycloalkyle,
      -COOR,
      -CONRR',
      -NRR',
      -NRCOalkyle, -NRCOalkyle-aryle, -NRCO-cycloalkyle, -NRCOaryle, -NRCOhétéroaryle, le groupe aryle étant éventuellement substitué par un atome d'halogène ou un groupe alkyle,
      -NRCOOAlkyle,
      -NRCO-NR-Alkyle, -NRCO-NR-Aryle ou -NRCO-NR-Alkyle-aryle, où le groupe aryle est éventuellement substitué par un atome d'halogène ou un groupe -COR;
      -NRCO-NR-Cycloalkyle,
      -NRS(O)ₚ-aryle, -NRS(O)ₚ-alkyle,
      - Aryle ou -OAryle, chaque groupe aryle étant éventuellement substitué par un ou plusieurs substituants choisis parmi les atomes d'halogène et les groupes -alkyle, -OR, perhalogénoalkyle-, perhalogénoalkyloxy-, -S(O)ₚ-R ;
p=0, 1 ou 2 ;
R, R', identiques ou différents, sont indépendamment choisis parmi H, -alkyle, -aryle, -hétéroaryle, -cycloalkyle, -hétérocycloalkyle, étant entendu que R et R' peuvent former ensemble un système mono- ou bicyclique saturé ou non de 3 à 10 atomes comportant de 1 à 3 hétéroatomes, par exemple et de façon non exhaustive, une pipéridine, une morpholine, une pipérazine substituée ou non, une pyrrolidine ;
sous forme de tautomères, énantiomères, diastéréoisomères, épimères, ainsi que les sels pharmaceutiquement acceptables, et les formes cristallines, ou leurs mélanges.

De façon toute préférentielle, la présente invention concerne les composés de formule générale (I) représentée ci-dessus dans laquelle :
R1 représente un groupe aryle éventuellement substitué par un à quatre groupes identiques ou différents indépendamment choisis parmi halogène, -Oalkyle, -S(O)-alkyle ;
R2 représente un atome d'hydrogène ; un groupe -cycloalkyle ; un groupe -alkyle;
R4 et R5, identiques ou différents, sont indépendamment choisis parmi un atome d'hydrogène et un groupe -alkyle ;
R3 représente :
   - un atome d'hydrogène ;
   - un groupe -alkyle,
   sous forme de tautomères, énantiomères, diastéréoisomères, épimères, ainsi que les sels pharmaceutiquement acceptables, et les formes cristallines, ou leurs mélanges.

L'invention se rapporte également aux formes tautomères, aux énantiomères, diastéréoisomères, épimères et aux sels organiques ou minéraux des composés de formule générale (I), ainsi qu'aux formes cristallines, y compris polymorphismes, de ceux-ci et des composés de formule (I).

L'invention englobe aussi bien les isomères et/ou diastéréoisomères, sous formes pures ou en mélange en toutes proportions de deux ou plusieurs d'entre eux, y compris les mélanges racémiques.

Les composés de formule (I) tel que définis précédemment possédant une fonction suffisamment acide ou une fonction suffisamment basique ou les deux, peuvent inclure les sels correspondants d'acide organique ou minéral et/ou de base organique ou minérale, acceptables du point de vue pharmaceutique.

Les sels d'acide sont par exemple les chlorhydrates, les bromhydrates, les sulfates, les hydrogénosulfates, les dihydrogénophosphates, les citrates, les maléates, les fumarates, les trifluoroacétates, les 2-naphtalènesulfonates et les *para*-toluènesulfonates.

Les bases utilisables pour la formation de sels de composés de formule (I) sont des bases organiques ou minérales. Les sels résultants sont par exemple les sels formés avec des métaux et notamment des métaux alcalins, alcalino-terreux et de transition (tels que le sodium, le potassium, le calcium, le magnésium, l'aluminium) ou avec des bases comme l'ammoniac ou des amines secondaires ou tertiaires (telles que la diéthylamine, la triéthylamine, la pipéridine, la pipérazine, la morpholine) ou avec des acides aminés basiques, ou avec des osamines (telles que la méglumine) ou avec des aminoalcools (tels que le 3-aminobutanol et le 2-aminoéthanol).

L'invention se rapporte également aux sels chiraux utilisés pour la séparation des racémates.

À titre d'exemple, les acides chiraux suivants sont utilisés : acide (+)-D-di-O-benzoyltartrique, acide (-)-L-di-O-benzoyltartrique, acide (-)-L-di-O,O'-p-toluyl-L-tartrique, acide (+)-D-di-O,O'-p-toluyl-L-tartrique, acide (*R*)-(+)-malique, acide (*S*)-(-)-malique, acide (+)-camphanique, acide (-)-camphanique, acide *R*-(-)-1,1'-binaphtalén-2,2'-diyl-hydrogénophosphonique, acide (+)-camphorique, acide (-)-camphorique, acide (*S*)-(+)-2-phénylpropionique, acide (*R*)-(+)-2-phényl-propionique , acide D-(-)-mandélique, acide L-(+)-mandélique, acide D-tartrique, acide L-tartrique, ou l'un de leurs mélanges de deux ou plusieurs d'entre eux.

On peut également éventuellement utiliser des amines chirales, et par exemple la quinine, la brucine, la (S)-1-(benzyloxyméthyl)propylamine (III), la (-)-éphédrine, la (4*S*,5*R*)-(+)-1,2,2,3,4-tétraméthyl-5-phényl-1,3-oxazolidine, la (*R*)-1-phényl-2-*p*-tolyléthylamine, le (*S*)-phénylglycinol, la (-)-N-méthyléphédrine, le (+)-(2*S*,3*R*)-4-diméthylamino-3-méthyl-1,2-diphényl-2-butanol, le (*S*)-phényl-glycinol, la (*S*)-α-méthyl-benzylamine ou l'un de leurs mélanges de deux ou plusieurs d'entres elles.

Les composés de formule (I) ci-dessus comprennent également les pro-drogues de ces composés.

Par « pro-drogues », on entend des composés qui, une fois administrés chez le patient, sont transformés chimiquement et/ou biologiquement dans l'organisme vivant, en composés de formule (I).

A titre de composés selon l'invention, on peut notamment citer :
- 5-[2-(4-méthoxyphényl)-1*H*-benzimidazol-5-yl]-3,6-dihydro-2*H*-1,3,4-thiadiazin-2-one,
- 6-méthyl-5-[2-(1*H*-pyrazol-3-yl)-1*H*-benzimidazol-5-yl]-3,6-dihydro-2*H*-1,3,4-thiadiazin-2-one,
- 6-méthyl-5-[2-(2-thienyl)-1*H*-benzimidazol-5-yl]-3,6-dihydro-2*H*-1,3,4-thiadiazin-2-one,
- 5-[2-(3-méthoxyphényl)-1*H*-benzimidazol-5-yl]-6-méthyl-3,6-dihydro-2*H*-1,3,4-thiadiazin-2-one,
- 6-méthyl-5-(2-pyridin-4-yl-1*H*-benzimidazol-5-yl)-3,6-dihydro-2*H*-1,3,4-thiadiazin-2-one,
- 5-{2-[3-méthoxy-4-(méthylthio)phényl]-1*H*-benzimidazol-5-yl}-6-méthyl-3,6-dihydro-2*H*-1,3,4-thiadiazin-2-one,
- 5-{2-[2-méthoxy-4-(méthylthio)phényl]-1*H*-benzimidazol-5-yl}-6-méthyl-3,6-dihydro-2*H*-1,3,4-thiadiazin-2-one,
- 6-méthyl-5-[2-(1,3-thiazol-4-yl)-1*H*-benzimidazol-5-yl]-3,6-dihydro-2*H*-1,3,4-thiadiazin-2-one,
- 5-{2-[3-méthoxy-4-(méthylsulfinyl)phényl]-1*H*-benzimidazol-5-yl}-6-méthyl-3,6-dihydro-2*H*-1,3,4-thiadiazin-2-one,
- 5-[2-(1*H*-imidazol-1-yl)-1*H*-benzimidazol-5-yl]-6-méthyl-3,6-dihydro-2*H*-1,3,4-thiadiazin-2-one,
- 5-{2-[4-(diméthylamino)phényl]-1*H*-benzimidazol-5-yl}-6-méthyl-3,6-dihydro-2*H*-1,3,4-thiadiazin-2-one,
- 6-méthyl-5-{2-[4-(méthylthio)phényl]-1*H*-benzimidazol-5-yl}-3,6-dihydro-2*H-*1,3,4-thiadiazin-2-one,
- 6-méthyl-5-{2-[4-(méthylsulfinyl)phényl]-1*H*-benzimidazol-5-yl}-3,6-dihydro-2*H-*1,3,4-thiadiazin-2-one,
- 5-[2-(2-méthoxyphényl)-1*H*-benzimidazol-5-yl]-6-méthyl-3,6-dihydro-2*H*-1,3,4-thiadiazin-2-one,
- 5-[2-(3-méthoxyphényl)-1*H*-benzimidazol-5-yl]-6-méthyl-3,6-dihydro-2*H*-1,3,4-thiadiazin-2-one,
- 3-méthoxy-4-[5-(6-méthyl-2-oxo-3,6-dihydro-2*H*-1,3,4-thiadiazin-5-yl)-1*H-*benzimidazol-2-yl]phényl thiocyanate,
-5-{2-[2-chloro-4-(méthylthio)phényl]-1*H*-benzimidazol-5-yl}-6-méthyl-3,6-dihydro-2*H*-1,3,4-thiadiazin-2-one,
- 6-méthyl-5-{2-[4-(pyridin-4-ylamino)phényl]-1*H*-benzimidazol-5-yl}-3,6-dihydro-2*H*-1,3,4-thiadiazin-2-one,
- 5-[2-(4-méthoxyphényl)-1*H*-benzimidazol-5-yl]-3,6-diméthyl-3,6-dihydro-2*H-*1,3,4-thiadiazin-2-one,
- 3,6-diméthyl-5-[2-(1*H*-pyrazol-3-yl)-1*H*-benzimidazol-5-yl]-3,6-dihydro-2*H-*1,3,4-thiadiazin-2-one,
- 3,6-diméthyl-5-[2-(1,3-thiazol-4-yl)-1*H*-benzimidazol-5-yl]-3,6-dihydro-2*H-*1,3,4-thiadiazin-2-one,
- 3,6-diméthyl-5-(2-pyridin-4-yl-1*H*-benzimidazol-5-yl)-3,6-dihydro-2*H*-1,3,4-thiadiazin-2-one,
- 3-éthyl-6-méthyl-5-[2-(1*H*-pyrazol-3-yl)-1*H*-benzimidazol-5-yl]-3,6-dihydro-2*H-*1,3,4-thiadiazin-2-one,
- 3-éthyl-6-méthyl-5-[2-(1,3-thiazol-4-yl)-1*H*-benzimidazol-5-yl]-3,6-dihydro-2*H-*1,3,4-thiadiazin-2-one,
- 3-éthyl-5-[2-(4-méthoxyphényl)-1*H*-benzimidazol-5-yl]-6-méthyl-3,6-dihydro-2*H*-1,3,4-thiadiazin-2-one,
- 5-[1-Cycloheptyl-2-(3-méthoxy-phényl)-1*H*-benzimidazol-5-yl]-3,6-dihydro-2*H-*1,3,4-thiadiazin-2-one,
- 5-[1-Isobutyl-2-(3-méthoxy-phényl)-1*H*-benzimidazol-5-yl]-3,6-dihydro-2*H-*1,3,4-thiadiazin-2-one,
- 5-[1-Isobutyl-2-(3-méthoxy-phényl)-1*H*-benzimidazol-5-yl]-3-(4-méthyl-benzyl)]-3,6-dihydro-2*H*-1,3,4-thiadiazin-2-one,
- 3-(4-tert-Butyl-benzyl)-5-[1-isobutyl-2-(3-méthoxy-phényl)-1 H-benzimidazol-5-yl]-3,6-dihydro-2*H*-1,3,4-thiadiazin-2-one,
- 3-(2-Fluoro-benzyl)-5-[1-isobutyl-2-(3-méthoxy-phényl)-1H-benzimidazol-5-yl]-3,6-dihydro-2*H*-1,3,4-thiadiazin-2-one,
- 5-[1-Cycloheptyl-2-(3-méthoxy-phényl)-1*H*-benzimidazol-5-yl]-3-méthyl]-3,6-dihydro-2*H*-1,3,4-thiadiazin-2-one,
- 5-[1-Cycloheptyl-2-(3-méthoxy-phényl)-1*H*-benzimidazol-5-yl]-3-propyl]-3,6-dihydro-2*H*-1,3,4-thiadiazin-2-one,
- 5-[1-Cycloheptyl-2-(3-méthoxy-phényl)-1*H*-benzimidazol-5-yl]-3-isobutyl]-3,6-dihydro-2*H*-1,3,4-thiadiazin-2-one,
- 5-[1-Cycloheptyl-2-(3-méthoxy-phényl)-1*H*-benzimidazol-5-yl]-3-(2-méthoxy-éthyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-méthyl-benzyl)]-3,6-dihydro-2*H*-1,3,4-thiadiazin-2-one,
- 3-(3-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-Benzyl-5-[1-isobutyl-2-(3-méthoxy-phényl)-1*H*-benzimidazol-5-yl]-3,6-dihydro-2*H*-1,3,4-thiadiazin-2-one,
- 3-(4-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3-Chloro-benzyl)-5-[1-isobutyl-2-(3-méthoxy-phényl)-1*H*-benzimidazol-5-yl]-3,6-dihydro-2*H*-1,3,4-thiadiazin-2-one,
- 5-[1-Isobutyl-2-(3-méthoxy-phényl)-1*H*-benzimidazol-5-yl]-3-(3-méthoxy-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(4-trifluorométhoxy-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-trifluorométhyl-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(4-trifluorométhyl-benzyl)]-3,6-dihydro-2*H*-1,3,4-thiadiazin-2-one,
- 3-(2,3-Difluoro-benzyl)-5-[1-isobutyl-2-(3-méthoxy-phényl)-1H-benzimidazol-5-yl]-3,6-dihydro-2*H*-1,3,4-thiadiazin-2-one,
- 3-(2,4-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,5-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,6-Difluoro-benzyl)-5-[1-isobutyl-2-(3-méthoxy-phényl)-1H-benzimidazol-5-yl]-3,6-dihydro-2*H*-1,3,4-thiadiazin-2-one,
- 3-(3,4-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3,5-Diméthoxy-benzyl)-5-[1-isobutyl-2-(3-méthoxy-phényl)-1*H-*benzimidazol-5-yl]-3,6-dihydro-2*H*-1,3,4-thiadiazin-2-one,
- 3-Cyclopropylméthyl-5-[1-isobutyl-2-(3-méthoxy-phényl)-1*H*-benzimidazol-5-yl]-3,6-dihydro-2*H*-1,3,4-thiadiazin-2-one,
- 3-(2-Hydroxy-éthyl)-5-[1-isobutyl-2-(3-méthoxy-phényl)-1*H*-benzimidazol-5-yl]-3,6-dihydro-2*H*-1,3,4-thiadiazin-2-one,
- 3-Éthyl-5-[1-isobutyl-2-(3-méthoxy-phényl)-1*H*-benzimidazol-5-yl]-3,6-dihydro-2*H*-1,3,4-thiadiazin-2-one,
- 5-[1-Isobutyl-2-(3-méthoxy-phényl)-1*H*-benzimidazol-5-yl]-3-propyl]-3,6-dihydro-2*H*-1,3,4-thiadiazin-2-one,
- 5-[1-Isobutyl-2-(3-méthoxy-phényl)-1*H*-benzimidazol-5-yl]-3-isopropyl]-3,6-dihydro-2*H*-1,3,4-thiadiazin-2-one,
- 2-{5-[1-Isobutyl-2-(3-méthoxy-phényl)-1*H*-benzimidazol-5-yl]-2-oxo-6H-[1,3,4]thiadiazin-3-yl}-acetamide,
- 5-[1-Isobutyl-2-(3-méthoxy-phényl)-1*H*-benzimidazol-5-yl]-3-(2-méthoxy-éthyl)]-3,6-dihydro-2*H*-1,3,4-thiadiazin-2-one,
- {5-[1-Isobutyl-2-(3-méthoxy-phényl)-1*H*-benzimidazol-5-yl]-2-oxo-6*H-*[1,3,4]thiadiazin-3-yl}-acetic acid,
- 5-[1-Isobutyl-2-(3-méthoxy-phényl)-1*H*-benzimidazol-5-yl]-6-méthyl]-3,6-dihydro-2*H*-1,3,4-thiadiazin-2-one,
- 3-(2-Hydroxy-éthyl)-5-[1-isobutyl-2-(3-méthoxy-phényl)-1*H*-benzimidazol-5-yl]-6-méthyl]-3,6-dihydro-2*H*-1,3,4-thiadiazin-2-one,
- 5-[1-Isobutyl-2-(3-méthoxy-phényl)-1*H*-benzimidazol-5-yl]-3-propyl]-3,6-dihydro-2*H*-1,3,4-thiadiazin-2-one,
- 5-[1-Isobutyl-2-(3-méthoxy-phényl)-1H-benzimidazol-5-yl]-3,6-diméthyl]-3,6-dihydro-2*H*-1,3,4-thiadiazin-2-one,
- 3-Éthyl-5-[1-isobutyl-2-(3-méthoxy-phényl)-1*H*-benzimidazol-5-yl]-6-méthyl]-3,6-dihydro-2*H*-1,3,4-thiadiazin-2-one,
- 5-[1-Isobutyl-2-(3-méthoxy-phényl)-1*H*-benzimidazol-5-yl]-3-isopropyl-6-méthyl]-3,6-dihydro-2*H*-1,3,4-thiadiazin-2-one,
- 3-Cyclopropylméthyl-5-[1-isobutyl-2-(3-méthoxy-phényl)-1 H-benzimidazol-5-yl]-6-méthyl]-3,6-dihydro-2*H*-1,3,4-thiadiazin-2-one,
- 3-Éthyl-5-[2-(3-méthoxy-phényl)-1-méthyl-1*H*-benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-3-propyl]-3,6-dihydro-2*H*-1,3,4-thiadiazin-2-one; hydrochloride,
- 3-Cyclohexylméthyl-5-[2-(3-méthoxy-phényl)-1-méthyl-1*H*-benzimidazol-5-yl]-3,6-dihydro-2*H*-1,3,4-thiadiazin-2-one,
- 3-[2-(4-Fluoro-phénoxy)-éthyl]-5-[2-(3-méthoxy-phényl)-1-méthyl-1*H-*benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-3-méthyl]-3,6-dihydro-2*H*-1,3,4-thiadiazin-2-one,
- 3-Isopropyl-5-[2-(3-méthoxy-phényl)-1-méthyl-1*H*-benzimidazol-5-yl]-3,6-dihydro-2*H*-1,3,4-thiadiazin-2-one,
- 3-Isobutyl-5-[2-(3-méthoxy-phényl)-1-méthyl-1*H*-benzimidazol-5-yl]-3,6-dihydro-2*H*-1,3,4-thiadiazin-2-one,
- 3-Cyclopropylméthyl-5-[2-(3-méthoxy-phényl)-1-méthyl-1*H*-benzimidazol-5-yl]-3,6-dihydro-2*H*-1,3,4-thiadiazin-2-one,
- 5-[2-(3-Méthoxy-phényl)-1-méthyl-1H-benzimidazol-5-yl]-6-méthyl]-3,6-dihydro-2*H*-1,3,4-thiadiazin-2-one,
- 3-Éthyl-5-[2-(3-méthoxy-phényl)-1-méthyl-1*H*-benzimidazol-5-yl]-6-méthyl]-3,6-dihydro-2*H*-1,3,4-thiadiazin-2-one,
- 5-[2-(3-Méthoxy-phényl)-1-méthyl-1*H*-benzimidazol-5-yl]-6-méthyl-3-propyl]-3,6-dihydro-2*H*-1,3,4-thiadiazin-2-one,
- 5-[2-(3-Méthoxy-phényl)-1-méthyl-1*H*-benzimidazol-5-yl]-6-méthyl-3-(2-piperidin-1-yl-éthyl)]-3,6-dihydro-2*H*-1,3,4-thiadiazin-2-one,
- 3-Isobutyl-5-[2-(3-méthoxy-phényl)-1-méthyl-1*H*-benzimidazol-5-yl]-6-méthyl]-3,6-dihydro-2*H*-1,3,4-thiadiazin-2-one,
- 5-[2-(3-Méthoxy-phényl)-1-méthyl-1*H*-benzimidazol-5-yl]-3,6-diméthyl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3,6-dihydro-2*H*-1,3,4-thiadiazin-2-one,
- 3-Benzyl-6-méthyl-5-[1-méthyl-2-(3-trifluorométhyl-phényl)-1*H*-benzimidazol-5-yl]-3,6-dihydro-2*H*-1,3,4-thiadiazin-2-one,
- 3-Cyclopropylméthyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 3-Isopropyl-6-méthyl-5-[1-méthyl-2-(3-trifluorométhyl-phényl)-1H-benzimidazol-5-yl]-3,6-dihydro-2*H*-1,3,4-thiadiazin-2-one,
- 3,6-Diméthyl-5-[1-méthyl-2-(3-trifluorométhyl-phényl)-1*H*-benzimidazol-5-yl]-3,6-dihydro-2*H*-1,3,4-thiadiazin-2-one,
- 3-Éthyl-6-méthyl-5-[1-méthyl-2-(3-trifluorométhyl-phényl)-1*H*-benzimidazol-5-yl]-3,6-dihydro-2*H*-1,3,4-thiadiazin-2-one,
- 5-[1-Benzyl-2-(1-méthyl-1*H*-pyrrol-2-yl)-1*H*-benzimidazol-5-yl]-6-méthyl]-3,6-dihydro-2*H*-1,3,4-thiadiazin-2-one,
- 5-[1-Benzyl-2-(1-méthyl-1*H*-imidazol-2-yl)-1*H*-benzimidazol-5-yl]-6-méthyl]-3,6-dihydro-2*H*-1,3,4-thiadiazin-2-one,
- 5-(1-Benzyl-2-thiophé-2-yl-1*H*-benzimidazol-5-yl)-6-méthyl]-3,6-dihydro-2*H-*1,3,4-thiadiazin-2-one,
- 5-[1-Benzyl-2-(3-méthoxy-phényl)-1*H*-benzimidazol-5-yl]-6-méthyl]-3,6-dihydro-2*H*-1,3,4-thiadiazin-2-one,
- 5-[1-Benzyl-2-(4-méthoxy-phényl)-1*H*-benzimidazol-5-yl]-6-méthyl]-3,6-dihydro-2*H*-1,3,4-thiadiazin-2-one,
- 5-[1-Benzyl-2-(3-hydroxy-4-méthoxy-phényl)-1*H*-benzimidazol-5-yl]-6-méthyl]-3,6-dihydro-2*H*-1,3,4-thiadiazin-2-one,
- 5-[1-Benzyl-2-(2,4-dihydroxy-phényl)-1*H*-benzimidazol-5-yl]-6-méthyl]-3,6-dihydro-2*H*-1,3,4-thiadiazin-2-one,
- 5-[1-Benzyl-2-(3,4-dihydroxy-phényl)-1*H*-benzimidazol-5-yl]-6-méthyl]-3,6-dihydro-2*H*-1,3,4-thiadiazin-2-one,
- 5-[1-Benzyl-2-(3,4,5-trihydroxy-phényl)-1*H*-benzimidazol-5-yl]-6-méthyl]-3,6-dihydro-2*H*-1,3,4-thiadiazin-2-one,
- 6-Méthyl-5-[1-méthyl-2-(1-méthyl-1*H*-pyrrol-2-yl)-1H-benzimidazol-5-yl]-3,6-dihydro-2*H*-1,3,4-thiadiazin-2-one,
- 5-[2-(1*H*-Imidazol-4-yl)-1-méthyl-1*H*-benzimidazol-5-yl]-6-méthyl]-3,6-dihydro-2*H*-1,3,4-thiadiazin-2-one,
- 5-[2-(1*H*-Imidazol-2-yl)-1-méthyl-1*H*-benzimidazol-5-yl]-6-méthyl]-3,6-dihydro-2*H*-1,3,4-thiadiazin-2-one,
- 6-Méthyl-5-[1-méthyl-2-(5-méthyl-3*H*-imidazol-4-yl)-1*H*-benzimidazol-5-yl]-3,6-dihydro-2*H*-1,3,4-thiadiazin-2-one,
- 6-Méthyl-5-[1-méthyl-2-(1-méthyl-1*H*-imidazol-2-yl)-1*H*-benzimidazol-5-yl]-3,6-dihydro-2*H*-1,3,4-thiadiazin-2-one,
- 6-Méthyl-5-(1-méthyl-2-thiophé-2-yl-1*H*-benzimidazol-5-yl)]-3,6-dihydro-2*H-*1,3,4-thiadiazin-2-one,
- 6-Méthyl-5-(1-méthyl-2-thiophé-3-yl-1*H*-benzimidazol-5-yl)]-3,6-dihydro-2*H-*1,3,4-thiadiazin-2-one,
- 5-[2-(2-Méthoxy-phényl)-1-méthyl-1*H*-benzimidazol-5-yl]-6-méthyl]-3,6-dihydro-2*H*-1,3,4-thiadiazin-2-one,
- 5-[2-(4-Méthoxy-phényl)-1-méthyl-1*H*-benzimidazol-5-yl]-6-méthyl]-3,6-dihydro-2*H*-1,3,4-thiadiazin-2-one,
- 5-[2-(3-Hydroxy-4-méthoxy-phényl)-1-méthyl-1*H*-benzimidazol-5-yl]-6-méthyl]-3,6-dihydro-2*H*-1,3,4-thiadiazin-2-one,
- 5-[2-(3,4-Dihydroxy-phényl)-1-méthyl-1*H*-benzimidazol-5-yl]-6-méthyl]-3,6-dihydro-2*H*-1,3,4-thiadiazin-2-one,
- 6-Méthyl-5-[1-méthyl-2-(3,4,5-trihydroxy-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(3-méthyl-benzyl)]-3,6-dihydro-2*H*-1,3,4-thiadiazin-2-one,
- 5-[1-Éthyl-2-(3-fluoro-phényl)-1*H*-benzimidazol-5-yl]-3-(4-méthyl-benzyl)]-3,6-dihydro-2*H*-1,3,4-thiadiazin-2-one,
- 3-(4-tert-Butyl-benzyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(2-fluoro-benzyl)]-3,6-dihydro-2*H*-1,3,4-thiadiazin-2-one,
- 5-[1-Éthyl-2-(3-fluoro-phényl)-1*H*-benzimidazol-5-yl]-3-(3-fluoro-benzyl)]-3,6-dihydro-2*H*-1,3,4-thiadiazin-2-one,
- 3-Benzyl-5-[1-éthyl-2-(3-fluoro-phényl)-1*H*-benzimidazol-5-yl]-3,6-dihydro-2*H-*1,3,4-thiadiazin-2-one,
- 3-(3-Chloro-benzyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-méthyl]-3,6-dihydro-2*H-*1,3,4-thiadiazin-2-one,
- 3-Cyclohexylméthyl-5-[1-éthyl-2-(3-fluoro-phényl)-1*H*-benzimidazol-5-yl]-3,6-dihydro-2*H*-1,3,4-thiadiazin-2-one,
- 3-(2-Diméthylamino-éthyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(2-pyrrolidin-1-yl-éthyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-méthyl-benzyl)]-3,6-dihydro-2*H*-1,3,4-thiadiazin-2-one,
- 5-[2-(4-Méthoxy-phényl)-1*H*-benzimidazol-5-yl]-6-méthyl]-3,6-dihydro-2*H-*1,3,4-thiadiazin-2-one,
- 3-(2-Diméthylamino-éthyl)-6-méthyl-5-[1-méthyl-2-(3-trifluorométhyl-phényl)-1*H*-benzimidazol-5-yl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-piperidin-1-yl-éthyl)]-3,6-dihydro-2*H*-1,3,4-thiadiazin-2-one,
- 3-Cyclohexylméthyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-morpholin-4-yl-éthyl)]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-pyrrolidin-1-yl-éthyl)]-3,6-dihydro-2*H*-1,3,4-thiadiazin-2-one,
- 2-{6-Méthyl-5-[1-méthyl-2-(3-trifluorométhyl-phényl)-1*H*-benzimidazol-5-yl]-2-oxo-6*H*-[1,3,4]thiadiazin-3-yl}-acetamide,
- 3-(2-Méthoxy-éthyl)-6-méthyl-5-[1-méthyl-2-(3-trifluorométhyl-phényl)-1*H-*benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-(2-hydroxy-éthyl)-6-méthyl]-3,6-dihydro-2*H*-1,3,4-thiadiazin-2-one,
- 3-Cyclohexylméthyl-5-[2-(3-fluoro-phényl)-1-méthyl-1*H*-benzimidazol-5-yl]-6-méthyl]-3,6-dihydro-2*H*-1,3,4-thiadiazin-2-one,
- 2-{5-[2-(3-Fluoro-phényl)-1-méthyl-1*H*-benzimidazol-5-yl]-6-méthyl-2-oxo-6H-[1,3,4]thiadiazin-3-yl}-acetamide,
- 3-[2-(4-Fluoro-phénoxy)-éthyl]-5-[2-(3-fluoro-phényl)-1-méthyl-1*H-*benzimidazol-5-yl]-6-méthyl]-3,6-dihydro-2*H*-1,3,4-thiadiazin-2-one,
- 5-[2-(3-Fluoro-phényl)-1-méthyl-1*H*-benzimidazol-5-yl]-3-isopropyl-6-méthyl]-3,6-dihydro-2*H*-1,3,4-thiadiazin-2-one,
- 5-[2-(3-Fluoro-phényl)-1-méthyl-1*H*-benzimidazol-5-yl]-3-isobutyl-6-méthyl]-3,6-dihydro-2*H*-1,3,4-thiadiazin-2-one,
- 4-{5-[2-(3-Fluoro-phényl)-1-méthyl-1*H*-benzimidazol-5-yl]-6-méthyl-2-oxo-6H-[1,3,4]thiadiazin-3-yl}-butyric acid éthyl ester,
- 3-{5-[2-(3-Fluoro-phényl)-1-méthyl-1*H*-benzimidazol-5-yl]-6-méthyl-2-oxo-6*H-*[1,3,4]thiadiazin-3-yl}-propionic acid éthyl ester,
- 3-Cyclopropylméthyl-5-[2-(3-fluoro-phényl)-1-méthyl-1*H*-benzimidazol-5-yl]-6-méthyl]-3,6-dihydro-2*H*-1,3,4-thiadiazin-2-one,
- 5-[2-(3-Fluoro-phényl)-1-méthyl-1*H*-benzimidazol-5-yl]-3-(2-méthoxy-éthyl)-6-méthyl]-3,6-dihydro-2*H*-1,3,4-thiadiazin-2-one,
- 3-Benzyl-5-[2-(3-fluoro-phényl)-1-méthyl-1*H*-benzimidazol-5-yl]-6-méthyl]-3,6-dihydro-2*H*-1,3,4-thiadiazin-2-one,
- {5-[2-(3-Fluoro-phényl)-1-méthyl-1H-benzimidazol-5-yl]-6-méthyl-2-oxo-6*H-*[1,3,4]thiadiazin-3-yl}-acetic acid éthyl ester,
- 5-[2-(3-Fluoro-phényl)-1-méthyl-1*H*-benzimidazol-5-yl]-6-méthyl-3-propyl]-3,6-dihydro-2*H*-1,3,4-thiadiazin-2-one,
- 5-[2-(3-Fluoro-phényl)-1-méthyl-1*H*-benzimidazol-5-yl]-6-méthyl-3-(2-morpholin-4-yl-éthyl)]-3,6-dihydro-2*H*-1,3,4-thiadiazin-2-one
- 5-[2-(3-Fluoro-phényl)-1-méthyl-1*H*-benzimidazol-5-yl]-6-méthyl-3-(2-piperidin-1-yl-éthyl)]-3,6-dihydro-2*H*-1,3,4-thiadiazin-2-one,
- 5-[2-(3-Fluoro-phényl)-1-méthyl-1*H*-benzimidazol-5-yl]-6-méthyl]-3,6-dihydro-2*H*-1,3,4-thiadiazin-2-one,
- 3-Cyclohexylméthyl-5-[2-(3-fluoro-phényl)-1-méthyl-1*H*-benzimidazol-5-yl]-3,6-dihydro-2*H*-1,3,4-thiadiazin-2-one,
- 2-{5-[2-(3-Fluoro-phényl)-1-méthyl-1H-benzimidazol-5-yl]-2-oxo-6*H-*[1,3,4]thiadiazin-3-yl}-acetamide,
- 3-[2-(4-Fluoro-phénoxy)-éthyl]-5-[2-(3-fluoro-phényl)-1-méthyl-1*H-*benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-isopropyl]-3,6-dihydro-2*H*-1,3,4-thiadiazin-2-one,
- 5-[2-(3-Fluoro-phényl)-1-méthyl-1*H*-benzimidazol-5-yl]-3-isobutyl]-3,6-dihydro-2*H*-1,3,4-thiadiazin-2-one,
- 3-Cyclopropylméthyl-5-[2-(3-fluoro-phényl)-1-méthyl-1*H*-benzimidazol-5-yl]-3,6-dihydro-2*H*-1,3,4-thiadiazin-2-one
- 5-[2-(3-Fluoro-phényl)-1-méthyl-1*H*-benzimidazol-5-yl]-3-(2-méthoxy-éthyl)]-3,6-dihydro-2*H*-1,3,4-thiadiazin-2-one,
- 3-Benzyl-5-[2-(3-fluoro-phényl)-1-méthyl-1*H*-benzimidazol-5-yl]-3,6-dihydro-2*H*-1,3,4-thiadiazin-2-one,
- {5-[2-(3-Fluoro-phényl)-1-méthyl-1H-benzimidazol-5-yl]-2-oxo-6*H-*[1,3,4]thiadiazin-3-yl}-acetic acid éthyl ester,
- 5-[2-(3-Fluoro-phényl)-1-méthyl-1*H*-benzimidazol-5-yl]-3-(2-piperidin-1-yl-éthyl)]-3,6-dihydro-2*H*-1,3,4-thiadiazin-2-one,
- 5-[2-(3-Fluoro-phényl)-1-méthyl-1*H*-benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-propyl]-3,6-dihydro-2*H*-1,3,4-thiadiazin-2-one,
- 5-(1-Benzyl-2-thiophé-3-yl-1*H*-benzimidazol-5-yl)-6-méthyl]-3,6-dihydro-2*H-*1,3,4-thiadiazin-2-one,
- 5-[1-Benzyl-2-(2-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(3-méthoxy-phényl)-1*H*-benzimidazol-5-yl]-6-méthyl]-3,6-dihydro-2*H*-1,3,4-thiadiazin-2-one,
- 3-Cyclohexylméthyl-5-(2-furan-2-yl-1-méthyl-1*H*-benzimidazol-5-yl)]-3,6-dihydro-2*H*-1,3,4-thiadiazin-2-one,
- 3-Benzyl-5-(2-furan-2-yl-1-méthyl-1*H*-benzimidazol-5-yl)]-3,6-dihydro-2*H-*1,3,4-thiadiazin-2-one,
- 3-Cyclopropylméthyl-5-(2-furan-2-yl-1-méthyl-1*H*-benzimidazol-5-yl)]-3,6-dihydro-2*H*-1,3,4-thiadiazin-2-one,
- 5-(2-Furan-2-yl-1-méthyl-1*H*-benzimidazol-5-yl)-3-méthyl]-3,6-dihydro-2*H-*1,3,4-thiadiazin-2-one,
- 3-Éthyl-5-(2-furan-2-yl-1-méthyl-1*H*-benzimidazol-5-yl)]-3,6-dihydro-2*H*-1,3,4-thiadiazin-2-one,
- 5-(2-Furan-2-yl-1-méthyl-1*H*-benzimidazol-5-yl)-3-(2-piperidin-1-yl-éthyl)]-3,6-dihydro-2*H*-1,3,4-thiadiazin-2-one,
- 5-(2-Furan-2-yl-1-méthyl-1*H*-benzimidazol-5-yl)-3-(2-pyrrolidin-1-yl-éthyl)]-3,6-dihydro-2*H*-1,3,4-thiadiazin-2-one,
- 5-(2-Furan-2-yl-1-méthyl-1*H*-benzimidazol-5-yl)-3-isobutyl]-3,6-dihydro-2*H-*1,3,4-thiadiazin-2-one,
- 5-(2-Furan-2-yl-1-méthyl-1*H*-benzimidazol-5-yl)]-3,6-dihydro-2*H*-1,3,4-thiadiazin-2-one,
- 2-{5-[2-(3-Méthoxy-phényl)-1-méthyl-1*H*-benzimidazol-5-yl]-6-méthyl-2-oxo-6*H*-[1,3,4]thiadiazin-3-yl}-acetamide,
- 3-Isopropyl-5-[2-(3-méthoxy-phényl)-1-méthyl-1*H*-benzimidazol-5-yl]-6-méthyl]-3,6-dihydro-2*H*-1,3,4-thiadiazin-2-one,
- 3-(4-tert-Butyl-benzyl)-5-[1-méthyl-2-(3-trifluorométhyl-phényl)-1*H-*benzimidazol-5-yl]-3,6-dihydro-2*H*-1,3,4-thiadiazin-2-one,
- 3-(4-Fluoro-benzyl)-5-[1-méthyl-2-(3-trifluorométhyl-phényl)-1*H*-benzimidazol-5-yl]-3,6-dihydro-2*H*-1,3,4-thiadiazin-2-one,
- 5-[1-Méthyl-2-(3-trifluorométhyl-phényl)-1*H*-benzimidazol-5-yl]-3-(4-trifluoro méthoxy-benzyl)]-3,6-dihydro-2*H*-1,3,4-thiadiazin-2-one,
- 5-[1-Méthyl-2-(3-trifluorométhyl-phényl)-1*H*-benzimidazol-5-yl]-3-(4-trifluorométhyl-benzyl)]-3,6-dihydro-2*H*-1,3,4-thiadiazin-2-one,
- 3-{5-[1-Méthyl-2-(3-trifluorométhyl-phényl)-1*H*-benzimidazol-5-yl]-2-oxo-6*H-*[1,3,4]thiadiazin-3-ylméthyl}-benzonitrile,
- 3-(4-Méthanesulfonyl-benzyl)-5-[1-méthyl-2-(3-trifluorométhyl-phényl)-1*H-*benzimidazol-5-yl]-3,6-dihydro-2*H*-1,3,4-thiadiazin-2-one,
- 3-(3,5-Diméthoxy-benzyl)-5-[1-méthyl-2-(3-trifluorométhyl-phényl)-1*H-*benzimidazol-5-yl]-3,6-dihydro-2*H*-1,3,4-thiadiazin-2-one,
- 5-[1-Méthyl-2-(3-trifluorométhyl-phényl)-1*H*-benzimidazol-5-yl]-3,6-dihydro-2*H-*1,3,4-thiadiazin-2-one,
- 5-[1-Éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(4-fluoro-benzyl)]-3,6-dihydro-2*H*-1,3,4-thiadiazin-2-one,
- 5-[1-Éthyl-2-(3-fluoro-phényl)-1*H*-benzimidazol-5-yl]-3-(3-méthoxy-benzyl)]-3,6-dihydro-2*H*-1,3,4-thiadiazin-2-one,
- 5-[1-Éthyl-2-(3-fluoro-phényl)-1*H*-benzimidazol-5-yl]-3-(4-trifluorométhoxy-benzyl)]-3,6-dihydro-2*H*-1,3,4-thiadiazin-2-one,
- 2-{5-[1-Éthyl-2-(3-fluoro-phényl)-1*H*-benzimidazol-5-yl]-2-oxo-6*H-*[1,3,4]thiadiazin-3-yl}-acetamide,
- 3-Cyclopropylméthyl-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-isopropyl]-3,6-dihydro-2*H*-1,3,4-thiadiazin-2-one
- 3-Éthyl-5-[1-éthyl-2-(3-fluoro-phényl)-1*H*-benzimidazol-5-yl]-3,6-dihydro-2*H-*1,3,4-thiadiazin-2-one,
- 5-[1-Éthyl-2-(3-fluoro-phényl)-1*H*-benzimidazol-5-yl]-3-propyl]-3,6-dihydro-2*H-*1,3,4-thiadiazin-2-one,
- 5-[1-Éthyl-2-(3-fluoro-phényl)-1*H*-benzimidazol-5-yl]-3-(2-méthoxy-éthyl)]-3,6-dihydro-2*H*-1,3,4-thiadiazin-2-one,
- {5-[1-Éthyl-2-(3-fluoro-phényl)-1*H*-benzimidazol-5-yl]-2-oxo-6H-[1,3,4]thiadiazin-3-yl}-acetic acid,
- 5-[1-Éthyl-2-(3-fluoro-phényl)-1*H*-benzimidazol-5-yl]-3-(2-hydroxy-éthyl)]-3,6-dihydro-2*H*-1,3,4-thiadiazin-2-one,
- 5-[1-Éthyl-2-(3-fluoro-phényl)-1*H*-benzimidazol-5-yl]-3-(3-hydroxy-propyl)]-3,6-dihydro-2*H*-1,3,4-thiadiazin-2-one,
- 3-(2-Amino-éthyl)-5-[1-éthyl-2-(3-fluoro-phényl)-1*H*-benzimidazol-5-yl]-3,6-dihydro-2*H*-1,3,4-thiadiazin-2-one,
- 3-(3-Amino-propyl)-5-[1-éthyl-2-(3-fluoro-phényl)-1*H*-benzimidazol-5-yl]-3,6-dihydro-2*H*-1,3,4-thiadiazin-2-one,
- 3-Benzyl-5-[1-éthyl-2-(3-méthoxy-phényl)-1*H*-benzimidazol-5-yl]-3,6-dihydro-2*H*-1,3,4-thiadiazin-2-one,
- 5-[1-Éthyl-2-(3-méthoxy-phényl)-1*H*-benzimidazol-5-yl]-3-(4-méthyl-benzyl)]-3,6-dihydro-2*H*-1,3,4-thiadiazin-2-one,
- 5-[1-Éthyl-2-(3-méthoxy-phényl)-1*H*-benzimidazol-5-yl]-3-(2-fluoro-benzyl)]-3,6-dihydro-2*H*-1,3,4-thiadiazin-2-one,
- 3-Benzyl-5-[1-méthyl-2-(3-trifluorométhyl-phényl)-1*H*-benzimidazol-5-yl]-3,6-dihydro-2*H*-1,3,4-thiadiazin-2-one,
- 3-Méthyl-5-[1-méthyl-2-(3-trifluorométhyl-phényl)-1*H*-benzimidazol-5-yl]-3,6-dihydro-2*H*-1,3,4-thiadiazin-2-one,
- 3-Cyclohexylméthyl-5-[1-méthyl-2-(3-trifluorométhyl-phényl)-1 *H*-benzimidazol-5-yl]-3,6-dihydro-2*H*-1,3,4-thiadiazin-2-one,
- 3-(3-Amino-propyl)-5-[1-méthyl-2-(3-trifluorométhyl-phényl)-1*H*-benzimidazol-5-yl]-3,6-dihydro-2*H*-1,3,4-thiadiazin-2-one,
- 3-(2-Amino-éthyl)-5-[1-méthyl-2-(3-trifluorométhyl-phényl)-1*H*-benzimidazol-5-yl]-3,6-dihydro-2*H*-1,3,4-thiadiazin-2-one,
- 3-(2-Amino-éthyl)-5-[1-éthyl-2-(3-méthoxy-phényl)-1*H*-benzimidazol-5-yl]-3,6-dihydro-2*H*-1,3,4-thiadiazin-2-one,
- 3-(3-Amino-propyl)-5-[1-éthyl-2-(3-méthoxy-phényl)-1*H*-benzimidazol-5-yl]-3,6-dihydro-2*H*-1,3,4-thiadiazin-2-one,
- 5-[1-Éthyl-2-(3-méthoxy-phényl)-1*H*-benzimidazol-5-yl]-3-(2-hydroxy-éthyl)]-3,6-dihydro-2*H*-1,3,4-thiadiazin-2-one
- 5-[1-Éthyl-2-(3-méthoxy-phényl)-1*H*-benzimidazol-5-yl]-3-(3-hydroxy-propyl)]-3,6-dihydro-2*H*-1,3,4-thiadiazin-2-one,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-acetamide,
- Furan-2-carboxylic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-isobutyramide,
- Cyclopentanecarboxylic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-benzamide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-méthanesulfonamide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-benzenesulfonamide,
- 5-[1-Méthyl-2-(3-trifluorométhyl-phényl)-1*H*-benzimidazol-5-yl]-6-propyl]-3,6-dihydro-2*H*-1,3,4-thiadiazin-2-one,
- 3-Éthyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-3,6-dipropyl]-3,6-dihydro-2*H*-1,3,4-thiadiazin-2-one,
- 3-Benzyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Cyclopropylméthyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Isopropyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Isobutyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-3-(2-piperidin-1-yl-éthyl)-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-6-propyl-3-(2-pyrrolidin-1-yl-éthyl)]-3,6-dihydro-2*H*-1,3,4-thiadiazin-2-one,
- 1-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-3-phényl-urée,
- 1-Éthyl-3-(2-{5-[1-méthyl-2-(3-trifluorométhyl-phényl)-1*H*-benzimidazol-5-yl]-2-oxo-6*H*-[1,3,4]thiadiazin-3-yl}-éthyl)-urée,
- 5-[1-Éthyl-2-(3-méthoxy-phényl)-1*H*-benzimidazol-5-yl]-3,6-dihydro-2*H*-1,3,4-thiadiazin-2-one,
- 3-Cyclopropylméthyl-5-[1-méthyl-2-(3-trifluorométhyl-phényl)-1*H*-benzimidazol-5-yl]-3,6-dihydro-2*H*-1,3,4-thiadiazin-2-one,
- Butane-1-sulfonic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-{2-[5-(2-Furan-2-yl-1-méthyl-1*H*-benzimidazol-5-yl)-2-oxo-6*H-*[1,3,4]thiadiazin-3-yl]-éthyl}-acetamide,
- N-{2-[5-(2-Furan-2-yl-1-méthyl-1*H*-benzimidazol-5-yl)-2-oxo-6*H-*[1,3,4]thiadiazin-3-yl]-éthyl}-isobutyramide,
- Cyclopentanecarboxylic acid {2-[5-(2-furan-2-yl-1-méthyl-1*H*-benzimidazol-5-yl)-2-oxo-6*H*-[1,3,4]thiadiazin-3-yl]-éthyl}-amide,
- N-{2-[5-(2-Furan-2-yl-1-méthyl-1*H*-benzimidazol-5-yl)-2-oxo-6H-[1,3,4]thiadiazin-3-yl]-éthyl}-benzamide,
- N-{2-[5-(2-Furan-2-yl-1-méthyl-1*H*-benzimidazol-5-yl)-2-oxo-6*H-*[1,3,4]thiadiazin-3-yl]-éthyl}-nicotinamide,
- Cyclopropanecarboxylic acid {2-[5-(2-furan-2-yl-1-méthyl-1*H*-benzimidazol-5-yl)-2-oxo-6*H*-[1,3,4]thiadiazin-3-yl]-éthyl}-amide,
- 3-Fluoro-N-{2-[5-(2-furan-2-yl-1-méthyl-1*H*-benzimidazol-5-yl)-2-oxo-6*H-*[1,3,4]thiadiazin-3-yl]-éthyl}-benzamide,
- N-{2-[5-(2-Furan-2-yl-1-méthyl-1*H*-benzimidazol-5-yl)-2-oxo-6*H-*[1,3,4]thiadiazin-3-yl]-éthyl}-4-méthyl-benzamide,
- 3-(2-Amino-éthyl)-5-(2-furan-2-yl-1-méthyl-1*H*-benzimidazol-5-yl)]-3,6-dihydro-2*H*-1,3,4-thiadiazin-2-one,
- N-{2-[5-(2-Furan-2-yl-1-méthyl-1*H*-benzimidazol-5-yl)-2-oxo-6*H-*[1,3,4]thiadiazin-3-yl]-éthyl}-benzenesulfonamide,
- Butane-1-sulfonic acid {2-[5-(2-furan-2-yl-1-méthyl-1*H*-benzimidazol-5-yl)-2-oxo-6*H*-[1,3,4]thiadiazin-3-yl]-éthyl}-amide,
- 1-{2-[5-(2-Furan-2-yl-1-méthyl-1*H*-benzimidazol-5-yl)-2-oxo-6*H-*[1,3,4]thiadiazin-3-yl]-éthyl}-3-isopropyl-urée,
- 1-{2-[5-(2-Furan-2-yl-1-méthyl-1*H*-benzimidazol-5-yl)-2-oxo-6*H-*[1,3,4]thiadiazin-3-yl]-éthyl}-3-phényl-urée,
- 1-Cyclopentyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-Éthyl-3-{2-[5-(2-furan-2-yl-1-méthyl-1*H*-benzimidazol-5-yl)-2-oxo-6H-[1,3,4]thiadiazin-3-yl]-éthyl}-urée,
- 1-Benzyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-(4-Fluoro-benzyl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-(4-Acetyl-phényl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-{2-[5-(2-Furan-2-yl-1-méthyl-1*H*-benzimidazol-5-yl)-2-oxo-6*H-*[1,3,4]thiadiazin-3-yl]-éthyl}-3-p-tolyl-urée,
- 1-Butyl-3-{2-[5-(2-furan-2-yl-1-méthyl-1*H*-benzimidazol-5-yl)-2-oxo-6*H-*[1,3,4]thiadiazin-3-yl]-éthyl}-urée,
- N-{3-[5-(2-Furan-2-yl-1-méthyl-1*H*-benzimidazol-5-yl)-2-oxo-6*H-*[1,3,4]thiadiazin-3-yl]-propyl}-benzamide,
- Cyclopropanecarboxylic acid {3-[5-(2-furan-2-yl-1-méthyl-1*H*-benzimidazol-5-yl)-2-oxo-6*H*-[1,3,4]thiadiazin-3-yl]-propyl}-amide,
- 1-(4-Chloro-phényl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-(4-Chloro-phényl)-3-{3-[5-(2-furan-2-yl-1-méthyl-1*H*-benzimidazol-5-yl)-2-oxo-6*H*-[1,3,4]thiadiazin-3-yl]-propyl}-urée,
- 1-Butyl-3-{3-[5-(2-furan-2-yl-1-méthyl-1H-benzimidazol-5-yl)-2-oxo-6H-[1,3,4]thiadiazin-3-yl]-propyl}-urée,
- 1-Benzyl-3-{3-[5-(2-furan-2-yl-1-méthyl-1*H*-benzimidazol-5-yl)-2-oxo-6*H-*[1,3,4]thiadiazin-3-yl]-propyl}-urée,
- 1-(3-Fluoro-phényl)-3-{3-[5-(2-furan-2-yl-1-méthyl-1*H*-benzimidazol-5-yl)-2-oxo-6*H*-[1,3,4]thiadiazin-3-yl]-propyl}-urée
- N-{2-[5-(2-Furan-2-yl-1-méthyl-1*H*-benzimidazol-5-yl)-2-oxo-6*H-*[1,3,4]thiadiazin-3-yl]-éthyl}-méthanesulfonamide,
- 5-(2-Furan-2-yl-1-méthyl-1*H*-benzimidazol-5-yl)-3-(2-méthoxy-éthyl)]-3,6-dihydro-2*H*-1,3,4-thiadiazin-2-one,
- 5-(2-Furan-2-yl-1-méthyl-1*H*-benzimidazol-5-yl)-3-(3-hydroxy-propyl)]-3,6-dihydro-2*H*-1,3,4-thiadiazin-2-one,
- 5-(2-Furan-2-yl-1-méthyl-1*H*-benzimidazol-5-yl)-3-(2-hydroxy-éthyl)]-3,6-dihydro-2*H*-1,3,4-thiadiazin-2-one,
- 3-(3-Amino-propyl)-5-(2-furan-2-yl-1-méthyl-1*H*-benzimidazol-5-yl)]-3,6-dihydro-2*H*-1,3,4-thiadiazin-2-one,
- 3-(2-Amino-éthyl)-5-[2-(3-fluoro-phényl)-1-méthyl-1*H*-benzimidazol-5-yl]-6-méthyl]-3,6-dihydro-2*H*-1,3,4-thiadiazin-2-one,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-2-phényl-acetamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-4-méthyl-benzamide,
- Cyclopropanecarboxylic acid (2-{5-[2-(3-fluoro-phényl)-1-méthyl-1*H-*benzimidazol-5-yl]-6-méthyl-2-oxo-6*H*-[1,3,4]thiadiazin-3-yl}-éthyl)-amide,
- Cyclopentanecarboxylic acid (2-{5-[2-(3-fluoro-phényl)-1-méthyl-1*H-*benzimidazol-5-yl]-6-méthyl-2-oxo-6*H*-[1,3,4]thiadiazin-3-yl}-éthyl)-amide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-2-méthoxy-acetamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-nicotinamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-benzamide,
- N-(2-{5-[2-(3-Fluoro-phényl)-1-méthyl-1*H*-benzimidazol-5-yl]-6-méthyl-2-oxo-6H-[1,3,4]thiadiazin-3-yl}-éthyl)-acétamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-isobutyramide,
- (S)-5-{2-[4-((S)-Méthanesulfinyl)-3-méthoxy-phényl]-1*H*-benzimidazol-5-yl}-6-méthyl]-3,6-dihydro-2*H*-1,3,4-thiadiazin-2-one,
- (R)-5-{2-[4-((S)-Méthanesulfinyl)-3-méthoxy-phényl]-1*H*-benzimidazol-5-yl}-6-méthyl]-3,6-dihydro-2*H*-1,3,4-thiadiazin-2-one,
- (S)-5-{2-[4-((R)-Méthanesulfinyl)-3-méthoxy-phényl]-1*H*-benzimidazol-5-yl}-6-méthyl]-3,6-dihydro-2*H*-1,3,4-thiadiazin-2-one,
- (R)-5-{2-[4-((R)-Méthanesulfinyl)-3-méthoxy-phényl]-1*H*-benzimidazol-5-yl}-6-méthyl]-3,6-dihydro-2*H*-1,3,4-thiadiazin-2-one,
- 1-(4-Acetyl-phényl)-3-(2-{5-[2-(3-fluoro-phényl)-1-méthyl-1*H*-benzimidazol-5-yl]-6-méthyl-2-oxo-6*H*-[1,3,4]thiadiazin-3-yl}-éthyl)-urée,
- 1-(3-Fluoro-phényl)-3-(2-{5-[2-(3-fluoro-phényl)-1-méthyl-1*H*-benzimidazol-5-yl]-6-méthyl-2-oxo-6*H*-[1,3,4]thiadiazin-3-yl}-éthyl)-urée,
- 5-[1-Cyclopropylméthyl-2-(1-méthyl-1*H*-pyrrol-2-yl)-1*H*-benzimidazol-5-yl]-6-méthyl]-3,6-dihydro-2*H*-1,3,4-thiadiazin-2-one,
- 5-[1-Cyclopropylméthyl-2-(1-méthyl-1*H*-imidazol-2-yl)-1*H*-benzimidazol-5-yl]-6-méthyl]-3,6-dihydro-2*H*-1,3,4-thiadiazin-2-one,
- 5-(1-Cyclopropylméthyl-2-thiophé-3-yl-1*H*-benzimidazol-5-yl)-6-méthyl]-3,6-dihydro-2*H*-1,3,4-thiadiazin-2-one,
- 5-[1-Cyclopropylméthyl-2-(2-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(4-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(3-hydroxy-4-méthoxy-phényl)-1*H*-benzimidazol-5-yl]-6-méthyl]-3,6-dihydro-2*H*-1,3,4-thiadiazin-2-one,
sous forme tautomères, énantiomères, diastéréoisomères, épimères, ainsi que les sels pharmaceutiquement acceptables, et les formes cristallines, ou leurs mélanges.

On préfère plus particulièrement les composés choisis parmi :
- 5-[2-(3-Fluorophényl)-1-méthyl-1*H*-benzimidazol-5-yl]-3-isopropyl-6-méthyl]-3,6-dihydro-2*H*-1,3,4-thiadiazin-2-one,
- 5-[2-(3-Fluorophényl)-1-méthyl-1*H*-benzimidazol-5-yl]-6-méthyl-3-propyl]-3,6-dihydro-2*H*-1,3,4-thiadiazin-2-one,
- 5-{2-[4-(1H-imidazol-1-yl)phényl]-1*H*-benzimidazol-5-yl}-6-méthyl-3,6-dihydro-2*H*-1,3,4-thiadiazin-2-one dichlorhydrate,
- 5-[1-Cycloheptyl-2-(3-méthoxy-phényl)-1*H*-benzimidazol-5-yl]-3-méthyl]-3,6-dihydro-2*H*-1,3,4-thiadiazin-2-one,
- 6-méthyl-5-{2-[3-(méthoxy)phényl]-1*H*-benzimidazol-5-yl}-3,6-dihydro-2*H-*1,3,4-thiadiazin-2-one,
- 5-{2-[3-méthoxy-4-(méthylsulfinyl)phényl]-1*H*-benzimidazol-5-yl}-6-méthyl-3,6-dihydro-2*H*-1,3,4-thiadiazin-2-one, chlorhydrate,
sous forme de tautomères, énantiomères, diastéréoisomères, épimères, ainsi que les formes libres ou les sels pharmaceutiquement acceptables, et les formes cristallines, ou leurs mélanges.

Dans la présente description, les termes utilisés ont les significations suivantes, sauf indication contraire :
- le terme « alkyl(e) » désigne un radical alkyle linéaire ou ramifié. De façon non limitative, parmi les radicaux alkyle(C₁-C₂₀), on peut notamment citer les radicaux méthyle, éthyle, propyle, isopropyle, butyle, sec-butyle, *tert*-butyle, pentyle et hexyle, octyle, décyle, dodécyle, hexadécyle et octadécyle ;
- le terme « alcényl(e) » désigne un radical hydrocarboné, linéaire ou ramifié, comportant une ou plusieurs insaturations sous forme de double liaison. De façon non limitative, on peut citer comme radical alcényle(C₂-C₂₀) les radicaux, éthényle, prop-2-ényle, but-2-ényle, but-3-ényle, pent-2-ényle, pent-3-ényle, pent-4-ényle ;
- le terme « alcynyl(e) » désigne un radical hydrocarboné, linéaire ou ramifié, comportant une ou plusieurs insaturations sous forme de triple liaison, pouvant éventuellement comprendre également une ou plusieurs insaturations sous forme de double liaison. De façon non limitative, on peut citer à titre de radical alcényle(C₂-C₂₀) les radicaux, éthynyle, prop-2-ynyle, but-2-ynyle, but-3-ynyle, pent-2-ynyle, pent-3-ynyle, pent-4-ynyle ;
- le terme « alkoxy » se réfère au terme « alkyl-oxy » ;
- parmi les « halogènes », on peut notamment citer le fluor, le chlore, le brome ;
- le terme « cycloalkyle » désigne un radical hydrocarboné cyclique saturé, éventuellement substitué, et comprend les composés mono-, bi- et tri-cycliques, possédant de 3 à 10 atomes de carbone. Parmi les « cycloalkyles », on peut notamment citer les radicaux cyclopropyle, cyclobutyle, cyclopentyle, cyclohexyle, cycloheptyle, cyclooctyle, cyclodécyle, adamantyle, et autres, tous étant éventuellement substitués ;
- le terme « cycloalcényle » désigne un radical hydrocarboné mono-, bi- ou tri-cyclique, éventuellement substitué, comprenant au moins une insaturation sous forme de double liaison, ayant de 3 à 10 atomes de carbone. Parmi les « cycloalcényles », on peut notamment citer les radicaux cyclopentényle, cyclopentadiényle, cyclohexényle, camphényle, norbornényle ;
- dans la présente invention, le terme « hétérocycloalkyle » désigne à la fois les hétérocycloalkyles et les hétérocycloalcényles. Ces radicaux sont éventuellement substitués et peuvent être mono-, bi- ou tri-cycliques et comprennent un ou plusieurs hétéroatomes, de préférence choisis parmi O, S and N, éventuellement à l'état oxydé (cas de S et de N), ainsi qu'éventuellement une ou deux doubles liaisons. De préférence, au moins un des cycles comprend de 1 à 4 hétéroatomes endocycliques, plus préférentiellement de 1 à 3 hétéroatomes.

Avantageusement, un radical hétérocycloalkyle comprend un ou plusieurs cycles, chacun comportant de 5 à 8 atomes. Des exemples de radicaux hétérocycloalkyles sont : morpholinyle, pipéridinyle, pipérazinyle, thiazolidinyle, oxazolidinyle, tétrahydrothiényle, dihydrofuranyle, tétrahydrofuranyle, pirazolidinyle, 1,3-dioxolanyle, pyrrolidinyle, pyranyle, dihydropyranyle, isoxazolidinyle, imidazolinyle, imidazolidinyle ou pyrazolidinyle ;
- le terme « aryle » désigne des radicaux aromatiques, mono ou polycycliques comprenant de 5 à 14 atomes cycliques, et au moins un cycle présente un système d'électrons pi (π) conjugués, y compris les groupes biaryle, chacun d'entre eux pouvant être éventuellement substitué. Parmi les « aryles », on peut notamment citer les radicaux phényle, naphtyle, biphényle, anthryle, phénanthryle, indényle ;
- le terme « hétéroaryle » désigne un radical hétérocyclique aromatique comprenant de 5 à 14 atomes endocycliques, parmi lesquels de 1 à 4 atomes sont des hétéroatomes, de préférence choisi parmi oxygène, soufre, azote. Parmi les « hétéroaryles », on peut notamment citer les radicaux furanyle, benzofuranyle, thiényle, pyridyle, pyridyl-N-oxyde, pyrimidinyle, pyrazinyle, oxazolyle, thiazolyle, isoxazolyle, quinolinyle, triazolyle, pyridazinyle, pyrrolyle, imidazolyle, indazolyle, isothiazolyle, indolyle, oxadiazolyle.

La présente invention concerne également le procédé de préparation des composés de formule générale (I).

Selon un premier mode de réalisation, le procédé de préparation des composés de formule (I) dans laquelle R3 est différent d'un atome d'hydrogène comprend l'étape consistant à substituer le composé correspondant de formule (I) dans laquelle R3 est égal à H , c'est-à-dire le composé de formule (F) ci-dessous: 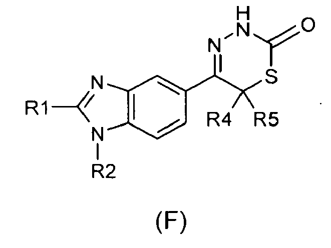 dans laquelle R1, R2, R4, R5 sont tels que définis en formule (I) au moyen d'un réactif approprié, selon la valeur du groupe R3 désiré. Cette réaction à la portée de l'homme du métier peut être effectuée par application ou adaptation de toute méthode connue de l'art antérieur, par exemple telle que décrite par J. March, Jerry, Advanced Organic Chemistry, 3rd Ed., Wiley Interscience, p. 310-316.

Cette réaction peut être réalisée selon l'un ou l'autre des modes de réalisation suivants :

Selon un premier mode de réalisation, la dihydrothiadiazinone peut être réalisée selon la réaction suivante : 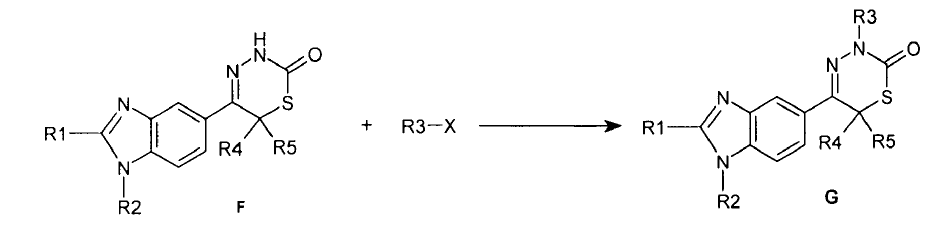 dans lesquels R1, R2, R4 et R5 sont tels que décrits précédemment et R3 est différent d'un atome d'hydrogène. Le composé G peut être obtenu par réaction du composé F avec, par exemple, un halogénure d'akyle ramifié ou non. La réaction peut être conduite en présence d'une base organique telle que, et ce de façon non limitative, la triéthylamine, la pyridine, la diisopropyleéthylamine ou bien en présence d'une base minérale telle que l'hydrogéno-carbonate de sodium ou le carbonate de césium dans un solvant tel que le diméthylformamide ou l'acétonitrile, ou bien dans un solvant de type éther tel que le THF ou le dioxane à une température allant de 0°C à la température d'ébullition du solvant utilisé, le temps de réaction pouvant être compris entre 10 mn et 48 h, de préférence 1 h à 24 h.

Selon un second mode de réalisation, lorsque R3 représente un groupe alkyle substitué par un groupe amino, la substitution de la dihydrothiadiazinone peut être réalisée selon les réactions suivantes : 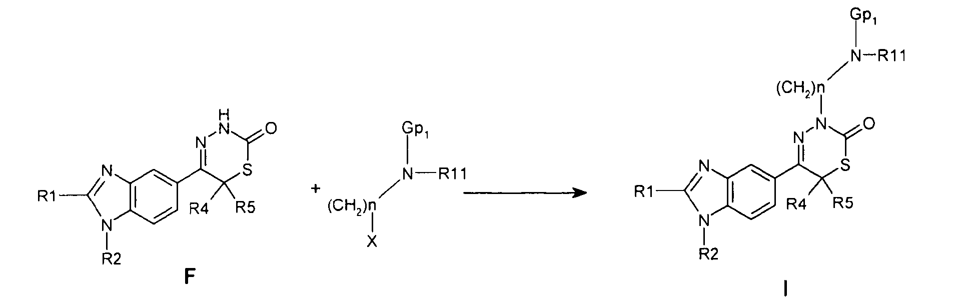 dans lesquels R1, R2, R4, R5, R11 et n sont tels que décrits précédemment, et où le groupe protecteur Gp1 peut être choisi parmi ceux utilisés communément dans la pratique de la synthèse organique pour la protection des amines et n est un entier compris entre 1et 10, puis 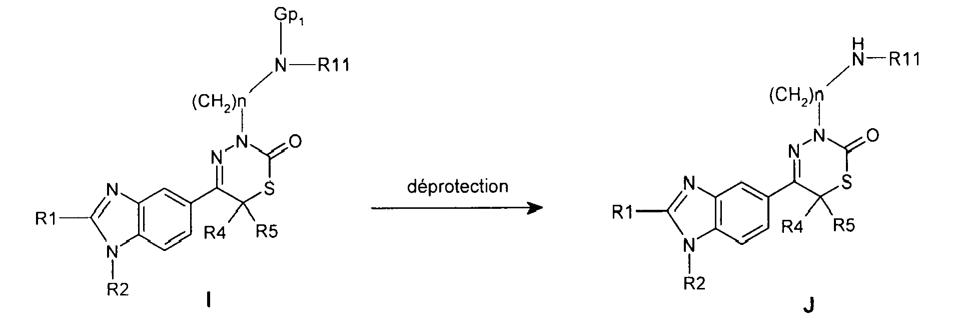 dans lesquels Gp1, R1, R2, R4, R5, R11 etn sont tels que décrits précédemment.

Dans des conditions de réaction similaires ou légérement modifiées, de même que pour le composé G, le composé I peut être obtenu par réaction du composé F avec, par exemple, un halogénure d'akyle, ramifié ou non, porteur dans ce cas d'une fonction amine substituée ou non et protégée par un groupe protecteur tel que, et ce de façon non limitative, un groupe terbutoxycarbonyle ou un groupe benzyloxycarbonyle.

Le composé J est obtenu à partir du composé I en utilisant les méthodes connues de déprotection ; on peut citer notamment l'utilisation d'acide trifluoroacétique, de façon avantageuse, on peut également utiliser une solution d'acide chlorhydrique dans un solvant tel que le dioxane à une température comprise entre 0°C et la température d'ébullition du solvant utilisé.

Selon un troisième mode de réalisation, lorsque R3 représente un groupe (CH₂)ₙ-NR11-COR14, tel que défini en formule générale (I), le procédé selon l'invention comprend l'étape suivante : 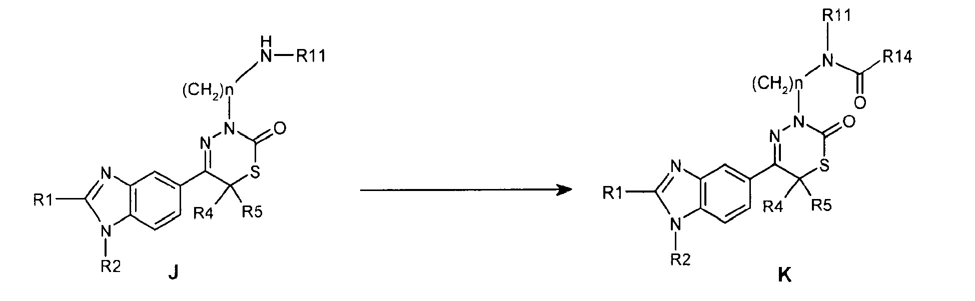 dans lesquels R1, R2, R4, R5, R11, R14 et n sont tels que décrits précédemment.

Le composé K peut être obtenu par action d'un halogénure d'acide convenablement choisi de formule

Hal-CO-R14

dans laquelle Hal représente un atome d'halogène, par exemple un chlorure d'acide tel que, et ce à titre d'exemple, le chlorure de phénacyle sur le composé J en présence d'une base organique telle que, et ce de façon non limitative, la triéthylamine, la pyridine, la diisopropyle-éthyleamine dans un solvant tel que l'acétonitrile, le toluène, le dichlorométhane ou bien encore le tétrahydrofurane.

On peut également utiliser une base minérale telle que, et ce de façon non limitative, l'hydrogénocarbonate de sodium ou le carbonate de césium. On peut également obtenir ces dérivés de type amide par les méthodes connues d'activation des acides en utilisant des agents de couplages tels que carbonyldiimidazole ou bien encore, et ce de façon non limitative, le 1-hydroxybenzotriazole (HOBt) ou le Benzotriazol-1-yl-oxytripyrrolidino-phosphonium hexafluorophosphate (PyBOP).

Selon un autre mode de réalisation, lorsque R3 représente un groupe alkyle substitué par un groupe de formule -NR11-S(O)ₚ-R15, tel que défini en formule générale (I), le procédé selon l'invention comprend l'étape suivante : 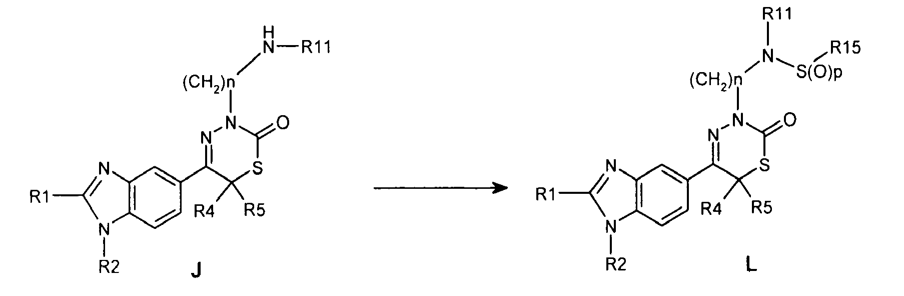 dans lesquels R1, R2, R4, R5, R11, R15, n et p sont tels que décrits précédemment.

Le composé L peut être obtenu par action d'un halogénure de sulfonyle convenablement choisi de formule

Hal-S(O)ₚ-R15

dans laquelle Hal représente un atome d'halogène et p est défini comme précédemment, par exemple un chlorure de sulfonyle tel que, et ce à titre d'exemple, le chlorure de benzènesulfonyle sur le composé J en présence d'une base organique telle que, et ce de façon non limitative, la triéthylamine, la pyridine, la diisopropyleéthyleamine dans un solvant inerte vis-à-vis de la réaction tel que l'acétonitrile, le toluène, le dichlorométhane ou bien encore un éther tel que le tétrahydrofurane ou le dioxane. On peut également utiliser une base minérale telle que, et ce de façon non limitative, l'hydrogénocarbonate de sodium ou le carbonate de césium. La réaction peut être conduite à une température comprise entre -10°C et la température d'ébullition du solvant utilisé ; dans le cas présent la réaction peut être conduite à une température proche de la température ambiante.

Selon un autre mode de réalisation, lorsque R3 représente un groupe alkyle substitué par un groupe de formule -NR11-C(O)-NR12R13, tel que défini en formule générale (I), le procédé selon l'invention comprend l'étape suivante : 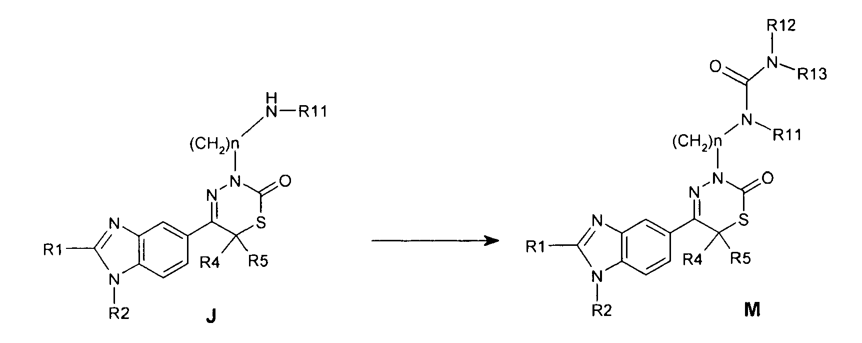 dans lesquels R1, R2, R4, R5, R11, R12, R13 et n sont tels que décrits précédemment.

Dans le cas où R12 est un hydrogène et R13 est différent d'un hydrogène, le composé M peut être obtenu par réaction du composé J avec un isothiocyanate convenablement choisi de formule

R13-N=C=O

dans un solvant de type éther tel que le tétrahydrofurane ou le dioxane à une température comprise entre 0°C et la température d'ébullition du solvant utilisé.

Dans le cas ou R12 et R13 sont tous les deux différents d'un hydrogène, le composé M peut être obtenu à partir du composé J par action du carbonyldiimidazole afin d'obtenir le composé J1 : 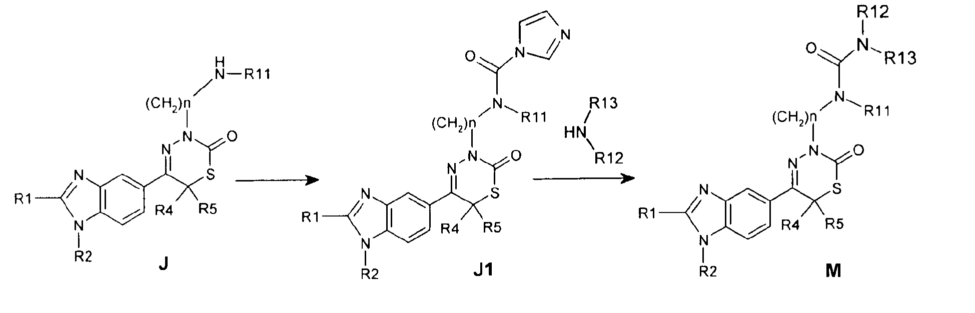 puis faire réagir le composé J1 obtenu avec une amine convenablement choisie pour obtenir le composé M.

La réaction peut être conduite dans un solvant tel que le dichlorométhane à une température allant de la température ambiante à la température d'ébullition du solvant utilisé.

Selon un autre mode de réalisation, lorsque R3 représente un groupe alkyle substitué par un groupe OH, le procédé selon l'invention comprend les étapes suivantes : 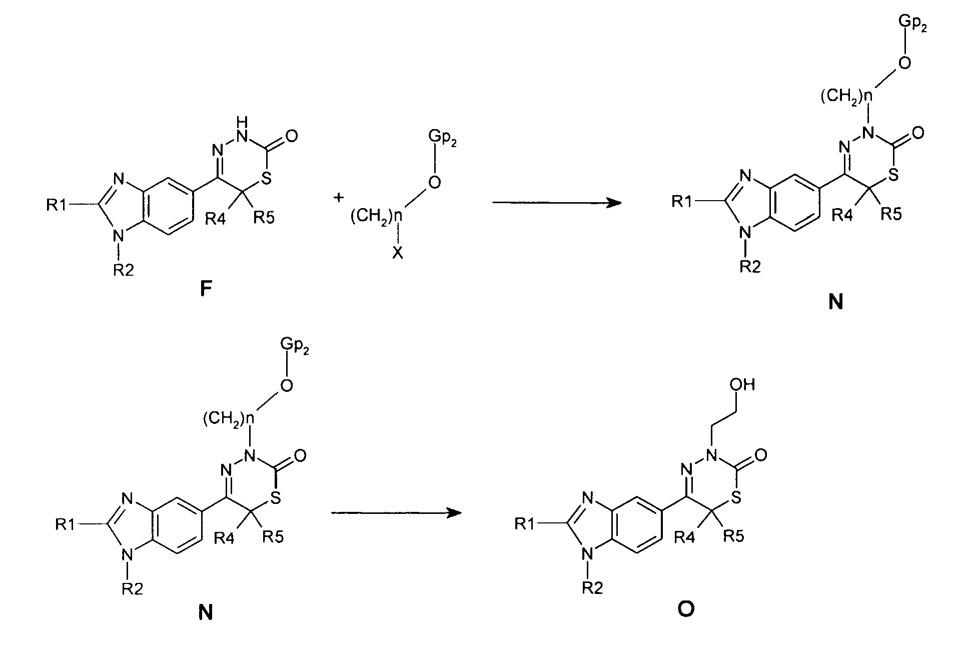 dans lesquels R1, R2, R4, R5, et n sont tels que décrits précédemment et où le groupe protecteur Gp2 peut être choisi parmi ceux utilisés communément dans la pratique de la synthèse organique pour la protection des alcools.

Dans des conditions de réaction similaires ou légérement modifiées, de même que pour le composé G, le composé N peut être obtenu par réaction du composé F avec, par exemple, un halogénure d'akyle, ramifié ou non, porteur dans ce cas d'une fonction alcool protégée par un groupe protecteur tel que, et ce de façon non limitative un groupe tétrahydropyranyle. L'alcool peut être également protégé sous forme d'éther silylé.

Le composé O peut être obtenu à partir du composé N en utilisant les méthodes connues de déprotection. On peut lorsque l'alcool est protégé par un groupe tétrahydropyranyle utiliser une solution d'acide chlorhydrique dans un solvant de type alcool tel que le méthanol à une température comprise entre 0°C et la température d'ébullition du solvant utilisé. Dans ce cas, on peut conduire la réaction à température ambiante le temps de réaction pouvant être compris entre 30 mn et 24 h.

De façon avantageuse, on peut également utiliser une solution d'acide chlorhydrique dans un solvant tel que le dioxane à une température comprise entre 0°C et la température d'ébullition du solvant utilisé.

Les composés de formule (I) dans lesquels le groupe R3 représente un groupe fonctionnel alternatif peuvent être obtenus à partir des intermédiaires F, G, I, J, K, L, M, N ou O, par application ou adaptation de méthodes connues de l'homme de l'art, notamment celles décrites par Larock dans Comprehensive Organic Transformations, VCH Pub 1989 ou celles décrites dans les exemples qui suivent.

Le composé de formule F peut être obtenu par l'un ou l'autre des modes de réalisations suivants :

Selon un premier mode de réalisation, le procédé de préparation du composé de formule F comprend les étapes suivantes :

### Etape 1a

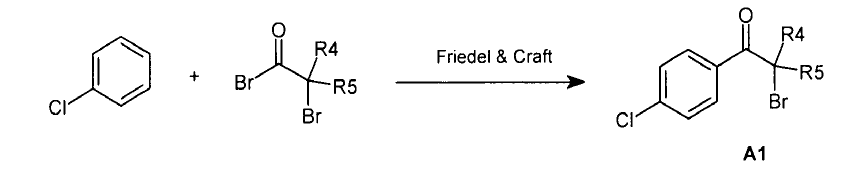

dans lesquels R4 et R5 sont tels que décrits précédemment ;
ou selon la variante 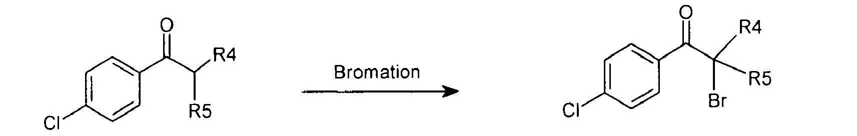 dans lesquels R4 et R5 sont tels que décrits précédemment ;

### Etape 2a

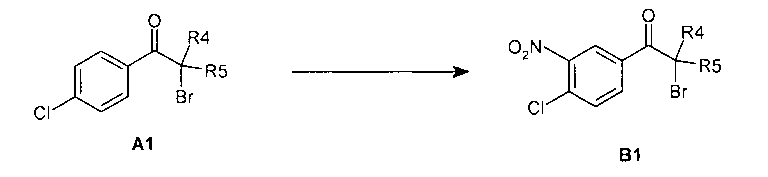

dans lesquels R4 et R5 sont tels que décrits précédemment ;

### Etape 3a

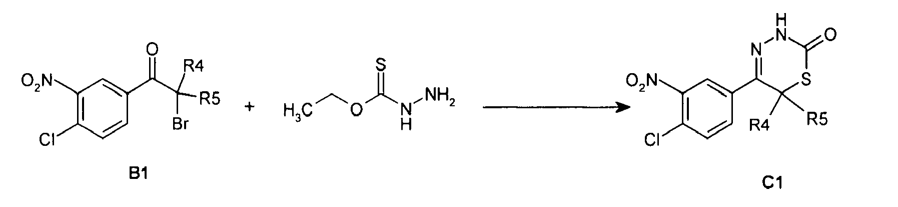

dans lesquels R4 et R5 sont tels que décrits précédemment ;

### Etape 4a

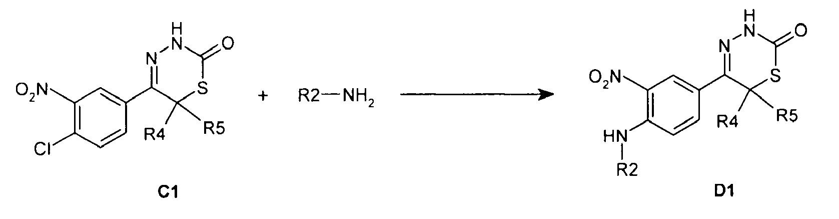

dans lesquels R2, R4 et R5 sont tels que décrits précédemment ;

### Etape 5a

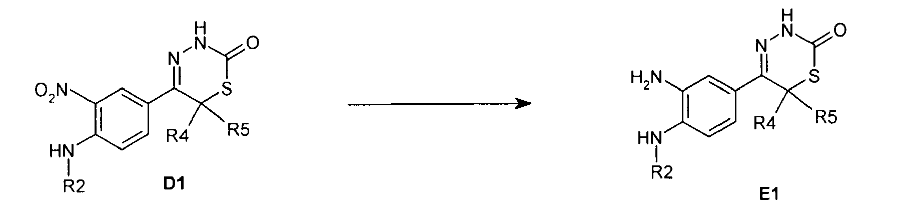

dans lesquels R2, R4 et R5 sont tels que décrits précédemment ;

### Etape 6a

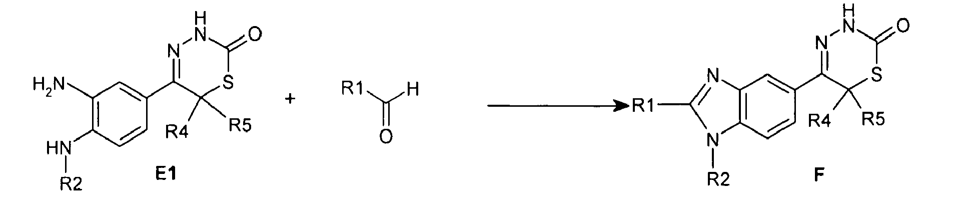

dans lesquels R1, R2, R4 et R5 sont tels que décrits précédemment ;
dans lesquelles étapes, plus précisément :
- Le composé **A1** peut être obtenu par action d'un halogénure d'acyle convenablement choisi sur du chlorobenzène en utilisant une réaction de Friedel et Crafts en présence d'un catalyseur tel que le chlorure d'aluminium.
   Le composé A1 peut également être obtenu par bromation d'une cétone convenablement choisie en utilisant les techniques connues en chimie organique.
   On peut utiliser le brome dans un solvant tel que, et ce de façon non limitative, l'acide acétique.
- La synthèse du composé **B1** peut être réalisée par action d'acide nitrique sur le composé A1. La réaction peut être conduite à une température allant de - 30°C à 0°C, de préférence de -25°C à 15°C, sur une période pouvant aller de 10 mn à 2 h.
- Le composé **C1** peut être obtenu par cyclisation du composé B1 avec le *O*-éthylhydrazinecarbothioate dans un solvant de type alcool tel que tel l'éthanol ou bien dans un solvant aprotique tel que l'acétonitrile. La réaction peut être conduite à une température allant de la température ambiante à la température d'ébullition du solvant utilisé, de préférence à la température débullition du solvant utilisé.
- Le composé **D1** peut être obtenu par réaction du composé C1 avec une amine convenablement choisie. La réaction peut être conduite dans un solvant tel que l'acétonitrile en présence d'une base minérale telle que le carbonate de sodium, le carbonate de potassium ou encore l'hydrogénocarbonate de sodium ou bien en présence d'une base organique telle que la triéthylamine ou bien encore en présence d'un excès de la base mise en réaction. La température de réaction peut être comprise entre 0°C et la température d'ébullition du solvant utilisé.
- Le composé **E1** peut être obtenu à partir du composé D1 par réduction du groupe nitro. Cette réduction sera effectuée en utilisant les méthodes connues de réduction ; on peut citer notamment l'hydrogénation en présence d'un catalyseur tel que le palladium sur charbon ou le nickel de Raney dans un solvant de type alcool tel que le méthanol à une température comprise entre 10°C et la température d'ébullition du solvant considéré.
   Cette réduction peut être menée à une pression allant de la pression atmosphérique à une pression de 100 bars.
   Dans le cas présent, on utilisera de façon avantageuse une réduction par utilisation d'un métal tel que le zinc dans un solvant tel que l'acide acétique.

Selon un second mode de réalisation, le procédé de préparation du composé de formule F comprend les étapes suivantes :

### Etape 1 b

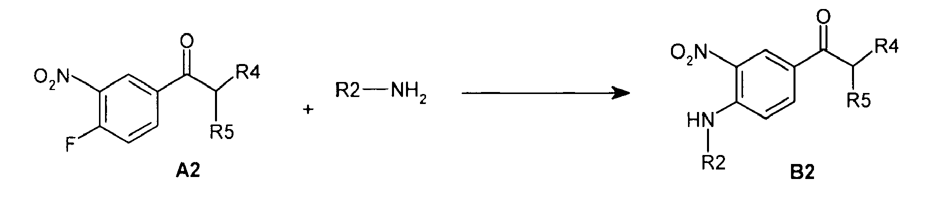

dans lesquels R2, R4 et R5 sont tels que décrits précédemment ;

### Etape 2b

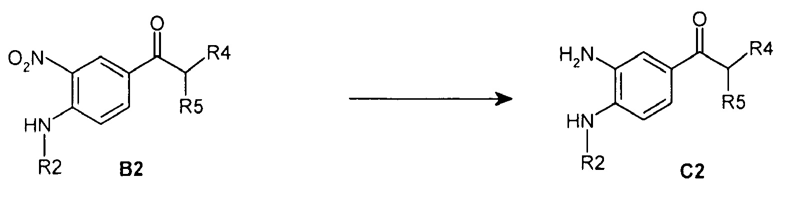

dans lesquels R2, R4 et R5 sont tels que décrits précédemment ;

### Etape 3b

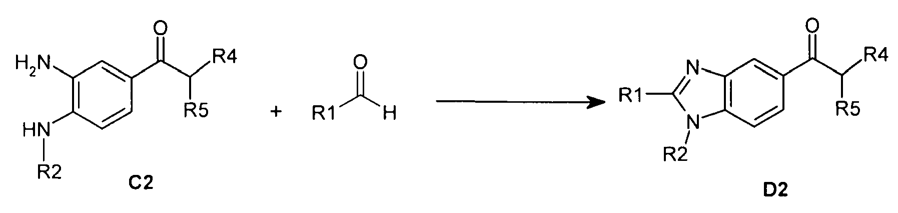

dans lesquels R1, R2, R4 et R5 sont tels que décrits précédemment.

### Etape 4b

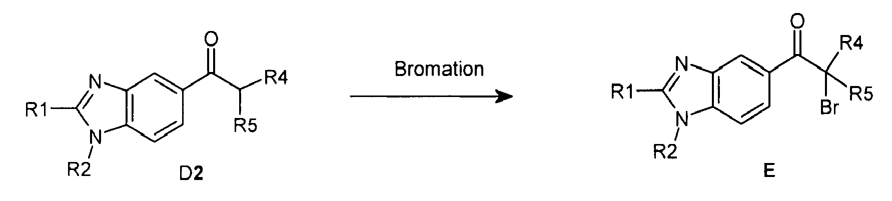

dans lesquels R1, R2, R4 et R5 sont tels que décrits précédemment ;

### Etape 5b

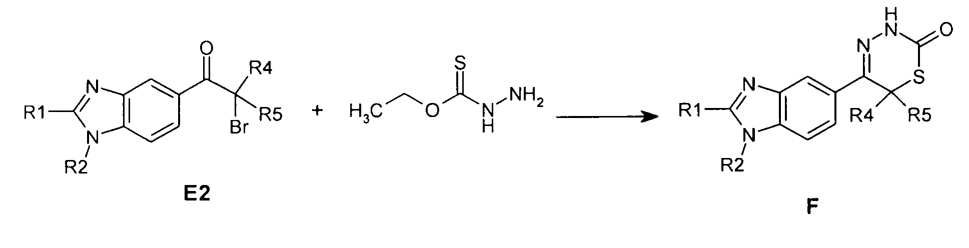

dans lesquels R1, R2, R4 et R5 sont tels que décrits précédemment ;
dans lesquelles étapes, plus précisément :
- Le composé **B2** peut être obtenu par réaction du composé A2 avec une amine convenablement choisie. La réaction peut être conduite dans un solvant tel que l'acétonitrile en présence d'une base minérale telle que, et ce de manière non limitative, le carbonate de sodium, le carbonate de potassium ou encore l'hydrogénocarbonate de sodium, ou bien en présence d'une base organique telle que la triéthylamine. On peut également conduire la réaction en présence d'un excès de la base mise en réaction. La température de réaction peut être comprise entre 0°C et la température d'ébullition du solvant utilisé.
- Le composé **C2** peut être obtenu à partir du composé B2 par réduction du groupe nitro. Cette réduction sera effectuée en utilisant les méthodes connues de réduction. On peut citer notamment l'hydrogénation en présence d'un catalyseur tel que le palladium sur charbon dans un solvant de type alcool tel que le méthanol, ou dans un solvant de type éther tel que le dioxane, à une température comprise entre 10°C et la température d'ébullition du solvant considéré. Cette réduction peut être menée à une pression allant de la pression atmosphérique à une pression de 100 bars.
- Le composé **D2** peut être obtenu par réaction du composé C2 avec un aldéhyde convenablement choisi afin d'obtenir le groupe benzimidazole par cyclisation.
   La réaction peut être conduite dans un solvant tel que la N-méthylpyrrolidone en présence de disulfite de sodium, à une température comprise entre la température ambiante et la température d'ébullition du solvant utilisé. De manière préférée, la réaction sera effectuée à une température proche de la température d'ébullition du solvant utilisé pendant une durée pouvant aller de 1H à 24 h.
- Le composé **E2** sera obtenu par halogénation sélective du composé D2 en utilisant un agent bromant tel que le brome dans un solvant tel que l'acide acétique.
- Le composé F peut être obtenu par cyclisation du composé E2 avec le *O-*éthyl hydrazinecarbothioate dans un solvant de type alcool tel que l'éthanol ou bien dans un solvant aprotique tel que l'acétonitrile. La réaction peut être conduite à une température allant de la température ambiante à la température d'ébullition du solvant utilisé, de préférence à la température débullition du solvant utilisé.

Eventuellement ledit procédé peut également comprendre l'étape consistant à isoler le produit obtenu.

Dans les réactions décrites ci-après, il peut être nécessaire de protéger les groupes fonctionnels réactifs, par exemples les groupes hydroxy, amino, imino, thio, carboxy, lorsqu'ils sont souhaités dans le produit final, pour éviter leur participation indésirable dans les réactions. Les groupes de protection traditionnels peuvent être utilisés conformément à la pratique standard, pour des exemples voir T.W. Green et P.G.M. Wuts dans Protective Groups in Organic Chemistry , John Wiley and Sons, 1991 ; J.F.W. McOmie in Protective Groups in Organic Synthesis, Plenum Press, 1973.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention peuvent contenir des centres asymétriques. Ces centres asymétriques peuvent être indépendamment en configuration R ou S. Il apparaîtra à l'homme du métier que certains composés utiles selon l'invention peuvent également présenter une isomérie géométrique. On doit comprendre que la présente invention comprend des isomères géométriques individuels et des stéréoisomères et des mélanges de ceux-ci, incluant des mélanges racémiques, de composés de formule (I) ci-dessus. Ce type d'isomères peuvent être séparés de leurs mélanges, par l'application ou l'adaptation de procédés connus, par exemple des techniques de chromatographie ou des techniques de recristallisation, ou ils sont préparés séparément à partir des isomères appropriés de leurs intermédiaires.

Aux fins de ce texte, il est entendu que les formes tautomériques sont comprises dans la citation d'un groupe donné, par exemple thio/mercapto ou oxo/hydroxy.

Les sels d'additions acides sont formés avec les composés utiles selon l'invention dans lesquels une fonction de base tels qu'un groupe amino, alkylamino ou dialkylamino est présente. Les sels d'addition acide pharmaceutiquement acceptables, c'est-à-dire non toxiques, sont préférés. Les sels sélectionnés sont choisis de façon optimale pour être compatibles avec les véhicules pharmaceutiques habituels et adaptés pour l'administration orale ou parentérale. Les sels d'addition acide des composés utiles selon cette invention peuvent être préparés par réaction de la base libre avec l'acide approprié, par l'application ou l'adaptation de procédés connus. Par exemples, les sels d'addition acide des composés utiles selon cette invention peuvent être préparés soit en dissolvant la base libre dans de l'eau ou dans une solution aqueuse alcoolisée ou des solvants adaptés contenant l'acide approprié et en isolant le sel en évaporant la solution, ou en faisant réagir la base libre et l'acide dans un solvant organique, auquel cas le sel se sépare directement ou peut être obtenu par concentration de la solution. Parmi les acides adaptés pour l'usage dans la préparation de ces sels on trouve acide chlorhydrique, acide bromhydrique, acide phosphorique, acide sulfurique, divers acides carboxyliques et sulfoniques organiques, tels que acide acétique, acide citrique, acide propionique, acide succinique, acide benzoïque, acide tartrique, acide fumarique, acide mandélique, acide ascorbique, acide malique, acide méthanesulfonique, acide toluène-sulfonique, acides gras, adipate, alginate, ascorbate, aspartate, benzènesulfonate, benzoate, propionate de cyclopentane, digluconate, dodécylsulfate, bisulfate, butyrate, lactate, laurate, sulfate de lauryle, malate, hydroiodide, 2-hydroxy-éthanesulfonate, glycérophosphate, picrate, pivalate, pamoate, pectinate, persulfate, 3-phénylpropionate, thiocyanate, 2-naphtalène-sulfonate, undécanoate, nicotinate, hemisulfate, heptonate, hexanoate, camphorate, camphersulfonate et autres.

Les sels d'addition acide des composés utiles selon cette invention peuvent être régénérés à partir des sels par l'application ou l'adaptation de procédés connus. Par exemple, les composés parents utiles selon l'invention peuvent être régénérés à partir de leurs sels d'addition acide par traitement avec un alkali, par exemple une solution de bicarbonate de sodium aqueuse ou une solution d'ammoniac aqueuse.

Les composés utiles selon cette invention peuvent être régénérés à partir de leurs sels d'addition de base par l'application ou l'adaptation de procédés connus. Par exemple, les composés parents utiles selon l'invention peuvent être régénérés à partir de leurs sels d'addition de base par le traitement avec un acide, par exemple un acide chlorhydrique.

Les sels d'addition de base peuvent être formés lorsque le composé utile selon l'invention contient un groupe carboxyle, ou un bioisostère suffisamment acide. Les bases qui peuvent être utilisées pour préparer les sels d'addition de base comprennent de préférence celle qui produisent, lorsqu'elles sont associées à un acide libre, des sels pharmaceutiquement acceptables, c'est-à-dire des sels dont les cations ne sont pas toxiques pour le patient dans les doses pharmaceutiques des sels, de sorte que les effets inhibiteurs bénéfiques inhérents à la base libre ne soient pas annulés par les effets secondaires imputables aux cations. Les sels pharmaceutiquement acceptables, comprenant ceux dérivés des sels de métal alcalino-terreux, dans la portée de l'invention comprennent ceux dérivés des bases suivantes : hydrure de sodium, hydroxyde de sodium, hydroxyde de potassium, hydroxyde de calcium, hydroxyde d'aluminium, hydroxyde de lithium, hydroxyde de magnésium, hydroxyde de zinc, ammoniac, éthylènediamine, N-méthyl-glucamine, lysine, arginine, ornithine, choline, N,N'-dibenzylethylenediamine, chloroprocaïne, diéthanolamine, procaïne, N-benzyl-phénéthylamine, diéthylamine, pipérazine, tris(hydroxy-methyl)-aminométhane, hydroxyde de tétraméthylammonium et analogues.

Les composés utiles selon la présente invention peuvent être facilement préparés, ou formés pendant le processus de l'invention, sous forme de solvates (par exemple hydrates). Les hydrates des composés utiles selon la présente invention peuvent être facilement préparés par la recristallisation d'un mélange de solvant aqueux/organique, en utilisant des solvants organiques tels que dioxan, tétrahydrofuranne ou méthanol.

Les produits de bases ou les réactifs utilisés sont disponibles commercialement et/ou peuvent être préparés par l'application ou l'adaptation de procédés connus, par exemple des procédés tels que décrits dans les Exemples de Référence ou leurs équivalents chimiques évidents.

Les composés de l'invention tels que définis ci-dessus présentent une activité hypoglycémiante et, à ce titre, sont utiles dans le traitement et/ou la prévention des pathologies associées au syndrome d'insulinorésistance.

En effet, l'insulinorésistance se caractérise par une réduction de l'action de l'insuline (cf. Presse Médicale (1997), 26(14), 671-677) et est impliquée dans un nombre important d'états pathologiques, tel que le diabète et plus particulièrement le diabète non insulinodépendant (diabète de type Il ou NIDDM), la dyslipidémie, l'obésité, l'hypertension artérielle, ainsi que certaines complications microvasculaires et macrovasculaires comme l'athérosclérose, les rétinopathies et les neuropathies.

À ce sujet, on se rapportera par exemple à Diabetes, 37, (1988), 1595-1607 ; Journal of Diabetes and its complications 12, (1998), 110-119 ou Horm. Res., 38, (1992), 28-32.

La présente invention a donc également pour objet des compositions pharmaceutiques comprenant, à titre de principe actif, au moins un composé selon l'invention.

Les compositions pharmaceutiques selon l'invention peuvent être présentées sous des formes destinées à l'administration par voie parentérale, orale, rectale, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les excipients acceptables sur le plan pharmaceutique, comme par exemple les dérivés de la cellulose ou de la cellulose microcristalline, les carbonates alcalino-terreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

Par exemple, si les composés selon la présente invention sont administrés par voie orale, sous forme de comprimés nus ou enrobés, de dragées, de capsules, de gélules, de pilules, de cachets ou de poudres, la posologie peut varier entre environ 0,1 mg/kg et environ 100 mg/kg, de préférence entre environ 0,5 mg/kg et environ 50 mg/kg, de préférence encore entre 1 mg/kg et 10 mg/kg, de manière tout à fait préférée entre environ 2 mg/kg et environ 5 mg/kg.

Si l'on considère que le poids du patient à traiter peut varier entre 10 kg et 100 kg et selon la posologie mentionnée ci-dessus, les prises journalières peuvent être comprises entre environ 1 à 10 mg/jour et environ 1000 à 10000 mg/jour, de préférence entre environ 5 à 50 mg/jour et environ 500 à 5000 mg/jour, de préférence encore entre environ 10 à 100 mg/jour et environ 100 à 1,000 mg/jour et de manière tout à fait préférée entre environ 20 à 200 mg/jour et environ 50 à 500 mg/jour.

Comme indiqué ci-dessus, les formulations de la présente invention convenable pour une administration orale peuvent se présenter sous forme de doses individuelles, telles que des comprimés, cachets ou dragées, chacune d'elles contenant une quantité prédéterminée de matière active; les formulations peuvent également se présenter sous forme de poudre ou de granulés, sous forme de solution ou de suspension dans un milieu aqueux ou non-aqueux, ou encore sous forme d'émulsion liquide de type huile-dans-eau ou sous forme d'émulsion liquide de type eau-dans-huile. La matière active peut également être administrée sous forme de bol alimentaire, de pâte ou d'électuaire.

Dans le diabète non insulinodépendant, chez l'homme, l'hyperglycémie est la résultante de deux défauts majeurs : une altération de la sécrétion d'insuline et une diminution de l'efficacité de l'insuline au niveau de trois sites à savoir le foie, les muscles et le tissu adipeux.

Les composés de la présente invention, en inhibant la gluconéogenèse par inhibition de l'enzyme clé fructose-1,6-bisphosphatase, sont donc susceptibles d'améliorer la glycémie des patients diabétiques non insulinodépendants.

Ainsi, et selon un autre aspect, la présente invention concerne l'utilisation d'au moins un composé de formule générale (I), ses éventuelles formes tautomères, ses éventuels énantiomères, diastéréoisomères, épimères et sels organiques ou minéraux, ainsi que ses « pro-drogues », pour le traitement ou la prévention de pathologies liées à un stockage excessif de glycogène ou encore des maladies telles que les maladies cardiovasculaires, y compris l'athérosclérose, l'accident ischémique du myocarde, pour le traitement ou la prévention du diabète, notamment de type Il et des maladies associées aux troubles du métabolisme, telles que l'hypercholestérolémie, la dyslipidémie, l'hyperlipidémie, qui sont exacerbées par l'hyperinsulinémie et l'hyperglycémie, le traitement et la prévention de l'obésité, ou encore les complications du diabète telles que la néphropathie, rétinopathie, neuropathie.

Selon un autre objet, la présente invention concerne également l'utilisation d'un composé de formule générale (I) pour la fabrication d'un médicament pour inhiber ou limiter la production hépatique de glucose.

Les exemples suivants illustrent l'invention, sans toutefois la limiter. Les produits de départs utilisés sont des produits connus ou préparés selon des modes opératoires connus.

Les pourcentages sont exprimés en poids sauf mention contraire.

Les composés ont été caractérisés notamment par les techniques analytiques suivantes :
Les spectres RMN ont été effectués à l'aide d'un spectromètre RMN Brücker Avance DPX 300 MHz.
Les masses ont été déterminées par H.P.L.C., couplée à un détecteur de masse Agilent Série 1100.
Les points de fusion (pf) ont été mesurés sur un banc de type Köfler Leica VMBH.

### Exemple 1 : 2-bromo-1-(4-chlorophényl)propan-1-one

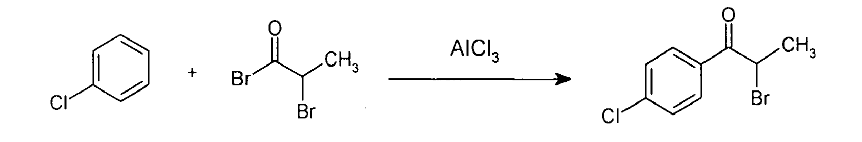

A 200 ml de dichlorométhane, on ajoute 23,7 g (0,176M) de chlorure d'aluminium. On refroidit à 10°C et sous agitation on ajoute goutte à goutte 10 g (0,089M) de chlorobenzène. On agite pendant une 1 heure à 15°C puis on coule goutte à goutte 9,2 ml (0,089M) de bromure de 2-bromopropionyle. Le milieu réactionnel est ensuite maintenu sous agitation pendant 16 h à température ambiante. On coule ensuite le milieu réactionnel sur un mélange glace/eau. La phase organique est décantée, lavée deux fois à l'eau déminéralisée puis séchée sur sulfate de sodium. Après évaporation sous vide, on obtient une huile qui cristallise pour donner 21 g de 2-bromo-1-(4-chloro phényl)propan-1-one. Rendement : 95,5%
RMN ¹H (300 MHz / DMSO-d6) δ:1,75(d, 3H), 5,78(q, 1 H), 7,62(d, 2H), 8,05(d, 2H)

### Exemple 2 : 1-(4-chloro-3-nitrophényl)-2-bromopropan-1-one

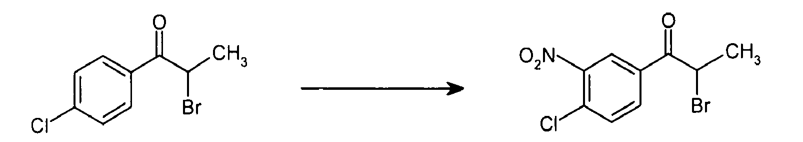

A 80 ml d'acide nitrique fumant à -20°C on ajoute par portions 20,5 g (0,08M) de 2-bromo-1-(4-chlorophényl)propan-1-one. Après addition, on maintient le milieu réactionnel sous agitation à -20°C pendant 15 mn. Le milieu réactionnel est ensuite versé sur de la glace et on extrait avec 300 ml de dichlorométhane.

Les phases organiques réunies sont lavées deux fois à l'eau puis séchées sur sulfate de sodium et concentrées sous vide. L'huile obtenue est purifiée par chromatographie sur silice en utilisant du dichlorométhane comme éluant pour obtenir 22,5 g de 1-(4-chloro-3-nitrophényl)-2-bromopropan-1-one sous forme d'une huile incolore
Rendement : 93,5%
RMN ¹H (300 MHz / DMSO-d6) δ: 1,79(d,3H), 5,87(q, 1 H), 7,98(d, 1 H), 8,29(dd, 1 H), 8,65(s, 1 H)

### Exemple 3 : 5-(4-chloro-3-nitrophényl)-6-méthyl-3,6-dihydro-2H-1,3,4-thiadiazin-2-one

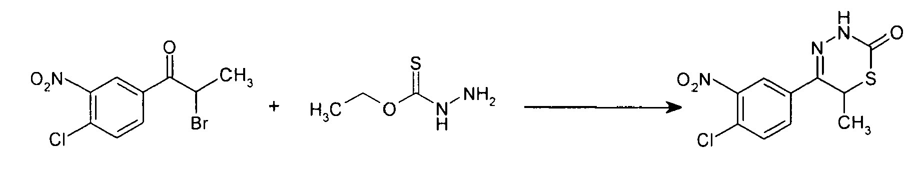

22 g (75 mM) de 1-(4-chloro-3-nitrophényl)-2-bromopropan-1-one et 8 ml (75 mM) de *O*-éthylhydrazinecarbothioate dans 150 ml d'éthanol sont portés au reflux pendant 16 h sous agitation. Le milieu réactionnel est ensuite concentré sous vide et le résidu obtenu est repris dans de l'eau. La phase aqueuse est extraite deux fois avec de l'acétate d'éthyle et les phases organiques réunies sont lavées avec de l'eau puis séchées sur du sulfate de sodium anhydre. Le solvant est évaporé sous vide pour donner une huile qui est purifiée sur une colonne de silice en utilisant un mélange dichlorométhane/acétone (95/5) comme éluant. On obtient 5g de 5-(4-chloro-3-nitro phényl)-6-méthyl-3,6-dihydro-2*H*-1,3,4-thiadiazin-2-one.
Rendement: 23%
RMN ¹H (300 MHz / CDCl₃) δ: 1,67(d, 3H), 4,23(q, 1 H), 7,62(d, 1 H), 7,88(dd, 1 H), 8,23(s, 1 H), 9,04(s, 1 H)

### Exemple 4 : 5-[4-(isobutylamino)-3-nitrophényl]-6-méthyl-3,6-dihydro-2H-1,3,4-thiadiazin-2-one

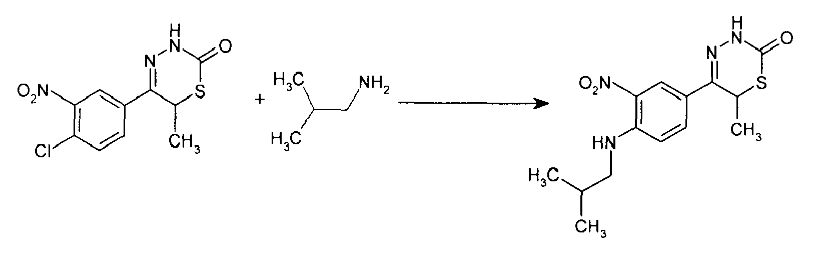

A 15 ml d'acétonitrile, on ajoute 2,9 g (10,1 mM) de 5-(4-chloro-3-nitrophényl)-6-méthyl-3,6-dihydro-2*H*-1,3,4-thiadiazin-2-one et 5 ml (50 mM) d'isobutylamine. Le milieu réactionnel est porté à 60°C pendant 20 h sous agitation et est ensuite concentré sous vide. Le résidu obtenu est purifié sur silice en utilisant du dichlorométhane comme éluant. Le produit obtenu est repris et cristallisé dans du diéthyle oxyde. Le solide est filtré et lavé pour donner 2,6 g de 5-[4-(isobutylamino)-3-nitrophényl]-6-méthyl-3,6-dihydro-2*H*-1,3,4-thiadiazin-2-one.
Rendement : 80,6%
RMN ¹H (300 MHz / CDCl₃) δ: 1,05(d, 6H), 1,62(d, 3H), 2,02(m, 1 H), 3,18(t, 2H), 4,27(q, 1 H), 6,92(d, 1 H), 8,00(dd, 1 H), 8,4(d, 1 H), 8,46(s, 1H), 9,10(s, 1 H)

### Exemple 5 : 5-[3-amino-4-(isobutylamino)phényl]-6-méthyl-3,6-dihydro-2H-1,3,4-thiadiazin-2-one

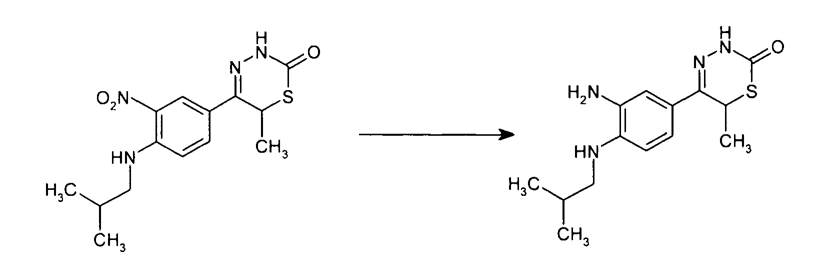

Sous agitation on ajoute 1,3 g (4,03 mM) de 5-{4-(isobutylamino)-3-nitrophényl}-6-méthyl-3,6-dihydro-2*H*-1,3,4-thiadiazin-2-one à 40 ml d'acide acétique. A la solution obtenue, en 45 mn, on ajoute par fractions, 1,7 g (26 mM) de zinc. On coule ensuite doucement le milieu réactionnel sur 300 ml d'une solution aqueuse saturée d'hydrogénocarbonate de sodium et 200 ml de dichlorométhane.

On termine la neutralisation par ajout d'hydrogénocarbonate de sodium. La phase organique est décantée, lavée à l'eau déminéralisée puis séchée sur sulfate de sodium anhydre. Après évaporation, on obtient 1,1g de 5-[3-amino-4-(isobutylamino)phényl]-6-méthyl-3,6-dihydro-2*H*-1,3,4-thiadiazin-2-one
Rendement: 93%.
RMN ¹H (300 MHz / CDCl₃) δ: 1,02(d, 6H), 1,63(d, 3H), 1,96(m, 1 H), 2,96(d, 2H), 3,4(s, 2H), 3,75(s, 1 H),4,25(q, 1 H), 6,60(d, 1H), 7,2(dd, 1 H), 7,28(s, 1 H), 9,27(s, 1 H)

### Exemple 6 : 5-[1-isobutyl-2-(3-méthoxyphényl)-1H-benzimidazol-5-yl]-6-méthyl-3,6-dihydro-2H-1,3,4-thiadiazin-2-one

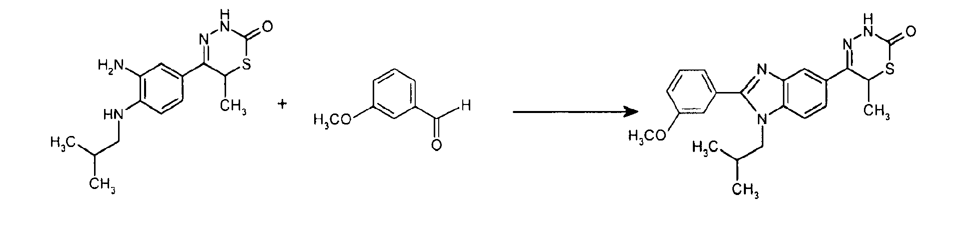

A 12 ml de 1-méthyl-2-pyrrolidone, on ajoute 560 mg (4,1 mM) de 4-méthoxy benzaldéhyde, 1,2 g (4,1 mM) de 5-[3-amino-4-(isobutylamino)phényl]-6-méthyl-3,6-dihydro-2*H*-1,3,4-thiadiazin-2-one et 780,2 mg (4,1 mM) de disulfite de sodium. On porte alors le milieu réactionnel à 110°C sous agitation pendant 3h. Le milieu réactionnel est ensuite coulé sur un mélange glace/eau.

On extrait avec de l'acétate d'éthyle, la phase organique est lavée avec de l'eau déminéralisée, puis séchée sur sulfate de sodium anhydre. Le solvant est évaporé sous vide pour donner une huile qui est purifiée sur une colonne de silice en utilisant un mélange heptane/acétate d'éthyle (80/20) comme éluant pour donner 960 mg de 5-[1-Isobutyl-2-(3-méthoxyphényl)-1*H*-benzimidazol-5-yl]-6-méthyl-3,6-dihydro-2*H*-1,3,4-thiadiazin-2-one.
Rendement: 57,3%
RMN ¹H (300 MHz / CDCl₃) δ: 0,68(d, 6H), 1,63(d, 3H), 2,04(m, 1 H), 3,81 (s, 3H), 4,07(d, 2H), 4,33(m, 1 H), 6,99(d, 1 H), 7,21(s, 1 H), 7,37(m, 2H), 7,75(m, 1 H), 8,09(s, 1 H), 9,47(s, 1 H)
Point de fusion: 100-105°C
C₂₂H₂₄N₄O₂S = 408,52
Spectrométrie de Masse M+1 = 409,1

Par une méthode similaire ou légèrement modifiée, on prépare les composés suivants :

### Exemple 6-2 :

### 5-[1-cyclopropylméthyl-2-(3-méthoxyphényl)-1H-benzimidazol-5-yl]-6-méthyl-3,6-dihydro-2H-1,3,4-thiadiazin-2-one

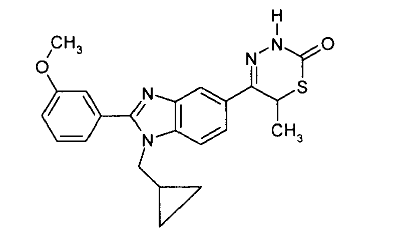

C₂₂H₂₂N₄O₂S = 406,5
Spectrométrie de masse M+1 = 407,2

### Exemple 6-3 :

### 5-[2-(3-méthoxyphényl)-1-méthyl-1H-benzimidazol-5-yl]-6-méthyl-3,6-dihydro-2H-1,3,4-thiadiazin-2-one

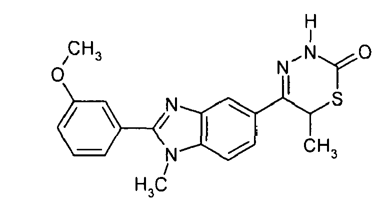

C₁₉H₁₈N₄O₂S = 366,4
Spectrométrie de masse M+1 = 367,1

### Exemple 6-4 :

### 5-[1-Benzyl-2-(4-méthoxyphényl)-1H-benzimidazol-5-yl]-6-méthyl-3,6-dihydro-2H-1,3,4-thiadiazin-2-one

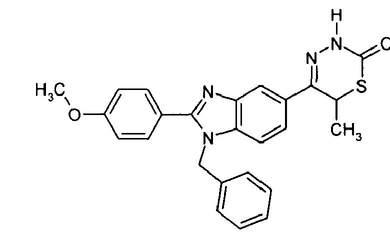

C₂₅H₂₂N₄O₂S = 442,53
Spectrométrie de masse M+1 = 443,0

### Exemple 6-5 :

### 5-[2-(2-méthoxyphényl)-1-méthyl-1H-benzimidazol-5-yl]-6-méthyl-3,6-dihydro-2H-1,3,4-thiadiazin-2-one

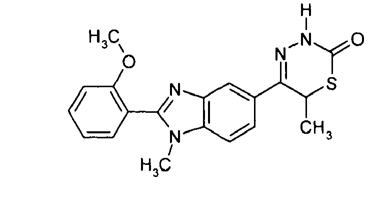

C₁₉H₁₈N₄O₂S = 366,44
Spectrométrie de masse M+1 = 367,1

### Exemple 6-6 :

### 5-[2-(3-fluorophényl)-1-méthyl-1H-benzimidazol-5-yl]-6-méthyl-3,6-dihydro-2H-1,3,4-thiadiazin-2-one

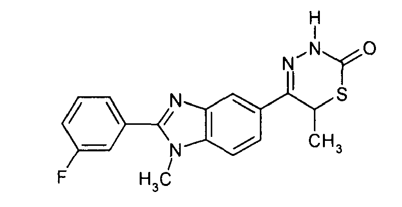

C₁₈H₁₅FN₄OS = 354,4
Spectrométrie de masse M+1 = 355,1

### Exemple 7 : 1-[4-(isobutylamino)-3-nitrophényl]éthanone

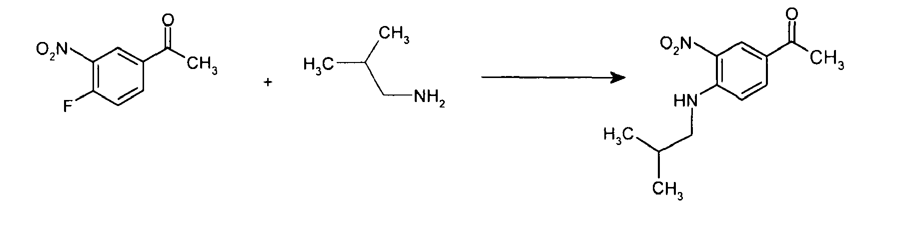

A 24,5 g (133,8 mM) de 1-(4-fluoro-3-nitrophényl)éthanone dans 100 ml d'acétonitrile et 11,2 g (133,8 mM) d'hydrogénocarbonate de sodium, on ajoute 26,6 ml (267,6 mM) d'isobutylamine. La réaction est exothermique, on maintient la température du milieu réactionnel inférieure à 50°C puis on agite pendant 1 h à température ambiante. On ajoute de l'eau déminéralisée, un solide précipite. Le solide est filtré et lavé à l'eau pour donner après séchage 31,6 g de 1-[4-(isobutylamino)-3-nitrophényl]éthanone avec un rendement quantitatif.
RMN ¹H (300 MHz / DMSO-d6) δ: 1,17(d, 6H), 2,16(m, 1 H), 2,71 (s, 3H), 3,46(t, 2H), 7,3(d, 1 H), 8,21 (d, 1 H), 8,97(s, 1 H)

### Exemple 8 : 1-[3-amino-4-(isobutylamino)phényl]éthanone

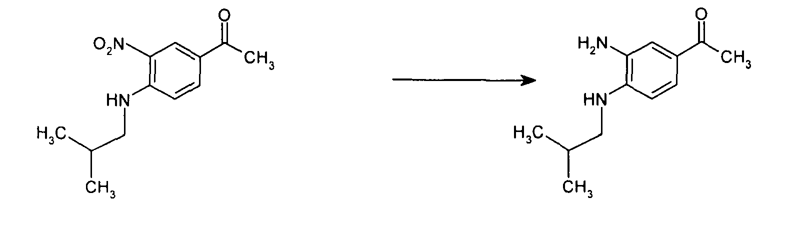

A une solution de 31,6g (134 mM) de 1-[4-(isobutylamino)-3-nitrophényl]éthanone dans 400 ml de méthanol, on ajoute 3,2 g de palladium sur charbon à 10%. Le milieu réactionnel est ensuite placé sous atmosphère d'hydrogène à pression ambiante sous agitation énergique pendant 1 H.On filtre le milieu réactionnel sur Celite et on évapore le solvant sous vide pour obtenir 27,6 g de 1-[3-amino-4-(isobutylamino)phényl] éthanone sous forme d'huile avec un rendement quantitatif.
RMN ¹H (300 MHz / DMSO-d6) δ: 1.16(d, 6H), 2,11 (m, 1 H) 2,58(s, 3H) 3,16(t, 2H), 4,97(s, 2H), 5.54(s, 1 H), 6.64(d, 1 H), 7.37(s, 1 H), 7.42(d, 1 H)

### Exemple 9 : 1-[1-(isobutyl)-2-(3-méthoxyphényl)-1H-benzimidazol-5-yl]éthanone

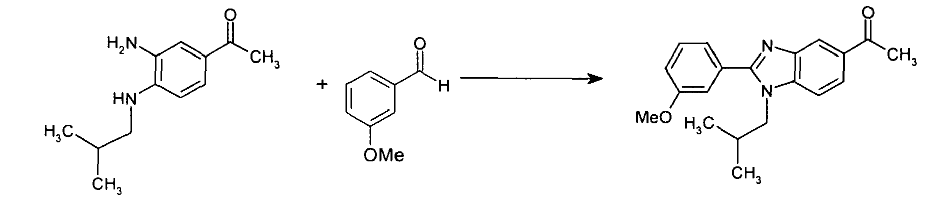

A 276 ml de 1-méthyl-2-pyrrolidone, on ajoute 27,6g (134 mM) de 1-[3-amino-4-(isobutylamino)phényl]éthanone, 16,3 ml (134 mM) de 3-méthoxybenzaldéhyde et 25,4g (134 mM) de disulfite de sodium. Le milieu réactionnel est porté à 130°C sous agitation pendant 18h, et après refroidissement, est ajouté à un mélange glace/eau. On extrait à l'acétate d'éthyle, et la phase organique est lavée à l'eau puis séchée sur sulfate de sodium anhydre. On évapore sous vide et le résidu est purifié sur une colonne de silice en utilisant un mélange dichlorométhane/acétone (90/10) comme éluant.

On obtient 32,1 g de 1-[1-isobutyl-2-(3-méthoxyphényl)-1*H*-benzimidazol-5-yl]étha none sous forme d'huile. Rendement: 74,4 %
RMN ¹H (300 MHz / DMSO-d6) δ: 1.07(d, 6H), 2.29(m, 1 H), 3.07(s, 3H), 4.25(s, 3H), 4.66(d, 2H), 7.57(dd, 1 H), 7,76(m, 2H), 7.87(t, 1 H), 8.15(d, 1 H), 8.36(d, 1 H), 8.79(s, 1 H)

### Exemple 10 : 2-bromo-1-[1-isobutyl-2-(3-méthoxyphényl)-1H-benzimidazol-5-yl]éthanone

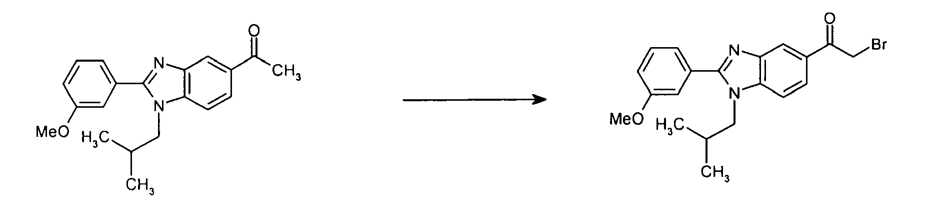

A une solution de 32,7 g (91,3 mM) de chlorhydrate de 1-[1-isobutyl-2-(3-méthoxy phényl)-1*H*-benzimidazol-5-yl]éthanone dans 330 ml d'acide acétique, on ajoute goutte à goutte 4,7 ml (91,4mM) de brome. On ajoute ensuite 100 ml de diéthyle oxyde et on on filtre le produit cristallisé. On lave avec du diéthyle oxyde et on sèche sous vide pour obtenir 35 g de chlorhydrate de 2-bromo-1-[1-isobutyl-2-(3-méthoxyphényl)-1*H*-benzimidazol-5-yl] éthanone.
Rendement : 63,2%
C₂₀H₂₁BrN₂O₂ = 401,3
Spectrométrie de masse M+1 = 403,1

### Exemple 11 : 5-[1-isobutyl-2-(3-méthoxyphényl)-1H-benzimidazol-5-yl]-3,6-dihydro-2H-1,3,4-thiadiazin-2-one

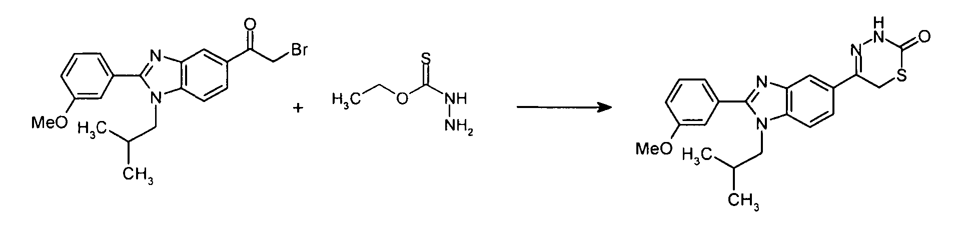

A une solution de 24 g (54,8 mM) de chlorhydrate de 2-bromo-1-[1-isobutyl-2-(3-méthoxyphényl)-1*H*-benzimidazol-5-yl]éthanone dans 500ml d'éthanol, on ajoute 6,3 ml (78,4 mM) de pyridine puis 8,2 g (78,4 mM) de *O*-éthyl hydrazinecarbothioate. Le milieu réactionnel est alors porté à 80°C sous agitation pendant 20h. On ajoute ensuite de l'eau et on extrait avec de l'acétate d'éthyle. La phase organique est lavée à l'eau puis séchée sur sulfate de sodium anhydre et évaporée sous vide. Le résidu est purifié sur une colonne de silice en utilisant un mélange dichlorométhane/méthanol (98/2). Le résidu obtenu après évaporation est repris dans du diéthyle oxyde pour donner après filtration 10 g de : 5-[-1-isobutyl-2-(3-méthoxyphényl)-1*H*-benzimidazol-5-yl]-3,6-dihydro-2*H*-1,3,4-thiadiazin-2-one sous forme d'un solide beige clair.
Rendement: 46,2%
RMN ¹H (300 MHz / DMSO-d6) δ: 0.65(d, 6H), 1.90(m, 1 H), 3.83(s, 3H), 4.20(d, 2H), 4.32(s, 2H), 7.14(dd, 1 H), 7.32(m, 2H), 7.48(t, 1 H), 7.77(m, 2H), 8.14(d, 1 H),11.56(s, 1 H)
Point de fusion: 207-209°C
C₂₁H₂₂N₄O₂S = 394,55
Spectrométrie de masse M+1 = 395,1

Par une méthode similaire ou légèrement modifiée, on prépare les composés suivants :

### Exemple 11-2 :

### 5-[2-(3-fluorophényl)-1-méthyl-1H-benzimidazol-5-yl]-3,6-dihydro-2H-1,3,4-thiadiazin-2-one

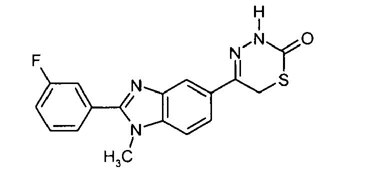

C₁₇H₁₃FN₄OS = 340,38
Spectrométrie de masse M+1 = 341,2

### Exemple 11-3 :

### 5-{1-méthyl-2-[3-(trifluorométhyl)phényl]-1H-benzimidazol-5-yl}-3,6-dihydro-2H-1,3,4-thiadiazin-2-one

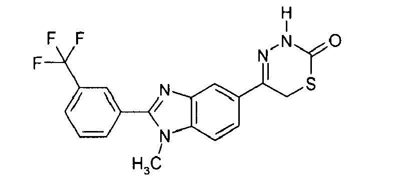

C₁₈H₁₃F₃N₄OS = 390,38
Spectrométrie de masse M+1 = 391,1

### Exemple 11-4 :

### 5-[1-éthyl-2-(3-fluorophényl)-1H-benzimidazol-5-yl]-3,6-dihydro-2H-1,3,4-thiadiazin-2-one

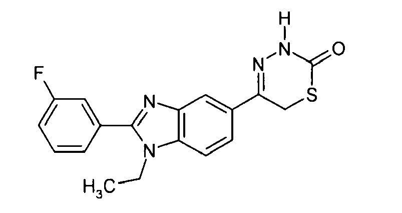

C₁₈H₁₅FN₄OS = 354,4
Spectrométrie de masse M+1 = 355,1
Point de fusion: 214-216°C

### Exemple 11-5 :

### 5-{1-méthyl-2-[3-(trifluorométhyl)phényl]-1H-benzimidazol-5-yl}-6-propyl-3,6 dihydro-2H-1,3,4-thiadiazin-2-one

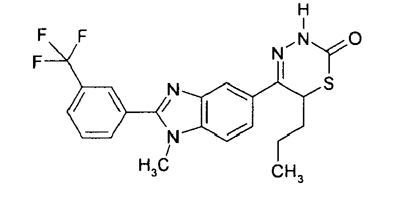

C₂₁H₁₉F₃N₄OS = 432,46
Spectrométrie de masse M+1 = 433,1
Point de fusion: 190-192 °C

### Exemple 11-6 :

### 5-[1-éthyl-2-(3-méthoxyphényl)-1H-benzimidazol-5-yl]-3,6-dihydro-2H-1,3,4-thiadiazin-2-one

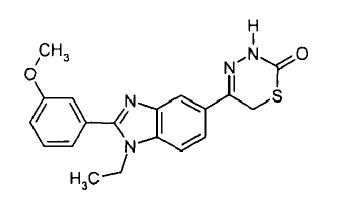

C₁₉H₁₈N₄O₂S = 366,44
Spectrométrie de masse M+1 = 367,1
Point de fusion : 221-223°C

### Exemple 12 : 3-Isopropyl-6-méthyl-5-{1-méthyl-2-[(3-trifluorométhyl)phényl]-1H-benzimidazol -5-yl}-3,6-dihydro-2H-1,3,4-thiadiazin-2-one

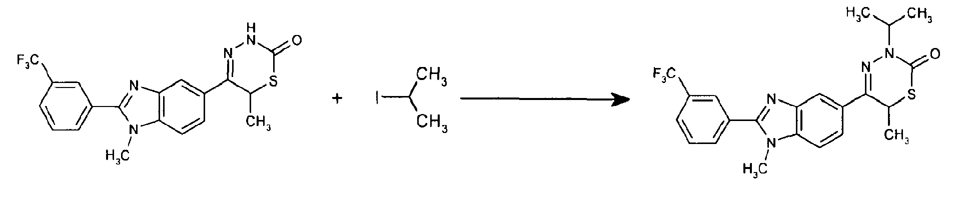

A 2 ml de diméthylformamide, on ajoute 200 mg (0,49 mM) de 6-méthyl-5-{1-méthyl-2-[(3-trifluorométhyl)phényl]-1*H*-benzimidazol-5-yl}-3,6-dihydro-2*H*-1,3,4-thiadiazin-2-one, 168,3 mg (0,99 mM) d'iodure d'isopropyle et 484 mg (1,48 mM) de carbonate de césium. Le milieu réactionnel est placé sous agitation à température ambiante pendant 16 h. On ajoute 5 ml d'eau et on extrait avec 5 ml d'acétate d'éthyle.

La phase organique est lavée à l'eau, séchée sur sulfate de sodium anhydre puis concentrée sous vide pour donner une huile qu'on purifie par chromatographie sur silice en utilisant un mélange dichlorométhane/acétone (98/2) comme éluant. On obtient 200 mg de 3-Isopropyl-6-méthyl-5-{1-méthyl-2-[(3-trifluorométhyl)phényl]-1*H*-benzimidazol-5-yl}-3,6-dihydro-2*H*-1,3,4-thiadiazin-2-one sous forme d'une huile.
Rendement: 90,5%
RMN ¹H (300 MHz / DMSO-d6) δ: 1,26(d, 3H), 1,36(d, 3H), 1,54(d, 3H), 3,98(s, 3H), 4,94(m, 2H), 7,95(m, 4H), 8, 25(m, 3H)
C₂₂H₂₁F₃N₄OS = 446,49
Spectrométrie de masse M+1 = 447,1

Par une méthode similaire ou légèrement modifiée, on prépare les composés suivants :

### Exemple 12-2 :

### 2-[5-[2-(3-méthoxyphényl)-1-méthyl-1H-benzimidazol-5-yl]-6-méthyl-2-oxo-2H-1,3,4-thiadiazin-3(6H)-yl]acétamide

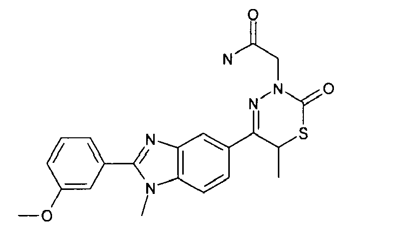

C₂₁H₂₁N₅O₃S = 423,49
Spectrométrie de masse M+1 = 424,1

### Exemple 12-3 :

### 5-[2-(3-fluorophényl)-1-méthyl-1H-benzimidazol-5-yl]-3-isopropyl-6-méthyl-3,6-dihydro-2H-1,3,4-thiadiazin-2-one

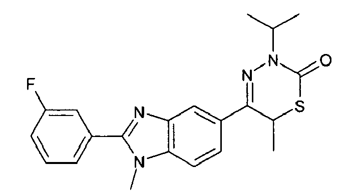

RMN ¹H (300 MHz / DMSO-d6) δ: 1,30(d,3H), 1,40(d, 3H), 1,58(d, 3H), 4,01 (s, 3H), 4,98(m, 2H), 7,51 (m, 1 H), 7,70(m, 1 H), 7,81 (m, 3H), 7,99(m, 1 H), 8,27(s, 1 H)
C₂₁H₂₁FNO₄S = 396,48
Spectrométrie de masse M+1 = 397,1

### Exemple 12-4 :

### 3-isobutyl-5-{1-méthyl-2-[3-(trifluorométhyl)phényl]-1H-benzimidazol-5-yl}-6-propyl-3,6-dihydro-2H-1,3,4-thiadiazin-2-one

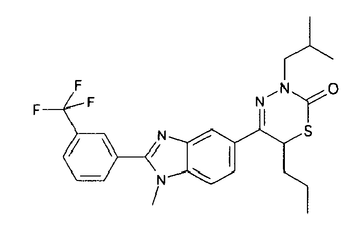

C₂₅H₂₇F₃N₄OS = 488,58
Spectrométrie de masse M+1 = 489,0

### Exemple 12-5 :

### 3-(cyclohexylméthyl)-5-[2-(2-furyl)-1-méthyl-1H-benzimidazol-5-yl]-3,6-dihydro-2H-1,3,4-thiadiazin-2-one

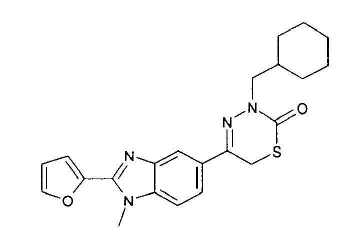

C₂₂H₂₄N₄O₂S = 408,53
Spectrométrie de masse M+1 = 409,2

### Exemple 12-6 :

### 5-[2-(3-fluorophényl)-1-méthyl-1H-benzimidazol-5-yl]-6-méthyl-3-propyl-3,6-dihydro-2H-1,3,4-thiadiazin-2-one

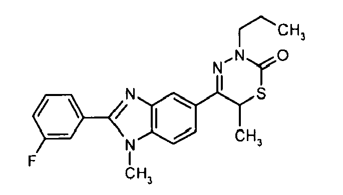

C₂₁H₂₁FN₄OS = 396,48
Spectrométrie de masse M+1 = 397,1

### Exemple 12-7 :

### 5-[1-cycloheptyl-2-(3-méthoxyphényl)-1H-benzimidazol-5-yl]-3-méthyl-3,6-dihydro-2H-1,3,4-thiadiazin-2-one

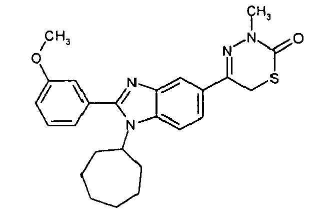

RMN ¹H (300 MHz / DMSO-d6) δ: 1,59(m, 6H), 1,86(m, 2H), 2,10(m, 2H), 2,93(m, 2H), 3,48(s, 3H), 3,89(s, 3H), 4,43(s, 2H), 4,53(m, 1 H), 7,39(m, 3H), 7,66(t, 1 H), 8,02(d, 1 H), 8,16(d, 1 H), 8,24(s, 1 H)
C₂₅H₂₈N₄O₂S = 448,59
Spectrométrie de masse M+1 = 449,1

### Exemple 13 : tert-butyl 2-[5-[2-(3-fluorophényl)-1-méthyl-1H-benzimidazol-5-yl]-6-méthyl-2-oxo-2H-1,3,4-thiadiazin-3(6H)-yl]éthylcarbamate

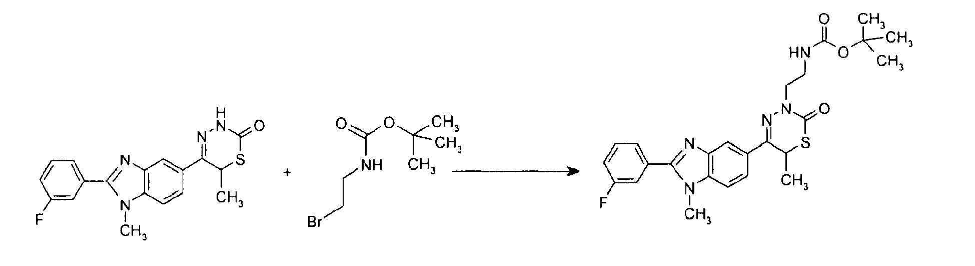

A 40 ml de diméthylformamide, on ajoute 4,9g (14mM) de 5-[2-(3-fluorophényl)-1-méthyl)-1*H*-benzimidazol-5-yl]-6-méthyl-3,6-dihydro-2*H*-1,3,4-thiadiazin-2-one, 3,4g 15 mM) de bromure de 2-(Boc-amino)éthyle et 16,6g (42mM) de carbonate de césium. On agite à température ambiante pendant 20h puis on ajoute 200 ml d'eau déminéralisée. Formation d'un précipité blanchâtre que l'on filtre. Après lavage à l'eau et séchage, on obtient 6,3g de *tert*-butyl 2-[5-[2-(3-fluorophényl)-1-méthyl-1H-benzimidazol-5-yl]-6-méthyl-2-oxo-2*H*-1,3,4-thiadiazin-3 (6*H*)-yl]éthylcarbamate. Rendement: 90,8%
RMN ¹H (300 MHz / DMSO-d6) δ: 1.35(s, 9H), 1.56(d, 3H), 3.37(m, 2H), 3.83(m, 1 H), 3.97(s, 3H), 4.14(m, 1 H), 4.94(q, 1 H), 6.99(t, 1 H), 7.46(t, 1 H), 7.74(m, 4H), 7.91(d, 1 H), 8.22(s, 1 H)
C₂₅H₂₈FN₅O₃S = 497,59
Spectrométrie de masse M+1 = 498

### Exemple 14 : 3-(2-aminoéthyl)-5-[2-(3-fluorophényl)-1-méthyl-1H-benzimidazol-5-yl]-6-méthyl-3,6-dihydro-2H-1,3,4-thiadiazin-2-one

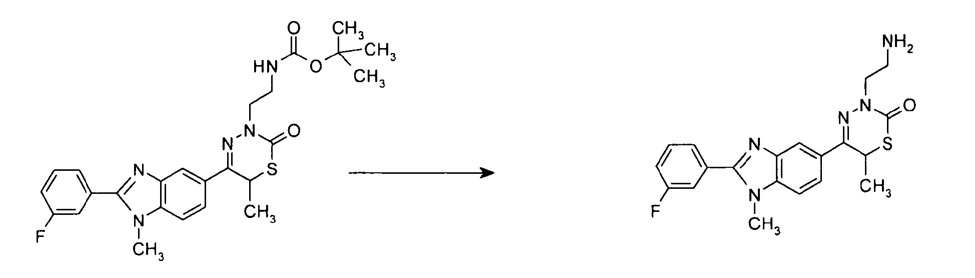

A 30 ml de dioxanne chlorhydrique 4N, on ajoute 6,3 g (13mM) de *tert*-butyl 2-[5-[2-(3-fluorophényl)-1-méthyl-1*H*-benzimidazol-5-yl]-6-méthyl-2-oxo-2*H*-1,3,4-thiadiazin-3(6*H*)-yl]éthylcarbamate. On place sous agitation à température ambiante pendant 20h. On filtre le solide cristallisé qui est le chlorhydrate du composé souhaité. On obtient la base libre par action d'une solution aqueuse d'hydrogénocarbonate de sodium et extraction par l'acétate d'éthyle. La phase aqueuse est lavée à l'eau puis séchée sur sulfate de sodium et concentrée sous vide pour donner 3,85g de 3-(2-aminoéthyl)-5-[2-(3-fluorophényl)-1-méthyl-1*H-*benzimidazol-5-yl]-6-méthyl-3,6-dihydro-2*H*-1,3,4-thiadiazin-2-one sous forme de solide.
Rendement: 76,5%
RMN ¹H (300 MHz / DMSO-d6) δ: 1.59(d, 3H), 3.20(m, 2H), 4.05(s, 3H), 4.21(m, 2H), 5.02(m, 1 H), 7.83(m, 5H), 8.29(m, 3H)
C₂₀H₂₀FN₅OS = 397,47
Spectrométrie de masse M+1 = 398,0

### Exemple 15 : N-(4-chlorophényl)-N'-{3-[5-[2-(2-furyl)-1-méthyl-1H-benzimidazol-5-yl]-2-oxo-2H-1,3,4-thiadiazin-3(6H)-yl]propyl}urée

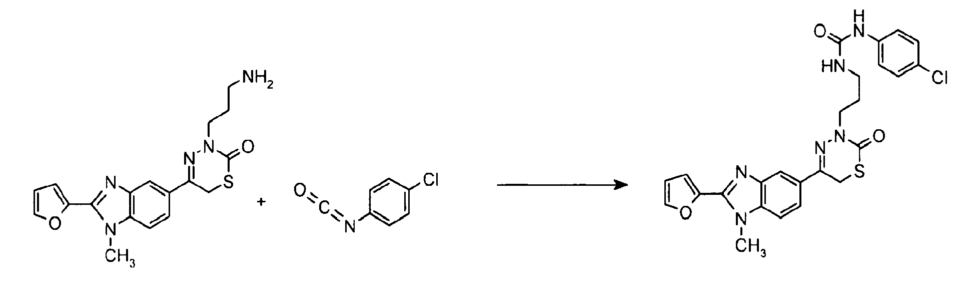

A 2 ml de THF et 164.33 µl (1,33 mM) de triéthylamine sont ajoutés 120 mg (0,30 mM) de 3-(3-aminopropyl)-5-[2-(2-furyl)-1-méthyl-1*H*-benzimidazol-5-yl]-3,6-dihydro-2*H*-1,3,4-thiadiazin-2-one et 50.1 mg (0,33 mM) de 1-chloro-4-isocyanato benzene. On place sous agitation à température ambiante pendant 20h. On filtre le solide qui est lavé à l'eau puis à l'isopropanol pour donner après séchage 106 mg de *N*-(4-chlorophényl)-*N*'-{3-[5-[2-(2-furyl)-1-méthyl-1*H*-benzimidazol-5-yl]-2-oxo-2*H*-1,3,4-thiadiazin-3(6*H*)-yl]propyl}urée.
Rendement : 68%
RMN ¹H (300 MHz / DMSO-d6) δ: 1.90(t, 2H), 3.20(q, 2H), 3.93(t, 2H), 4.07(s, 3H), 4.38(s, 2H), 6.28(t, 1 H), 6.81 (m, 1 H), 7.25(d, 2H), 7.32(d, 1 H), 7.42(d, 2H), 7.71(d, 1H), 7.87(d, 1 H), 8.05(s, 1 H), 8.16(s, 1 H)

Par une méthode similaire ou légèrement modifiée, on prépare les composés suivants :

### Exemple 15-2 :

### N-éthyl-N'-[2-[5-[1-méthyl-2-[3-(trifluorométhyl)phényl]-1H-benzimidazol-5-yl]-2-oxo-2H-1,3,4-thiadiazin-3(6H)-yl]éthyl]-urée

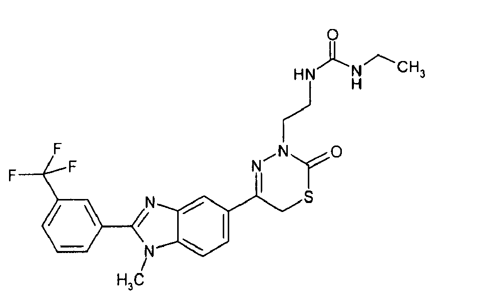

C₂₃H₂₃F₃N₆O₂S = 504,53
Spectrométrie de masse M+1 = 505,1

### Exemple 15-3 :

### N-benzyl-N'-{2-[5-[2-(2-furyl)-1-méthyl-1H-benzimidazol-5-yl]-2-oxo-2H-1,3,4-thiadiazin-3(6H)-yl]éthyl}urée

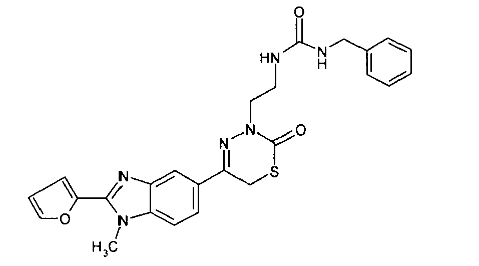

C₂₅H₂₄N₆O₃S = 488,56
Spectrométrie de masse M+1 = 489,0

### Exemple 15-4 :_N-(2-fluorophényl)-N'-{3-[5-[2-(2-furyl)-1-méthyl-1H-benzimidazol-5-yl]-2-oxo-2H-1,3,4-thiadiazin-3(6H)-yl]propyl}urée

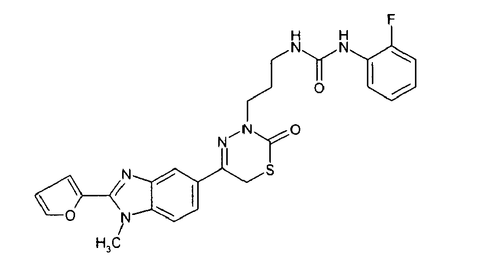

C₂₅H₂₃FN₆O₃S = 506,55
Spectrométrie de masse M+1 = 507,0

### Exemple 15-5 :

### N-(4-acétylphényl)-N'-{2-[5-[2-(3-fluorophényl)-1-méthyl-1H-benzimidazol-5-yl]-6-méthyl-2-oxo-2H-1,3,4-thiadiazin-3(6H)-yl]éthyl}urée

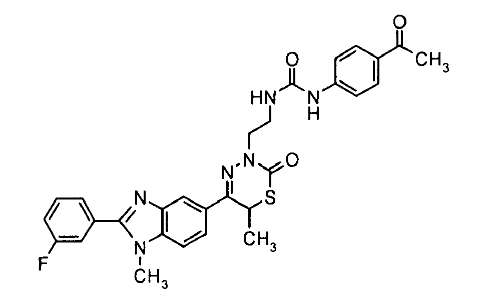

C₂₉H₂₇FN₆O3S = 558,63
Spectrométrie de masse M+1 = 559,1

### Exemple 16 : N-{2-[5-{1-méthyl-2-[3-(trifluorométhyl)phényl]-1H-benzimidazol-5-yl}-2-oxo-2H-1,3,4-thiadiazin-3(6H)-yl]éthyl}benzenesulfonamide

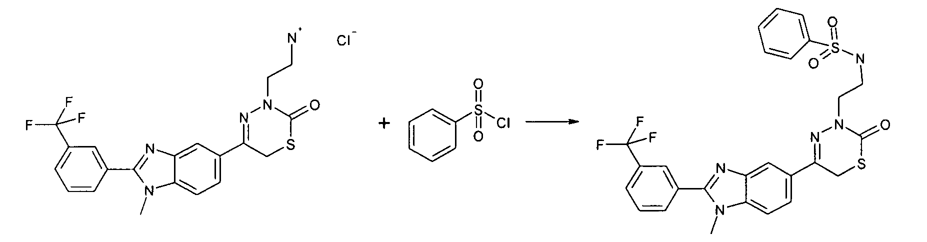

A 2 ml de dichlorométhane 177 µl (1,28 mM) de triéthylamine sont ajoutés 150 mg (0,32 mM) de chlorhydrate de 3-(2-aminoéthyl)-5-{1-méthyl-2-[3-(trifluorométhyl) phényl]-1*H*-benzimidazol-5-yl}-3,6-dihydro-2*H*-1,3,4-thiadiazin-2-one et 67.7 µl (0,38mM) de chlorure de benzene sulfonyle. On place sous agitation à température ambiante pendant 20h. On filtre le solide qui est lavé à l'eau pour donner après séchage 138 mg de *N*-{2-[5-{1-méthyl-2-[3-(trifluorométhyl)phényl]-1*H*-benzimidazol-5-yl}-2-oxo-2*H*-1,3,4-thiadiazin-3(6*H*)-yl]éthyl}benzenesulfonamide.
Rendement : 75%
RMN ¹H (300 MHz / DMSO-d6) δ: 3,14(m, 2H), 3,93(t, 2H), 4,02(s, 3H), 4,37(s, 2H), 7,32(m, 1 H), 7,61(m, 3H), 7,81(m, 1 H), 7,91(m, 3H), 8,03(d, 2H), 8,24(m,3H)
C₂₆H₂₂F₃N₅O₃S₂ = 573,61
Spectrométrie de masse M+1 = 574.1

Par une méthode similaire ou légèrement modifiée, on prépare les composés suivants :

### Exemple 16-2 :

### N-{2-[5-[2-(2-furyl)-1-méthyl-1H-benzimidazol-5-yl]-2-oxo-2H-1,3,4-thiadiazin-3(6H)-yl]éthyl}butane-1-sulfonamide

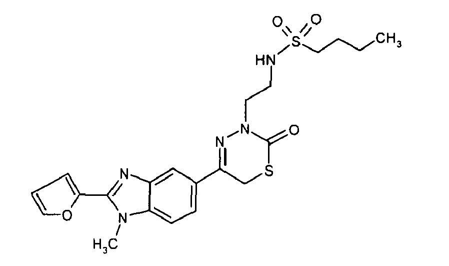

C₂₁H₂₅N₅O₄S₂ = 475,59
Spectrométrie de masse M+1 = 476,0

### Exemple 16-3 :

### N-{2-[5-[2-(2-furyl)-1-méthyl-1H-benzimidazol-5-yl]-2-oxo-2H-1,3,4-thiadiazin-3(6H)-yl]éthyl}méthane sulfonamide

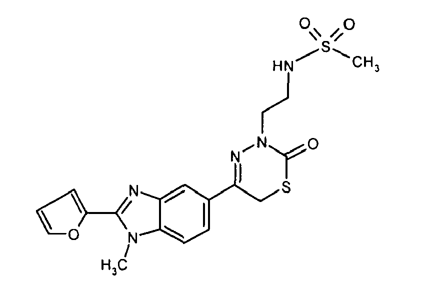

C₁₈H₁₉N₅O₄S₂ = 433,51
Spectrométrie de masse M+1 = 434,1

### Exemple 16-4 :

### N-{2-[5-{1-méthyl-2-[3-(trifluorométhyl)phényl]-1H-benzimidazol-5-yl}-2-oxo-2H-1,3,4-thiadiazin-3(6H)-yl]éthyl}méthanesulfonamide

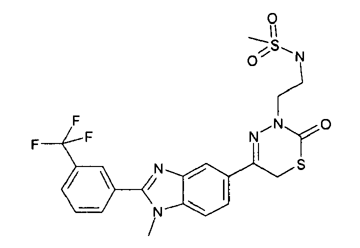

C₂₁H₂₀F₃N₅O₃S₂ = 511,54
Spectrométrie de masse M+1 = 512,0

### Exemple 17 : N-{3-[5-[2-(2-furyl)-1-méthyl-1H-benzimidazol-5-yl]-2-oxo-2H-1,3,4-thiadiazin-3(6H)-yl]éthyl}cyclopropanecarboxamide

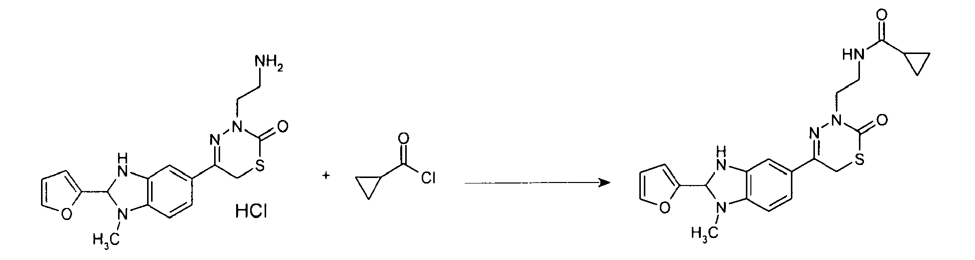

A 4 ml de THF et 212 µl (1,53 mM) de triéthylamine sont ajoutés 150 mg (0,38 mM) de chlorhydrate de 3-(2-aminoéthyl)-5-[2-(2-furyl)-1-méthyl-1*H-*benzimidazol-5-yl]-3,6-dihydro-2*H*-1,3,4-thiadiazin-2-one et 41,7 µl (0,46 mM) de chlorure de cyclopropanecarbonyle. On place sous agitation à température ambiante pendant 20h. On filtre le solide qui est lavé à l'eau pour donner après séchage 106 mg de *N*-{3-[5-[2-(2-furyl)-1-méthyl-1*H*-benzimidazol-5-yl]-2-oxo-2*H-*1,3,4-thiadiazin-3(6*H*)-yl]éthyl}cyclopropane carboxamide.
Rendement: 65%
RMN ¹H (300 MHZ / DMSO-d6) δ: 0,66(m, 4H), 1,51(m, 1H), 3,43(q, 2H), 3,95 (t, 2H), 4,08(s, 3H), 4,34(s, 2H), 6,80(m, 1 H), 7,33(d, 1 H), 7,77(d, 1 H), 7,88(d, 1 H), 8,05(s, 1H), 8,17(s, 1H), 8,23(s, 1H)
C₂₁H₂₁N₅O₃S = 423,49
Spectrométrie de masse M+1 = 424,1

Par une méthode similaire ou légèrement modifiée, on prépare les composés suivants :

### Exemple 17-2 :

### N-{2-[5-{1-méthyl-2-[3-(trifluorométhyl)phényl]-1H-benzimidazol-5-yl}-2-oxo-2H-1,3,4-thiadiazin-3(6H)-yl]éthyl}acétamide

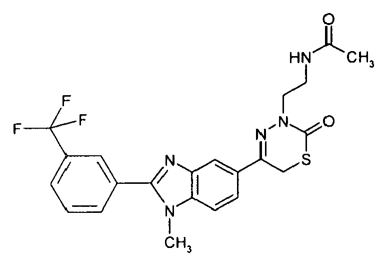

C₂₂H₂₀F₃N₅O₂S = 475,49
Spectrométrie de masse M+1 = 476,0

### Exemple 17-3 :

### N-{2-[5-[2-(3-fluorophényl)-1-méthyl-1H-benzimidazol-5-yl]-6-méthyl-2-oxo-2H-1,3,4-thiadiazin-3(6H)-yl]éthyl}-4-méthylbenzamide

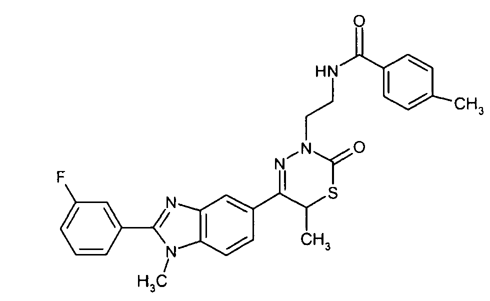

C₂₈H₂₆FN₅O₂S = 515,6
Spectrométrie de masse M+1 = 516,2

### Exemple 17-4 :

### N-{2-[5-[2-(2-furyl)-1-méthyl-1H-benzimidazol-5-yl]-2-oxo-2H-1,3,4-thiadiazin-3(6H)-yl]éthyl}benzamide

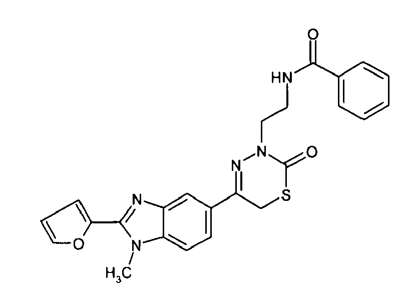

C₂₄H₂₁N₅O₃S = 459,52
Spectrométrie de masse M+1 = 460,1

### Exemple 17-5 :

### N-{3-[5-[2-(2-furyl)-1-méthyl-1H-benzimidazol-5-yl]-2-oxo-2H-1,3,4-thiadiazin-3(6H)-yl]propyl}benzamide

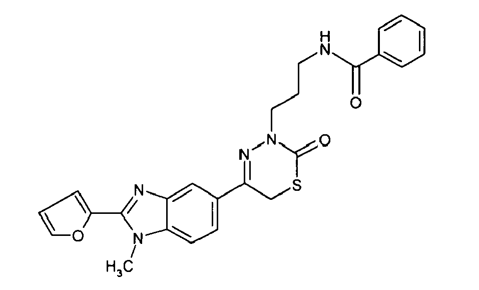

C₂₅H₂₃N₅O₃S = 473,55
Spectrométrie de masse M+1 = 474,0

### Exemple 18 :

### 5-[1-éthyl-2-(3-méthoxyphényl)-1H-benzimidazol-5-yl]-3-[2-(tétrahydro-2H-pyran-2-yloxy)éthyl]-3,6-dihydro-2H-1,3,4-thiadiazin-2-one

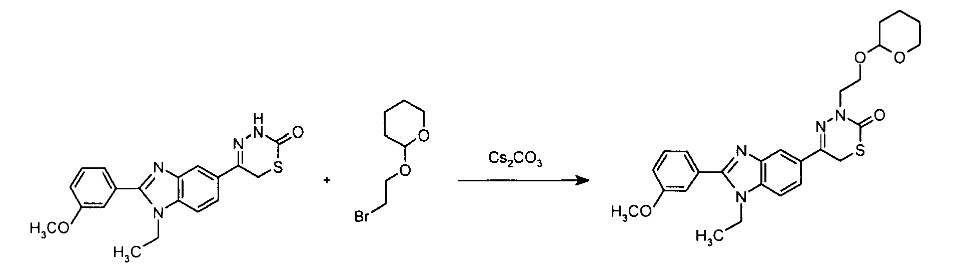

A 200 mg (0,55 mM) de 5-[1-éthyl-2-(3-méthoxyphényl)-1*H*-benzimidazol-5-yl]-3,6-dihydro-2*H*-1,3,4-thiadiazin-2-one dans 4 ml de diméthylformamide, on ajoute 91 µl (0,6 mM) de 2-(2-bromoéthoxy)tetrahydro-2*H*-pyrane et 533,5 mg (1,64 mM) de carbonate de césium. Le milieu réactionnel est ensuite placé sous agitation à température ambiante pendant 16 h. On ajoute de l'eau déminéralisée et la phase organique est isolée, lavée avec de l'eau déminéralisée puis séchée sur du sulfate de sodium anhydre. Le solvant est ensuite évaporé sous vide et le résidu est purifié par chromatographie sur silice en utilisant un mélange dichlorométhane /acétone (90/10) comme éluant pour obtenir 200 mg de 5-[1-éthyl-2-(3-méthoxyphényl)-1*H*-benzimidazol-5-yl]-3-[2-(tetrahydro-2H-pyran-2-yloxy)éthyl]-3,6-dihydro-2*H*-1,3,4-thiadiazin-2-one sous forme d'une huile.
Rendement: 74%
C₂₆H₃₀N₄O₄S = 494,61
Spectrométrie de masse M+1 = 495,2

### Exemple 19 : 5-[1-éthyl-2-(3-méthoxyphényl)-1H-benzimidazol-5-yl]-3-(2-hydroxyéthyl)-3,6-dihydro-2H-1,3,4-thiadiazin-2-one

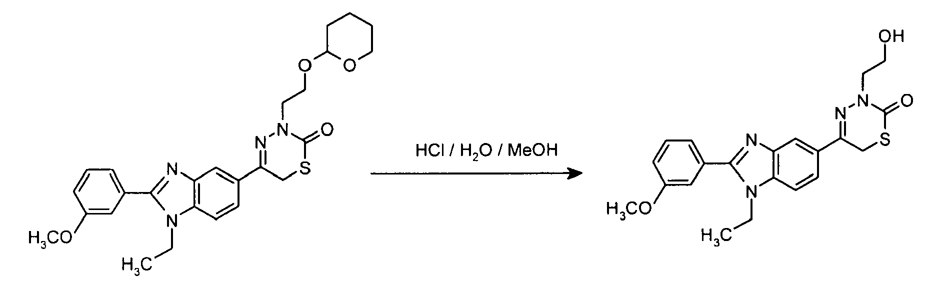

200 mg (0,40 mM) de 5-[1-éthyl-2-(3-méthoxyphényl)-1*H*-benzimidazol-5-yl]-3-[2-(tétrahydro-2*H*-pyran-2-yloxy)éthyl]-3,6-dihydro-2*H*-1,3,4-thiadiazin-2-one et 50 µl (0,6 mM) d'acide chlorhydrique à 37% dans 2 ml de méthanol sont placés sous agitation pendant 16 h à température ambiante. Le solvant est évaporé sous vide et le résidu obtenu est repris dans une solution aqueuse d'hydrogénocarbonate de sodium. On extrait à l'acétate d'éthyle et la phase organique est lavée avec de l'eau déminéralisée puis séchée sur du sulfate de sodium anhydre. Le solvant est évaporé sous vide pour donner une huile qui est purifiée par chromatographie sur silice en utilisant un mélange dichlorométhane /acétone (90/10) comme éluant pour obtenir 119 mg de 5-[1-éthyl-2-(3-méthoxyphényl)-1*H*-benzimidazol-5-yl]-3-(2-hydroxyéthyl)-3,6-dihydro-2*H*-1,3,4-thiadiazin-2-one sous forme d'une huile qui cristallise.
Rendement: 72%
RMN ¹H (300 MHz / DMSO-d6) δ: 1,40(t, 3H), 3,74(t, 2H), 3,91(s, 3H), 4,00(t, 2H), 4,42/4,44(m, 2x2H), 7,24(d, 1H), 7,38(m, 2H), 7,58(m, 1H), 7,85(d, 1 H), 7,93(d, 1H), 8,26(s, 1 H)
C₂₁H₂₂N₄O₃S = 410,49
Spectrométrie de masse M+1 = 411,1

### Exemple 20 : N-(4-propanoylphényl)acétamide

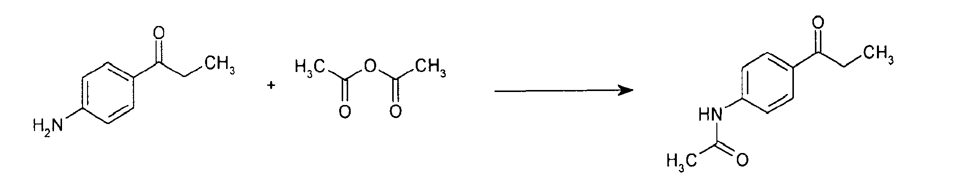

A 400 ml de toluène, on ajoute 30 g de 1-(4-aminophényl)propan-1-one et 38 ml (402 mM) d'anhydride acétique. Le milieu réactionnel est chauffé à 60°C sous agitation pendant 1 H. Un solide précipite. Après filtration et lavage au toluène, on obtient 37,9 g de *N*-(4-propanoylphényl)acétamide sous forme d'une poudre blanche.
Rendement: 99%
RMN ¹H (300 MHz / DMSO-d6) δ: 1.08(t, 3H), 2.10(s, 3H), 3.00(q, 2H), 7.73(d, 2H) 7.92(d, 2H), 10.29(s, 1 H)

### Exemple 21 : N-(2-nitro-4-propanoylphényl)acétamide

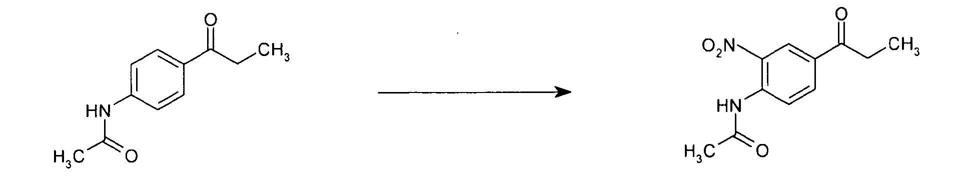

A 140 ml d'acide nitrique, en maintenant la température à -20°C, on ajoute par fractions 37,8 g (198 mM) de N-(4-propanoylphényl)acétamide. Le milieu réactionnel est ensuite coulé sur un mélange eau/glace. Le solide qui précipite est filtré et lavé à l'eau pour donner après séchage, 37,6 g de *N*-(2-nitro-4-propanoylphényl)acétamide sous forme d'un solide jaune pâle.
Rendement: 80,4%
RMN ¹H (300 MHz / DMSO-d6) δ: 1.1(t, 3H), 2.14(s, 3H), 3.11(q, 2H), 7.85(d, 1 H), 8.23(d, 1H), 8.41 (s, 1 H), 10.56(s, 1 H)

### Exemple 22 : N-[4-(2-bromopropanoyl)-2-nitrophényl]acétamide

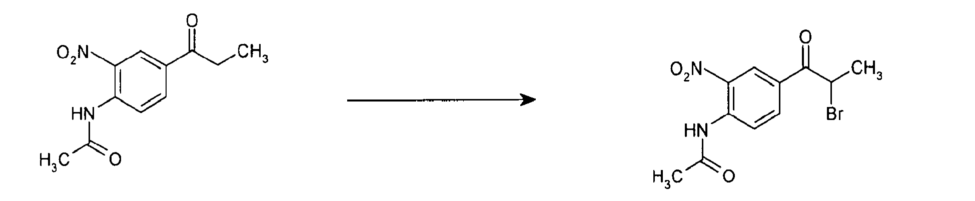

A 1600 ml d'acide acétique, on ajoute 46,7 g (198mM) de *N*-(2-nitro-4-propanoylphényl)acétamide. Sur la solution obtenue chauffée à 35°, on coule en 2 h une solution de 10,13 ml (198mM) de brome dans 200 ml d'acide acétique. Le milieu réactionnel est ensuite ajouté doucement à 5000 ml d'eau, un solide cristallise. Après filtration et lavage du solide à l'eau, on obtient 56,8 g de N-[4-(2-bromopropanoyl)-2-nitrophényl]acétamide sous forme d'un solide jaune pâle. Rendement : 91%
C₁₁H₁₁BrN₂O₄ = 315,12
Spectrométrie de masse M-1 = 313,0

### Exemple 23 : 1-(4-amino-3-nitrophényl)-2-bromopropan-1-one

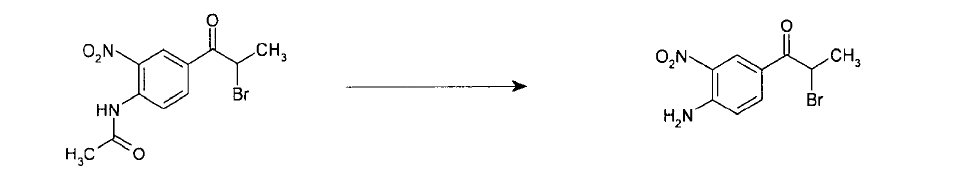

11,65g (37mM) de N-[4-(2-bromopropanoyl)-2-nitrophényl]acétamide en solution dans une solution aqueuse d'acide bromhydrique à 47% sont portés à 120 °C pendant 15 mn sous agitation. Le milieu réactionnel est ensuite coulé sur de l'eau et extrait à l'acétate d'éthyle. La phase organique est lavée à l'eau et avec une solution aqueuse d'hydrogénocarbonate de sodium et enfin une solution aqueuse saturée en chlorure de sodium. La phase organique est décantée, séchée sur sulfate de sodium anhydre puis évaporée sous vide pour donner 35,7 g de 1-(4-amino-3-nitrophényl)-2-bromopropan-1-one sous forme d'un solide jaune.
Rendement: 96,5%
RMN ¹H (300 MHz / DMSO-d6) δ: 1.91 (d, 3H), 5.92(q, 1 H), 7.26(d, 1 H), 8.15(d, 1 H) 8.35(s, 2H), 8.85(s, 1 H)

### Exemple 24 : 5-(4-amino-3-nitrophényl)-6-méthyl-3,6-dihydro-2H-1,3,4-thiadiazin-2-one

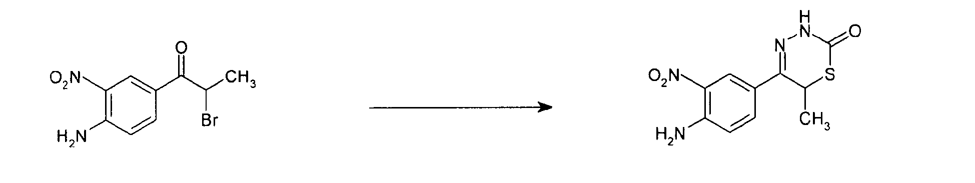

36,5 g (134mM) de 1-(4-amino-3-nitrophényl)-2-bromopropan-1-one et 24,3 g (202,5mM) de O-éthyl hydrazinecarbothioate en solution dans 190 ml d'acétonitrile sont portés au reflux pendant 4h sous agitation. Le mélange réactionnel est refroidi et placé à 0°C pendant 16 h. Le solide cristallisé est filtré et lavé à l'acétonitrile froid pour donner après séchage 10,4g de 5-(4-amino-3-nitrophényl)-6-méthyl-3,6-dihydro-2*H*-1,3,4-thiadiazin-2-one sous forme d'un solide.
Rendement: 29%
RMN ¹H (300 MHz / DMSO-d6) δ: 1.51 (d, 3H), 4.80(q, 1 H), 7.14(d, 1 H), 7.88(m, 3H) 8.41 (s, 1 H)

### Exemple 25 : 5-(3,4-diaminophényl)-6-méthyl-3,6-dihydro-2H-1,3,4-thiadiazin-2-one

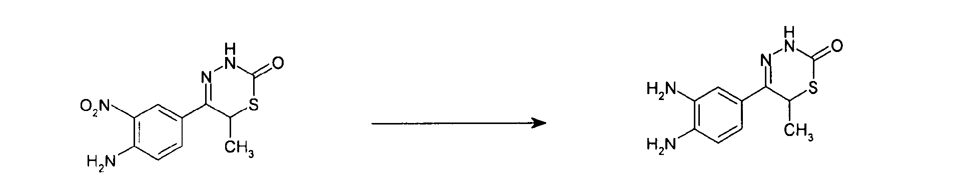

13,7 g (51,4 mM) de 5-(4-amino-3-nitrophényl)-6-méthyl-3,6-dihydro-2*H-*1,3,4-thiadiazin-2-one sont placés sous atmosphère d'hydrogène à 3 bars pendant 4h en présence de 24 g de nickel de Raney dans 280 ml de méthanol. On filtre ensuite le milieu réactionnel et le filtrat est concentré sous vide pour donner 10,5 g de 5-(3,4-diaminophényl)-6-méthyl-3,6-dihydro-2*H*-1,3,4-thiadiazin-2-one sous forme d'un solide traité avec de l'oxyde de diisopropyle, filtré et conservé sous argon à basse température.
Rendement : 86%
C₁₀H₁₂N₄OS = 236,295
Spectrométrie de masse M+1 = 237,2

### Exemple 26 : 5-[2-(3-méthoxyphényl)-1H-benzimidazol-5-yl]-6-méthyl-3,6-dihydro-2H-1,3,4-thiadiazin-2-one

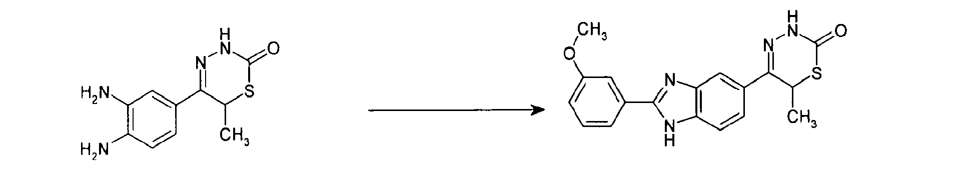

A 145 ml de 1-méthyl-2-pyrrolidone on ajoute 8g (33,8 mM) de 5-(3,4-diaminophényl)-6-méthyl-3,6-dihydro-2*H*-1,3,4-thiadiazin-2-one, 4,11 ml (33,8 mM) de 3-méthoxy benzaldéhyde et 6,43 g (33,8 mM) de disulfite de sodium. Le milieu réactionnel est porté à 110 °C pendant 2h 30 mn. Le milieu réactionnel est coulé sur 1500 ml d'eau, le solide qui précipite est filtré et lavé à l'eau pour donner 9,6 g de 5-[(2-[3-méthoxyphényl)-1*H*-benzimidazol-5-yl]-6-méthyl-3,6-dihydro-2*H*-1,3,4-thiadiazin-2-one sous forme d'un solide.
Rendement : 81 %
RMN ¹H (300 MHz / DMSO-d6) δ: 1,55(d, 3H), 3,94(s, 3H), 4,92(q, 1 H), 7,33(d, 1 H), 7,64(t, 1H), 7,91(d, 1H), 8, 00(t, 2H), 8,05(s, 1H), 8,18(s, 1H), 11,84(s, 1H)

Par une méthode similaire ou légèrement modifiée, on prépare les composés suivants.

### Exemple 26-2 :

### 5-{2-[3-méthoxy-4-(méthylthio)phényl]-1H-benzimidazol-5-yl}-6-méthyl-3,6-dihydro-2H-1,3,4-thiadiazin-2-one, chlorhydrate

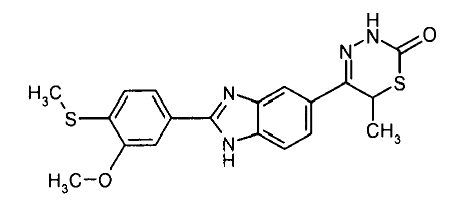

RMN ¹H (300 MHz / DMSO-d6) δ: 1,45(d, 3H), 2,39(s, 3H), 3,89(s, 3H), 4,79(q, 1H), 7,34(d, 1 H), 7,73(d, 1 H), 7,81 (m, 3H), 7,99(s, 1 H), 11,68(m, 1 H)

### Exemple 26-3 :

### 5-{2-[3-méthoxy-4-(méthylsulfinyl)phényl]-1H-benzimidazol-5-yl}-6-méthyl-3,6-dihydro-2H-1,3,4-thiadiazin-2-one, chlorhydrate

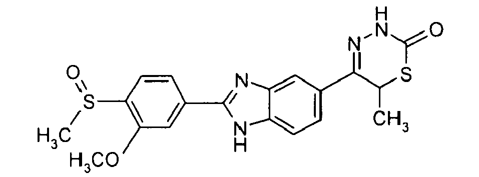

RMN ¹H (300 MHz / DMSO-d6) δ: 1,56(d, 3H), 2,83(s, 3H), 4,07(s, 3H), 4,93(q, 1 H), 7, 90(m,3H), 8,17(m, 2H), 8,23(s, 1 H), 11, 81 (s, 1 H)

### Exemple 26-4 :

### 5-{2-[4-(1H-imidazol-1-yl)phényl]-1H-benzimidazol-5-yl}-6-méthyl-3,6-dihydro-2H-1,3,4-thiadiazin-2-one dichlorhydrate

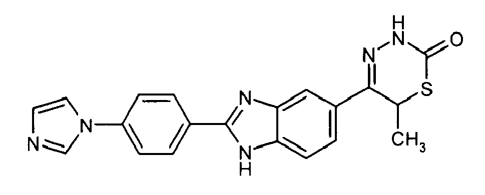

RMN ¹H (300 MHz / DMSO-d6) δ: 1,58(d, 3H), 4,92(q, 1 H), 7,80(d, 1 H), 7,86(d, 1 H), 7,99(s, 1 H), 8,13(m, 3H), 8,44(m, 1 H), 8,55(d, 2H), 9,83(s, 1 H), 11,74(s, 1 H)

Les composés suivants ont été préparés par application ou adaptation des méthodes décrites ci-dessus :
- 5-[2-(4-méthoxyphényl)-1*H*-benzimidazol-5-yl]-3,6-dihydro-2*H*-1,3,4-thiadiazin-2-one,
- 6-méthyl-5-[2-(1*H*-pyrazol-3-yl)-1*H*-benzimidazol-5-yl]-3,6-dihydro-2*H*-1,3,4-thiadiazin-2-one,
- 6-méthyl-5-[2-(2-thienyl)-1*H*-benzimidazol-5-yl]-3,6-dihydro-2*H*-1,3,4-thiadiazin-2-one,
- 5-[2-(3-méthoxyphényl)-1*H*-benzimidazol-5-yl]-6-méthyl-3,6-dihydro-2*H*-1,3,4-thiadiazin-2-one,
- 6-méthyl-5-(2-pyridin-4-yl-1*H*-benzimidazol-5-yl)-3,6-dihydro-2*H*-1,3,4-thiadiazin-2-one,
- 5-{2-[3-méthoxy-4-(méthylthio)phényl]-1*H*-benzimidazol-5-yl}-6-méthyl-3,6-dihydro-2*H*-1,3,4-thiadiazin-2-one,
- 5-{2-[2-méthoxy-4-(méthylthio)phényl]-1*H*-benzimidazol-5-yl}-6-méthyl-3,6-dihydro-2*H*-1,3,4-thiadiazin-2-one,
- 6-méthyl-5-[2-(1,3-thiazol-4-yl)-1*H*-benzimidazol-5-yl]-3,6-dihydro-2*H*-1,3,4-thiadiazin-2-one,
- 5-{2-[3-méthoxy-4-(méthylsulfinyl)phényl]-1*H*-benzimidazol-5-yl}-6-méthyl-3,6-dihydro-2*H*-1,3,4-thiadiazin-2-one,
- 5-[2-(1*H*-imidazol-1-yl)-1*H*-benzimidazol-5-yl]-6-méthyl-3,6-dihydro-2*H*-1,3,4-thiadiazin-2-one,
- 5-{2-[4-(diméthylamino)phényl]-1*H*-benzimidazol-5-yl}-6-méthyl-3,6-dihydro-2*H*-1,3,4-thiadiazin-2-one,
- 6-méthyl-5-{2-[4-(méthylthio)phényl]-1*H*-benzimidazol-5-yl}-3,6-dihydro-2*H-*1,3,4-thiadiazin-2-one,
- 6-méthyl-5-{2-[4-(méthylsulfinyl)phényl]-1*H*-benzimidazol-5-yl}-3,6-dihydro-2*H-*1,3,4-thiadiazin-2-one,
- 5-[2-(2-méthoxyphényl)-1*H*-benzimidazol-5-yl]-6-méthyl-3,6-dihydro-2*H*-1,3,4-thiadiazin-2-one,
- 5-[2-(3-méthoxyphényl)-1*H*-benzimidazol-5-yl]-6-méthyl-3,6-dihydro-2*H*-1,3,4-thiadiazin-2-one,
- 3-méthoxy-4-[5-(6-méthyl-2-oxo-3,6-dihydro-2*H*-1,3,4-thiadiazin-5-yl)-1*H-*benzimidazol-2-yl]phényl thiocyanate,
- 5-{2-[2-chloro-4-(méthylthio)phényl]-1*H*-benzimidazol-5-yl}-6-méthyl-3,6-dihydro-2*H*-1,3,4-thiadiazin-2-one,
- 6-méthyl-5-{2-[4-(pyridin-4-ylamino)phényl]-1*H*-benzimidazol-5-yl}-3,6-dihydro-2*H*-1,3,4-thiadiazin-2-one,
- 5-[2-(4-méthoxyphényl)-1*H*-benzimidazol-5-yl]-3,6-diméthyl-3,6-dihydro-2*H-*1,3,4-thiadiazin-2-one,
- 3,6-diméthyl-5-[2-(1*H*-pyrazol-3-yl)-1*H*-benzimidazol-5-yl]-3,6-dihydro-2*H-*1,3,4-thiadiazin-2-one,
- 3,6-diméthyl-5-[2-(1,3-thiazol-4-yl)-1*H*-benzimidazol-5-yl]-3,6-dihydro-2*H-*1,3,4-thiadiazin-2-one,
- 3,6-diméthyl-5-(2-pyridin-4-yl-1*H*-benzimidazol-5-yl)-3,6-dihydro-2*H*-1,3,4-thiadiazin-2-one,
- 3-éthyl-6-méthyl-5-[2-(1*H*-pyrazol-3-yl)-1*H*-benzimidazol-5-yl]-3,6-dihydro-2*H-*1,3,4-thiadiazin-2-one,
- 3-éthyl-6-méthyl-5-[2-(1,3-thiazol-4-yl)-1*H*-benzimidazol-5-yl]-3,6-dihydro-2*H-*1,3,4-thiadiazin-2-one,
- 3-éthyl-5-[2-(4-méthoxyphényl)-1*H*-benzimidazol-5-yl]-6-méthyl-3,6-dihydro-2*H*-1,3,4-thiadiazin-2-one,
- 5-[1-Cycloheptyl-2-(3-méthoxy-phényl)-1*H*-benzimidazol-5-yl]-3,6-dihydro-2*H-*1,3,4-thiadiazin-2-one,
- 5-[1-Isobutyl-2-(3-méthoxy-phényl)-1*H*-benzimidazol-5-yl]-3,6-dihydro-2*H-*1,3,4-thiadiazin-2-one,
- 5-[1-Isobutyl-2-(3-méthoxy-phényl)-1*H*-benzimidazol-5-yl]-3-(4-méthyl-benzyl)-3,6-dihydro-2*H*-1,3,4-thiadiazin-2-one,
- 3-(4-tert-Butyl-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 5-[1-Cycloheptyl-2-(3-méthoxy-phényl)-1*H*-benzimidazol-5-yl]-3-méthyl]-3,6-dihydro-2*H*-1,3,4-thiadiazin-2-one,
- 5-[1-Cycloheptyl-2-(3-méthoxy-phényl)-1*H*-benzimidazol-5-yl]-3-propyl]-3,6-dihydro-2*H*-1,3,4-thiadiazin-2-one,
- 5-[1-Cycloheptyl-2-(3-méthoxy-phényl)-1*H*-benzimidazol-5-yl]-3-isobutyl]-3,6-dihydro-2*H*-1,3,4-thiadiazin-2-one,
- 5-[1-Cycloheptyl-2-(3-méthoxy-phényl)-1*H*-benzimidazol-5-yl]-3-(2-méthoxy-éthyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-méthyl-benzyl)]-3,6-dihydro-2*H*-1,3,4-thiadiazin-2-one,
- 3-(3-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-Benzyl-5-[1-isobutyl-2-(3-méthoxy-phényl)-1*H*-benzimidazol-5-yl]-3,6-dihydro-2*H*-1,3,4-thiadiazin-2-one,
- 3-(4-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3-Chloro-benzyl)-5-[1-isobutyl-2-(3-méthoxy-phényl)-1*H*-benzimidazol-5-yl]-3,6-dihydro-2*H*-1,3,4-thiadiazin-2-one,
- 5-[1-Isobutyl-2-(3-méthoxy-phényl)-1*H*-benzimidazol-5-yl]-3-(3-méthoxy-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(4-trifluorométhoxy-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-trifluorométhyl-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(4-trifluorométhyl-benzyl)]-3,6-dihydro-2*H*-1,3,4-thiadiazin-2-one,
- 3-(2,3-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,4-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,5-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,6-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3,4-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3,5-Diméthoxy-benzyl)-5-[1-isobutyl-2-(3-méthoxy-phényl)-1*H-*benzimidazol-5-yl]-3,6-dihydro-2*H*-1,3,4-thiadiazin-2-one,
- 3-Cyclopropylméthyl-5-[1-isobutyl-2-(3-méthoxy-phényl)-1*H*-benzimidazol-5-yl]-3,6-dihydro-2*H*-1,3,4-thiadiazin-2-one,
- 3-(2-Hydroxy-éthyl)-5-[1-isobutyl-2-(3-méthoxy-phényl)-1*H*-benzimidazol-5-yl]-3,6-dihydro-2*H*-1,3,4-thiadiazin-2-one,
- 3-Éthyl-5-[1-isobutyl-2-(3-méthoxy-phényl)-1*H*-benzimidazol-5-yl]-3,6-dihydro-2*H*-1,3,4-thiadiazin-2-one,
- 5-[1-Isobutyl-2-(3-méthoxy-phényl)-1*H*-benzimidazol-5-yl]-3-propyl]-3,6-dihydro-2*H*-1,3,4-thiadiazin-2-one,
- 5-[1-Isobutyl-2-(3-méthoxy-phényl)-1*H*-benzimidazol-5-yl]-3-isopropyl]-3,6-dihydro-2*H*-1,3,4-thiadiazin-2-one,
- 2-{5-[1-Isobutyl-2-(3-méthoxy-phényl)-1*H*-benzimidazol-5-yl]-2-oxo-6*H-*[1,3,4]thiadiazin-3-yl}-acetamide,
- 5-[1-Isobutyl-2-(3-méthoxy-phényl)-1*H*-benzimidazol-5-yl]-3-(2-méthoxy-éthyl)]-3,6-dihydro-2*H*-1,3,4-thiadiazin-2-one,
- {5-[1-Isobutyl-2-(3-méthoxy-phényl)-1*H*-benzimidazol-5-yl]-2-oxo-6*H-*[1,3,4]thiadiazin-3-yl}-acetic acid,
- 5-[1-Isobutyl-2-(3-méthoxy-phényl)-1*H*-benzimidazol-5-yl]-6-méthyl]-3,6-dihydro-2*H*-1,3,4-thiadiazin-2-one,
- 3-(2-Hydroxy-éthyl)-5-[1-isobutyl-2-(3-méthoxy-phényl)-1*H*-benzimidazol-5-yl]-6-méthyl]-3,6-dihydro-2*H*-1,3,4-thiadiazin-2-one,
- 5-[1-Isobutyl-2-(3-méthoxy-phényl)-1*H*-benzimidazol-5-yl]-3-propyl]-3,6-dihydro-2*H*-1,3,4-thiadiazin-2-one,
- 5-[1-Isobutyl-2-(3-méthoxy-phényl)-1*H*-benzimidazol-5-yl]-3,6-diméthyl]-3,6-dihydro-2*H*-1,3,4-thiadiazin-2-one,
- 3-Éthyl-5-[1-isobutyl-2-(3-méthoxy-phényl)-1*H*-benzimidazol-5-yl]-6-méthyl]-3,6-dihydro-2*H*-1,3,4-thiadiazin-2-one,
- 5-[1-Isobutyl-2-(3-méthoxy-phényl)-1*H*-benzimidazol-5-yl]-3-isopropyl-6-méthyl]-3,6-dihydro-2*H*-1,3,4-thiadiazin-2-one,
- 3-Cyclopropylméthyl-5-[1-isobutyl-2-(3-méthoxy-phényl)-1*H*-benzimidazol-5-yl]-6-méthyl]-3,6-dihydro-2*H*-1,3,4-thiadiazin-2-one,
- 3-Éthyl-5-[2-(3-méthoxy-phényl)-1-méthyl-1*H*-benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-3-propyl]-3,6-dihydro-2*H*-1,3,4-thiadiazin-2-one; hydrochloride,
- 3-Cyclohexylméthyl-5-[2-(3-méthoxy-phényl)-1-méthyl-1*H*-benzimidazol-5-yl]-3,6-dihydro-2*H*-1,3,4-thiadiazin-2-one,
- 3-[2-(4-Fluoro-phénoxy)-éthyl]-5-[2-(3-méthoxy-phényl)-1-méthyl-1*H-*benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-3-méthyl]-3,6-dihydro-2*H*-1,3,4-thiadiazin-2-one,
- 3-Isopropyl-5-[2-(3-méthoxy-phényl)-1-méthyl-1*H*-benzimidazol-5-yl]-3,6-dihydro-2*H*-1,3,4-thiadiazin-2-one,
- 3-Isobutyl-5-[2-(3-méthoxy-phényl)-1-méthyl-1*H*-benzimidazol-5-yl]-3,6-dihydro-2*H*-1,3,4-thiadiazin-2-one,
- 3-Cyclopropylméthyl-5-[2-(3-méthoxy-phényl)-1-méthyl-1*H*-benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-6-méthyl]-3,6-dihydro-2*H*-1,3,4-thiadiazin-2-one,
- 3-Éthyl-5-[2-(3-méthoxy-phényl)-1-méthyl-1*H*-benzimidazol-5-yl]-6-méthyl]-3,6-dihydro-2*H*-1,3,4-thiadiazin-2-one,
- 5-[2-(3-Méthoxy-phényl)-1-méthyl-1*H*-benzimidazol-5-yl]-6-méthyl-3-propyl]-3,6-dihydro-2*H*-1,3,4-thiadiazin-2-one,
- 5-[2-(3-Méthoxy-phényl)-1-méthyl-1*H*-benzimidazol-5-yl]-6-méthyl-3-(2-piperidin-1-yl-éthyl)]-3,6-dihydro-2*H*-1,3,4-thiadiazin-2-one,
- 3-Isobutyl-5-[2-(3-méthoxy-phényl)-1-méthyl-1*H*-benzimidazol-5-yl]-6-méthyl]-3,6-dihydro-2*H*-1,3,4-thiadiazin-2-one,
- 5-[2-(3-Méthoxy-phényl)-1-méthyl-1*H*-benzimidazol-5-yl]-3,6-diméthyl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3,6-dihydro-2*H*-1,3,4-thiadiazin-2-one,
- 3-Benzyl-6-méthyl-5-[1-méthyl-2-(3-trifluorométhyl-phényl)-1*H*-benzimidazol-5-yl]-3,6-dihydro-2*H*-1,3,4-thiadiazin-2-one,
- 3-Cyclopropylméthyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 3-Isopropyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 3,6-Diméthyl-5-[1-méthyl-2-(3-trifluorométhyl-phényl)-1*H*-benzimidazol-5-yl]-3,6-dihydro-2*H*-1,3,4-thiadiazin-2-one,
- 3-Éthyl-6-méthyl-5-[1-méthyl-2-(3-trifluorométhyl-phényl)-1*H*-benzimidazol-5-yl]-3,6-dihydro-2*H*-1,3,4-thiadiazin-2-one,
- 5-[1-Benzyl-2-(1-méthyl-1*H*-pyrrol-2-yl)-1*H*-benzimidazol-5-yl]-6-méthyl]-3,6-dihydro-2*H*-1,3,4-thiadiazin-2-one,
- 5-[1-Benzyl-2-(1-méthyl-1*H*-imidazol-2-yl)-1*H*-benzimidazol-5-yl]-6-méthyl]-3,6-dihydro-2*H*-1,3,4-thiadiazin-2-one,
- 5-(1-Benzyl-2-thiophé-2-yl-1*H*-benzimidazol-5-yl)-6-méthyl]-3,6-dihydro-2*H-*1,3,4-thiadiazin-2-one,
- 5-[1-Benzyl-2-(3-méthoxy-phényl)-1*H*-benzimidazol-5-yl]-6-méthyl]-3,6-dihydro-2*H*-1,3,4-thiadiazin-2-one,
- 5-[1-Benzyl-2-(4-méthoxy-phényl)-1*H*-benzimidazol-5-yl]-6-méthyl]-3,6-dihydro-2*H*-1,3,4-thiadiazin-2-one,
- 5-[1-Benzyl-2-(3-hydroxy-4-méthoxy-phényl)-1*H*-benzimidazol-5-yl]-6-méthyl]-3,6-dihydro-2*H*-1,3,4-thiadiazin-2-one,
- 5-[1-Benzyl-2-(2,4-dihydroxy-phényl)-1*H*-benzimidazol-5-yl]-6-méthyl]-3,6-dihydro-2*H*-1,3,4-thiadiazin-2-one,
- 5-[1-Benzyl-2-(3,4-dihydroxy-phényl)-1*H*-benzimidazol-5-yl]-6-méthyl]-3,6-dihydro-2*H*-1,3,4-thiadiazin-2-one,
- 5-[1-Benzyl-2-(3,4,5-trihydroxy-phényl)-1*H*-benzimidazol-5-yl]-6-méthyl]-3,6-dihydro-2*H*-1,3,4-thiadiazin-2-one,
- 6-Méthyl-5-[1-méthyl-2-(1-méthyl-1*H*-pyrrol-2-yl)-1*H*-benzimidazol-5-yl]-3,6-dihydro-2*H*-1,3,4-thiadiazin-2-one,
- 5-[2-(1*H*-Imidazol-4-yl)-1-méthyl-1*H*-benzimidazol-5-yl]-6-méthyl]-3,6-dihydro-2*H*-1,3,4-thiadiazin-2-one,
- 5-[2-(1*H*-Imidazol-2-yl)-1-méthyl-1*H*-benzimidazol-5-yl]-6-méthyl]-3,6-dihydro-2*H*-1,3,4-thiadiazin-2-one,
- 6-Méthyl-5-[1-méthyl-2-(5-méthyl-3*H*-imidazol-4-yl)-1*H*-benzimidazol-5-yl]-3,6-dihydro-2*H*-1,3,4-thiadiazin-2-one,
- 6-Méthyl-5-[1-méthyl-2-(1-méthyl-1*H*-imidazol-2-yl)-1*H*-benzimidazol-5-yl]-3,6-dihydro-2*H*-1,3,4-thiadiazin-2-one,
- 6-Méthyl-5-(1-méthyl-2-thiophé-2-yl-1*H*-benzimidazol-5-yl)]-3,6-dihydro-2*H-*1,3,4-thiadiazin-2-one,
- 6-Méthyl-5-(1-méthyl-2-thiophé-3-yl-1*H*-benzimidazol-5-yl)]-3,6-dihydro-2*H-*1,3,4-thiadiazin-2-one,
- 5-[2-(2-Méthoxy-phényl)-1-méthyl-1*H*-benzimidazol-5-yl]-6-méthyl]-3,6-dihydro-2*H*-1,3,4-thiadiazin-2-one,
- 5-[2-(4-Méthoxy-phényl)-1-méthyl-1*H*-benzimidazol-5-yl]-6-méthyl]-3,6-dihydro-2*H*-1,3,4-thiadiazin-2-one,
- 5-[2-(3-Hydroxy-4-méthoxy-phényl)-1-méthyl-1*H*-benzimidazol-5-yl]-6-méthyl]-3,6-dihydro-2*H*-1,3,4-thiadiazin-2-one,
- 5-[2-(3,4-Dihydroxy-phényl)-1-méthyl-1*H*-benzimidazol-5-yl]-6-méthyl]-3,6-dihydro-2*H*-1,3,4-thiadiazin-2-one,
- 6-Méthyl-5-[1-méthyl-2-(3,4,5-trihydroxy-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(3-méthyl-benzyl)]-3,6-dihydro-2*H*-1,3,4-thiadiazin-2-one,
- 5-[1-Éthyl-2-(3-fluoro-phényl)-1*H*-benzimidazol-5-yl]-3-(4-méthyl-benzyl)]-3,6-dihydro-2*H*-1,3,4-thiadiazin-2-one,
- 3-(4-tert-Butyl-benzyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(2-fluoro-benzyl)]-3,6-dihydro-2*H*-1,3,4-thiadiazin-2-one,
- 5-[1-Éthyl-2-(3-fluoro-phényl)-1*H*-benzimidazol-5-yl]-3-(3-fluoro-benzyl)]-3,6-dihydro-2*H*-1,3,4-thiadiazin-2-one,
- 3-Benzyl-5-[1-éthyl-2-(3-fluoro-phényl)-1*H*-benzimidazol-5-yl]-3,6-dihydro-2*H-*1,3,4-thiadiazin-2-one,
- 3-(3-Chloro-benzyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-méthyl]-3,6-dihydro-2*H-*1,3,4-thiadiazin-2-one,
- 3-Cyclohexylméthyl-5-[1-éthyl-2-(3-fluoro-phényl)-1*H*-benzimidazol-5-yl]-3,6-dihydro-2*H*-1,3,4-thiadiazin-2-one,
- 3-(2-Diméthylamino-éthyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(2-pyrrolidin-1-yl-éthyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-méthyl-benzyl)]-3,6-dihydro-2*H*-1,3,4-thiadiazin-2-one,
- 5-[2-(4-Méthoxy-phényl)-1*H*-benzimidazol-5-yl]-6-méthyl]-3,6-dihydro-2*H-*1,3,4-thiadiazin-2-one,
- 3-(2-Diméthylamino-éthyl)-6-méthyl-5-[1-méthyl-2-(3-trifluorométhyl-phényl)-1*H*-benzimidazol-5-yl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-piperidin-1-yl-éthyl)]-3,6-dihydro-2*H*-1,3,4-thiadiazin-2-one,
- 3-Cyclohexylméthyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-morpholin-4-yl-éthyl)]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-pyrrolidin-1-yl-éthyl)]-3,6-dihydro-2*H*-1,3,4-thiadiazin-2-one,
- 2-{6-Méthyl-5-[1-méthyl-2-(3-trifluorométhyl-phényl)-1*H*-benzimidazol-5-yl]-2-oxo-6*H*-[1,3,4]thiadiazin-3-yl}-acetamide,
- 3-(2-Méthoxy-éthyl)-6-méthyl-5-[1-méthyl-2-(3-trifluorométhyl-phényl)-1*H-*benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-(2-hydroxy-éthyl)-6-méthyl]-3,6-dihydro-2*H*-1,3,4-thiadiazin-2-one,
- 3-Cyclohexylméthyl-5-[2-(3-fluoro-phényl)-1-méthyl-1*H*-benzimidazol-5-yl]-6-méthyl]-3,6-dihydro-2*H*-1,3,4-thiadiazin-2-one,
- 2-{5-[2-(3-Fluoro-phényl)-1-méthyl-1*H*-benzimidazol-5-yl]-6-méthyl-2-oxo-6H-[1,3,4]thiadiazin-3-yl}-acetamide,
- 3-[2-(4-Fluoro-phénoxy)-éthyl]-5-[2-(3-fluoro-phényl)-1-méthyl-1*H-*benzimidazol-5-yl]-6-méthyl]-3,6-dihydro-2*H*-1,3,4-thiadiazin-2-one,
- 5-[2-(3-Fluoro-phényl)-1-méthyl-1*H*-benzimidazol-5-yl]-3-isopropyl-6-méthyl]-3,6-dihydro-2*H*-1,3,4-thiadiazin-2-one,
- 5-[2-(3-Fluoro-phényl)-1-méthyl-1*H*-benzimidazol-5-yl]-3-isobutyl-6-méthyl]-3,6-dihydro-2*H*-1,3,4-thiadiazin-2-one,
- 4-{5-[2-(3-Fluoro-phényl)-1-méthyl-1*H*-benzimidazol-5-yl]-6-méthyl-2-oxo-6*H-*[1,3,4]thiadiazin-3-yl}-butyric acid éthyl ester,
- 3-{5-[2-(3-Fluoro-phényl)-1-méthyl-1*H*-benzimidazol-5-yl]-6-méthyl-2-oxo-6*H-*[1,3,4]thiadiazin-3-yl}-propionic acid éthyl ester,
- 3-Cyclopropylméthyl-5-[2-(3-fluoro-phényl)-1-méthyl-1*H*-benzimidazol-5-yl]-6-méthyl]-3,6-dihydro-2*H*-1,3,4-thiadiazin-2-one,
- 5-[2-(3-Fluoro-phényl)-1-méthyl-1*H*-benzimidazol-5-yl]-3-(2-méthoxy-éthyl)-6-méthyl]-3,6-dihydro-2*H*-1,3,4-thiadiazin-2-one,
- 3-Benzyl-5-[2-(3-fluoro-phényl)-1-méthyl-1*H*-benzimidazol-5-yl]-6-méthyl]-3,6-dihydro-2*H*-1,3,4-thiadiazin-2-one,
- {5-[2-(3-Fluoro-phényl)-1-méthyl-1*H*-benzimidazol-5-yl]-6-méthyl-2-oxo-6*H-*[1,3,4]thiadiazin-3-yl}-acetic acid éthyl ester,
- 5-[2-(3-Fluoro-phényl)-1-méthyl-1*H*-benzimidazol-5-yl]-6-méthyl-3-propyl]-3,6-dihydro-2*H*-1,3,4-thiadiazin-2-one,
- 5-[2-(3-Fluoro-phényl)-1-méthyl-1*H*-benzimidazol-5-yl]-6-méthyl-3-(2-morpholin-4-yl-éthyl)]-3,6-dihydro-2*H*-1,3,4-thiadiazin-2-one
- 5-[2-(3-Fluoro-phényl)-1-méthyl-1*H*-benzimidazol-5-yl]-6-méthyl-3-(2-piperidin-1-yl-éthyl)]-3,6-dihydro-2*H*-1,3,4-thiadiazin-2-one,
- 5-[2-(3-Fluoro-phényl)-1-méthyl-1*H*-benzimidazol-5-yl]-6-méthyl]-3,6-dihydro-2*H*-1,3,4-thiadiazin-2-one,
- 3-Cyclohexylméthyl-5-[2-(3-fluoro-phényl)-1-méthyl-1*H*-benzimidazol-5-yl]-3,6-dihydro-2*H*-1,3,4-thiadiazin-2-one,
- 2-{5-[2-(3-Fluoro-phényl)-1-méthyl-1*H*-benzimidazol-5-yl]-2-oxo-6*H-*[1,3,4]thiadiazin-3-yl}-acetamide,
- 3-[2-(4-Fluoro-phénoxy)-éthyl]-5-[2-(3-fluoro-phényl)-1-méthyl-1*H-*benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-isopropyl]-3,6-dihydro-2*H*-1,3,4-thiadiazin-2-one,
- 5-[2-(3-Fluoro-phényl)-1-méthyl-1*H*-benzimidazol-5-yl]-3-isobutyl]-3,6-dihydro-2*H*-1,3,4-thiadiazin-2-one,
- 3-Cyclopropylméthyl-5-[2-(3-fluoro-phényl)-1-méthyl-1*H*-benzimidazol-5-yl]-3,6-dihydro-2*H*-1,3,4-thiadiazin-2-one
- 5-[2-(3-Fluoro-phényl)-1-méthyl-1*H*-benzimidazol-5-yl]-3-(2-méthoxy-éthyl)]-3,6-dihydro-2*H*-1,3,4-thiadiazin-2-one,
- 3-Benzyl-5-[2-(3-fluoro-phényl)-1-méthyl-1*H*-benzimidazol-5-yl]-3,6-dihydro-2*H*-1,3,4-thiadiazin-2-one,
- {5-[2-(3-Fluoro-phényl)-1-méthyl-1*H*-benzimidazol-5-yl]-2-oxo-6*H-*[1,3,4]thiadiazin-3-yl}-acetic acid éthyl ester,
- 5-[2-(3-Fluoro-phényl)-1-méthyl-1*H*-benzimidazol-5-yl]-3-(2-piperidin-1-yl-éthyl)]-3,6-dihydro-2*H*-1,3,4-thiadiazin-2-one,
- 5-[2-(3-Fluoro-phényl)-1-méthyl-1*H*-benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-propyl]-3,6-dihydro-2*H*-1,3,4-thiadiazin-2-one,
- 5-(1-Benzyl-2-thiophé-3-yl-1*H*-benzimidazol-5-yl)-6-méthyl]-3,6-dihydro-2*H-*1,3,4-thiadiazin-2-one,
- 5-[1-Benzyl-2-(2-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(3-méthoxy-phényl)-1*H*-benzimidazol-5-yl]-6-méthyl]-3,6-dihydro-2*H*-1,3,4-thiadiazin-2-one,
- 3-Cyclohexylméthyl-5-(2-furan-2-yl-1-méthyl-1*H*-benzimidazol-5-yl)]-3,6-dihydro-2*H*-1,3,4-thiadiazin-2-one,
- 3-Benzyl-5-(2-furan-2-yl-1-méthyl-1*H*-benzimidazol-5-yl)]-3,6-dihydro-2*H-*1,3,4-thiadiazin-2-one,
- 3-Cyclopropylméthyl-5-(2-furan-2-yl-1-méthyl-1*H*-benzimidazol-5-yl)]-3,6-dihydro-2*H*-1,3,4-thiadiazin-2-one,
- 5-(2-Furan-2-yl-1-méthyl-1*H*-benzimidazol-5-yl)-3-méthyl]-3,6-dihydro-2*H-*1,3,4-thiadiazin-2-one,
- 3-Éthyl-5-(2-furan-2-yl-1-méthyl-1*H*-benzimidazol-5-yl)]-3,6-dihydro-2*H*-1,3,4-thiadiazin-2-one,
- 5-(2-Furan-2-yl-1-méthyl-1*H*-benzimidazol-5-yl)-3-(2-piperidin-1-yl-éthyl)]-3,6-dihydro-2*H*-1,3,4-thiadiazin-2-one,
- 5-(2-Furan-2-yl-1-méthyl-1*H*-benzimidazol-5-yl)-3-(2-pyrrolidin-1-yl-éthyl)]-3,6-dihydro-2*H*-1,3,4-thiadiazin-2-one,
- 5-(2-Furan-2-yl-1-méthyl-1*H*-benzimidazol-5-yl)-3-isobutyl]-3,6-dihydro-2*H-*1,3,4-thiadiazin-2-one,
- 5-(2-Furan-2-yl-1-méthyl-1*H*-benzimidazol-5-yl)]-3,6-dihydro-2*H*-1,3,4-thiadiazin-2-one,
- 2-{5-[2-(3-Méthoxy-phényl)-1-méthyl-1*H*-benzimidazol-5-yl]-6-méthyl-2-oxo-6*H*-[1,3,4]thiadiazin-3-yl}-acetamide,
- 3-Isopropyl-5-[2-(3-méthoxy-phényl)-1-méthyl-1*H*-benzimidazol-5-yl]-6-méthyl]-3,6-dihydro-2*H*-1,3,4-thiadiazin-2-one,
- 3-(4-tert-Butyl-benzyl)-5-[1-méthyl-2-(3-trifluorométhyl-phényl)-1*H-*benzimidazol-5-yl]-3,6-dihydro-2*H*-1,3,4-thiadiazin-2-one,
- 3-(4-Fluoro-benzyl)-5-[1-méthyl-2-(3-trifluorométhyl-phényl)-1*H*-benzimidazol-5-yl]-3,6-dihydro-2*H*-1,3,4-thiadiazin-2-one,
- 5-[1-Méthyl-2-(3-trifluorométhyl-phényl)-1*H*-benzimidazol-5-yl]-3-(4-trifluorométhoxy-benzyl)]-3,6-dihydro-2*H*-1,3,4-thiadiazin-2-one,
- 5-[1-Méthyl-2-(3-trifluorométhyl-phényl)-1*H*-benzimidazol-5-yl]-3-(4-trifluorométhyl-benzyl)]-3,6-dihydro-2*H*-1,3,4-thiadiazin-2-one,
- 3-{5-[1-Méthyl-2-(3-trifluorométhyl-phényl)-1*H*-benzimidazol-5-yl]-2-oxo-6*H-*[1,3,4]thiadiazin-3-ylméthyl}-benzonitrile,
- 3-(4-Méthanesulfonyl-benzyl)-5-[1-méthyl-2-(3-trifluorométhyl-phényl)-1*H-*benzimidazol-5-yl]-3,6-dihydro-2*H*-1,3,4-thiadiazin-2-one,
- 3-(3,5-Diméthoxy-benzyl)-5-[1-méthyl-2-(3-trifluorométhyl-phényl)-1*H-*benzimidazol-5-yl]-3,6-dihydro-2*H*-1,3,4-thiadiazin-2-one,
- 5-[1-Méthyl-2-(3-trifluorométhyl-phényl)-1*H*-benzimidazol-5-yl]-3,6-dihydro-2*H-*1,3,4-thiadiazin-2-one,
- 5-[1-Éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(4-fluoro-benzyl)]-3,6-dihydro-2*H*-1,3,4-thiadiazin-2-one,
- 5-[1-Éthyl-2-(3-fluoro-phényl)-1*H*-benzimidazol-5-yl]-3-(3-méthoxy-benzyl)]-3,6-dihydro-2*H*-1,3,4-thiadiazin-2-one,
- 5-[1-Éthyl-2-(3-fluoro-phényl)-1*H*-benzimidazol-5-yl]-3-(4-trifluorométhoxy-benzyl)]-3,6-dihydro-2*H*-1,3,4-thiadiazin-2-one,
- 2-{5-[1-Éthyl-2-(3-fluoro-phényl)-1*H*-benzimidazol-5-yl]-2-oxo-6*H-*[1,3,4]thiadiazin-3-yl}-acetamide,
- 3-Cyclopropylméthyl-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-isopropyl]-3,6-dihydro-2*H*-1,3,4-thiadiazin-2-one
- 3-Éthyl-5-[1-éthyl-2-(3-fluoro-phényl)-1*H*-benzimidazol-5-yl]-3,6-dihydro-2*H-*1,3,4-thiadiazin-2-one,
- 5-[1-Éthyl-2-(3-fluoro-phényl)-1*H*-benzimidazol-5-yl]-3-propyl]-3,6-dihydro-2*H-*1,3,4-thiadiazin-2-one,
- 5-[1-Éthyl-2-(3-fluoro-phényl)-1*H*-benzimidazol-5-yl]-3-(2-méthoxy-éthyl)]-3,6-dihydro-2*H*-1,3,4-thiadiazin-2-one, - {5-[1-Éthyl-2-(3-fluoro-phényl)-1*H*-benzimidazol-5-yl]-2-oxo-6*H-*[1,3,4]thiadiazin-3-yl}-acetic acid,
- 5-[1-Éthyl-2-(3-fluoro-phényl)-1*H*-benzimidazol-5-yl]-3-(2-hydroxy-éthyl)]-3,6-dihydro-2*H*-1,3,4-thiadiazin-2-one,
- 5-[1-Éthyl-2-(3-fluoro-phényl)-1*H*-benzimidazol-5-yl]-3-(3-hydroxy-propyl)]-3,6-dihydro-2*H*-1,3,4-thiadiazin-2-one,
- 3-(2-Amino-éthyl)-5-[1-éthyl-2-(3-fluoro-phényl)-1*H*-benzimidazol-5-yl]-3,6-dihydro-2*H*-1,3,4-thiadiazin-2-one,
- 3-(3-Amino-propyl)-5-[1-éthyl-2-(3-fluoro-phényl)-1*H*-benzimidazol-5-yl]-3,6-dihydro-2*H*-1,3,4-thiadiazin-2-one,
- 3-Benzyl-5-[1-éthyl-2-(3-méthoxy-phényl)-1*H*-benzimidazol-5-yl]-3,6-dihydro-2*H*-1,3,4-thiadiazin-2-one,
- 5-[1-Éthyl-2-(3-méthoxy-phényl)-1*H*-benzimidazol-5-yl]-3-(4-méthyl-benzyl)]-3,6-dihydro-2*H*-1,3,4-thiadiazin-2-one,
- 5-[1-Éthyl-2-(3-méthoxy-phényl)-1*H*-benzimidazol-5-yl]-3-(2-fluoro-benzyl)]-3,6-dihydro-2*H*-1,3,4-thiadiazin-2-one,
- 3-Benzyl-5-[1-méthyl-2-(3-trifluorométhyl-phényl)-1*H*-benzimidazol-5-yl]-3,6-dihydro-2*H*-1,3,4-thiadiazin-2-one,
- 3-Méthyl-5-[1-méthyl-2-(3-trifluorométhyl-phényl)-1*H*-benzimidazol-5-yl]-3,6-dihydro-2*H*-1,3,4-thiadiazin-2-one,
- 3-Cyclohexylméthyl-5-[1-méthyl-2-(3-trifluorométhyl-phényl)-1*H*-benzimidazol-5-yl]-3,6-dihydro-2*H*-1,3,4-thiadiazin-2-one,
- 3-(3-Amino-propyl)-5-[1-méthyl-2-(3-trifluorométhyl-phényl)-1*H*-benzimidazol-5-yl]-3,6-dihydro-2*H*-1,3,4-thiadiazin-2-one,
- 3-(2-Amino-éthyl)-5-[1-méthyl-2-(3-trifluorométhyl-phényl)-1*H*-benzimidazol-5-yl]-3,6-dihydro-2*H*-1,3,4-thiadiazin-2-one,
- 3-(2-Amino-éthyl)-5-[1-éthyl-2-(3-méthoxy-phényl)-1*H*-benzimidazol-5-yl]-3,6-dihydro-2*H*-1,3,4-thiadiazin-2-one,
- 3-(3-Amino-propyl)-5-[1-éthyl-2-(3-méthoxy-phényl)-1*H*-benzimidazol-5-yl]-3,6-dihydro-2*H*-1,3,4-thiadiazin-2-one,
- 5-[1-Éthyl-2-(3-méthoxy-phényl)-1*H*-benzimidazol-5-yl]-3-(2-hydroxy-éthyl)]-3,6-dihydro-2*H*-1,3,4-thiadiazin-2-one
- 5-[1-Éthyl-2-(3-méthoxy-phényl)-1*H*-benzimidazol-5-yl]-3-(3-hydroxy-propyl)]-3,6-dihydro-2*H*-1,3,4-thiadiazin-2-one,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-acetamide,
- Furan-2-carboxylic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-isobutyramide,
- Cyclopentanecarboxylic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-benzamide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-méthanesulfonamide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-benzenesulfonamide,
- 5-[1-Méthyl-2-(3-trifluorométhyl-phényl)-1*H*-benzimidazol-5-yl]-6-propyl]-3,6-dihydro-2*H*-1,3,4-thiadiazin-2-one,
- 3-Éthyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-3,6-dipropyl]-3,6-dihydro-2*H*-1,3,4-thiadiazin-2-one,
- 3-Benzyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Cyclopropylméthyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Isopropyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Isobutyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-3-(2-piperidin-1-yl-éthyl)-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-6-propyl-3-(2-pyrrolidin-1-yl-éthyl)]-3,6-dihydro-2*H*-1,3,4-thiadiazin-2-one,
- 1-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-3-phényl-urée,
- 1-Éthyl-3-(2-{5-[1-méthyl-2-(3-trifluorométhyl-phényl)-1*H*-benzimidazol-5-yl]-2-oxo-6*H*-[1,3,4]thiadiazin-3-yl}-éthyl)-urée,
- 5-[1-Éthyl-2-(3-méthoxy-phényl)-1*H*-benzimidazol-5-yl]-3,6-dihydro-2*H*-1,3,4-thiadiazin-2-one,
- 3-Cyclopropylméthyl-5-[1-méthyl-2-(3-trifluorométhyl-phényl)-1*H*-benzimidazol-5-yl]-3,6-dihydro-2*H*-1,3,4-thiadiazin-2-one,
- Butane-1-sulfonic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-{2-[5-(2-Furan-2-yl-1-méthyl-1*H*-benzimidazol-5-yl)-2-oxo-6*H-*[1,3,4]thiadiazin-3-yl]-éthyl}-acetamide,
- N-{2-[5-(2-Furan-2-yl-1-méthyl-1*H*-benzimidazol-5-yl)-2-oxo-6*H-*[1,3,4]thiadiazin-3-yl]-éthyl}-isobutyramide,
- Cyclopentanecarboxylic acid {2-[5-(2-furan-2-yl-1-méthyl-1*H*-benzimidazol-5-yl)-2-oxo-6*H*-[1,3,4]thiadiazin-3-yl]-éthyl}-amide,
- N-{2-[5-(2-Furan-2-yl-1-méthyl-1*H*-benzimidazol-5-yl)-2-oxo-6*H-*[1,3,4]thiadiazin-3-yl]-éthyl}-benzamide,
- N-{2-[5-(2-Furan-2-yl-1-méthyl-1*H*-benzimidazol-5-yl)-2-oxo-6*H-*[1,3,4]thiadiazin-3-yl]-éthyl}-nicotinamide,
- Cyclopropanecarboxylic acid {2-[5-(2-furan-2-yl-1-méthyl-1*H*-benzimidazol-5-yl)-2-oxo-6*H*-[1,3,4]thiadiazin-3-yl]-éthyl}-amide,
- 3-Fluoro-N-{2-[5-(2-furan-2-yl-1-méthyl-1*H*-benzimidazol-5-yl)-2-oxo-6*H-*[1,3,4]thiadiazin-3-yl]-éthyl}-benzamide,
- N-{2-[5-(2-Furan-2-yl-1-méthyl-1*H*-benzimidazol-5-yl)-2-oxo-6*H-*[1,3,4]thiadiazin-3-yl]-éthyl}-4-méthyl-benzamide,
- 3-(2-Amino-éthyl)-5-(2-furan-2-yl-1-méthyl-1*H*-benzimidazol-5-yl)]-3,6-dihydro-2*H*-1,3,4-thiadiazin-2-one,
- N-{2-[5-(2-Furan-2-yl-1-méthyl-1*H*-benzimidazol-5-yl)-2-oxo-6*H-*[1,3,4]thiadiazin-3-yl]-éthyl}-benzenesulfonamide,
- Butane-1-sulfonic acid {2-[5-(2-furan-2-yl-1-méthyl-1*H*-benzimidazol-5-yl)-2-oxo-6*H*-[1,3,4]thiadiazin-3-yl]-éthyl}-amide,
- 1-{2-[5-(2-Furan-2-yl-1-méthyl-1*H*-benzimidazol-5-yl)-2-oxo-6*H-*[1,3,4]thiadiazin-3-yl]-éthyl}-3-isopropyl-urée,
- 1-{2-[5-(2-Furan-2-yl-1-méthyl-1*H*-benzimidazol-5-yl)-2-oxo-6*H-*[1,3,4]thiadiazin-3-yl]-éthyl}-3-phényl-urée,
- 1-Cyclopentyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-Éthyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-Benzyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-(4-Fluoro-benzyl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-(4-Acetyl-phényl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-{2-[5-(2-Furan-2-yl-1-méthyl-1H-benzimidazol-5-yl)-2-oxo-6*H-*[1,3,4]thiadiazin-3-yl]-éthyl}-3-p-tolyl-urée,
- 1-Butyl-3-{2-[5-(2-furan-2-yl-1-méthyl-1*H*-benzimidazol-5-yl)-2-oxo-6*H-*[1,3,4]thiadiazin-3-yl]-éthyl}-urée,
- N-{3-[5-(2-Furan-2-yl-1-méthyl-1*H*-benzimidazol-5-yl)-2-oxo-6*H-*[1,3,4]thiadiazin-3-yl]-propyl}-benzamide,
- Cyclopropanecarboxylic acid {3-[5-(2-furan-2-yl-1-méthyl-1*H*-benzimidazol-5-yl)-2-oxo-6*H*-[1,3,4]thiadiazin-3-yl]-propyl}-amide,
- 1-(4-Chloro-phényl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-(4-Chloro-phényl)-3-{3-[5-(2-furan-2-yl-1-méthyl-1*H*-benzimidazol-5-yl)-2-oxo-6*H*-[1,3,4]thiadiazin-3-yl]-propyl}-urée,
- 1-Butyl-3-{3-[5-(2-furan-2-yl-1-méthyl-1*H*-benzimidazol-5-yl)-2-oxo-6*H-*[1,3,4]thiadiazin-3-yl]-propyl}-urée,
- 1-Benzyl-3-{3-[5-(2-furan-2-yl-1-méthyl-1*H*-benzimidazol-5-yl)-2-oxo-6*H-*[1,3,4]thiadiazin-3-yl]-propyl}-urée,
- 1-(3-Fluoro-phényl)-3-{3-[5-(2-furan-2-yl-1-méthyl-1*H*-benzimidazol-5-yl)-2-oxo-6*H*-[1,3,4]thiadiazin-3-yl]-propyl}-urée
- N-{2-[5-(2-Furan-2-yl-1-méthyl-1*H*-benzimidazol-5-yl)-2-oxo-6*H-*[1,3,4]thiadiazin-3-yl]-éthyl}-méthanesulfonamide,
- 5-(2-Furan-2-yl-1-méthyl-1*H*-benzimidazol-5-yl)-3-(2-méthoxy-éthyl)]-3,6-dihydro-2*H*-1,3,4-thiadiazin-2-one,
- 5-(2-Furan-2-yl-1-méthyl-1*H*-benzimidazol-5-yl)-3-(3-hydroxy-propyl)]-3,6-dihydro-2*H*-1,3,4-thiadiazin-2-one,
- 5-(2-Furan-2-yl-1-méthyl-1*H*-benzimidazol-5-yl)-3-(2-hydroxy-éthyl)]-3,6-dihydro-2*H*-1,3,4-thiadiazin-2-one,
- 3-(3-Amino-propyl)-5-(2-furan-2-yl-1-méthyl-1*H*-benzimidazol-5-yl)]-3,6-dihydro-2*H*-1,3,4-thiadiazin-2-one,
- 3-(2-Amino-éthyl)-5-[2-(3-fluoro-phényl)-1-méthyl-1*H*-benzimidazol-5-yl]-6-méthyl]-3,6-dihydro-2*H*-1,3,4-thiadiazin-2-one,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-2-phényl-acetamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-4-méthyl-benzamide,
- Cyclopropanecarboxylic acid (2-{5-[2-(3-fluoro-phényl)-1-méthyl-1*H-*benzimidazol-5-yl]-6-méthyl-2-oxo-6*H*-[1,3,4]thiadiazin-3-yl}-éthyl)-amide,
- Cyclopentanecarboxylic acid (2-{5-[2-(3-fluoro-phényl)-1-méthyl-1*H-*benzimidazol-5-yl]-6-méthyl-2-oxo-6*H*-[1,3,4]thiadiazin-3-yl}-éthyl)-amide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-2-méthoxy-acetamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-nicotinamide,
- N-(2-{5-[2-(3-Fluoro-phényl)-1-méthyl-1H-benzimidazol-5-yl]-6-méthyl-2-oxo-6*H*-[1,3,4]thiadiazin-3-yl}-éthyl)-benzamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-acétamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-isobutyramide,
- (S)-5-{2-[4-((S)-Méthanesulfinyl)-3-méthoxy-phényl]-1*H*-benzimidazol-5-yl}-6-méthyl]-3,6-dihydro-2*H*-1,3,4-thiadiazin-2-one,
- (R)-5-{2-[4-((S)-Méthanesulfinyl)-3-méthoxy-phényl]-1*H*-benzimidazol-5-yl}-6-méthyl]-3,6-dihydro-2*H*-1,3,4-thiadiazin-2-one,
- (S)-5-{2-[4-((R)-Méthanesulfinyl)-3-méthoxy-phényl]-1*H*-benzimidazol-5-yl}-6-méthyl]-3,6-dihydro-2*H*-1,3,4-thiadiazin-2-one,
- (R)-5-{2-[4-((R)-Méthanesulfinyl)-3-méthoxy-phényl]-1*H*-benzimidazol-5-yl}-6-méthyl]-3,6-dihydro-2*H*-1,3,4-thiadiazin-2-one,
- 1-(4-Acetyl-phényl)-3-(2-{5-[2-(3-fluoro-phényl)-1-méthyl-1*H*-benzimidazol-5-yl]-6-méthyl-2-oxo-6*H*-[1,3,4]thiadiazin-3-yl}-éthyl)-urée,
- 1-(3-Fluoro-phényl)-3-(2-{5-[2-(3-fluoro-phényl)-1-méthyl-1*H*-benzimidazol-5-yl]-6-méthyl-2-oxo-6*H*-[1,3,4]thiadiazin-3-yl}-éthyl)-urée,
- 5-[1-Cyclopropylméthyl-2-(1-méthyl-1*H*-pyrrol-2-yl)-1*H*-benzimidazol-5-yl]-6-méthyl]-3,6-dihydro-2*H*-1,3,4-thiadiazin-2-one,
- 5-[1-Cyclopropylméthyl-2-(1-méthyl-1*H*-imidazol-2-yl)-1*H*-benzimidazol-5-yl]-6-méthyl]-3,6-dihydro-2*H*-1,3,4-thiadiazin-2-one,
- 5-(1-Cyclopropylméthyl-2-thiophé-3-yl-1*H*-benzimidazol-5-yl)-6-méthyl]-3,6-dihydro-2*H*-1,3,4-thiadiazin-2-one,
- 5-[1-Cyclopropylméthyl-2-(2-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(4-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(3-hydroxy-4-méthoxy-phényl)-1H-benzimidazol-5-yl]-6-méthyl]-3,6-dihydro-2*H*-1,3,4-thiadiazin-2-one,

### Méthode de mesure de l'inhibition de la Fructose-1,6-bisphosphatase recombinante humaine de foie

La mesure d'activité enzymatique est réalisée par utilisation d'une méthode spectrophotométrique à l'aide de réactions couplant la formation du produit (Fructose-6-Phosphate) à la réduction du NADP+ via la phosphoglucoisomérase (PGI) et la glucose 6-phosphate déhydrogénase (G6PDH).

Les mélanges réactionnels (250 µl) sont réalisés en plaques 96 puits et sont composés de Triéthanolamine 20 mM, PH 7.5, MgCl₂ 2 mM, EDTA 0.1 mM, Sulfate d'ammonium 40 mM, NADP 0,5 mM, G6PDH 1 U/ml, PGI 1 U/ml et 0,167 mM de substrat (Fructose-1,6-bisphosphate).

Les inhibiteurs sont préparés à 10⁻²M dans le DMSO 100% et testés à 10⁻⁵M (DMSO 0.1% final)

Les réactions sont déclenchées par addition de l'enzyme recombinante humaine de foie, Fructose-1-6-bisphosphatase et suivies pendant 30 mn à 340 nm, à température ambiante, dans un lecteur de plaques Tecan.

### Inhibition de la Fructose-1,6-bisphosphatase recombinante humaine de foie

| **Exemple** | **Structure** | Inhibition de la F-1,6-BPase humaine IC₅₀ µM |
|---|---|---|
| **12-6** | 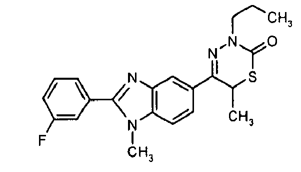 | 8,5 |
| **12-3** | 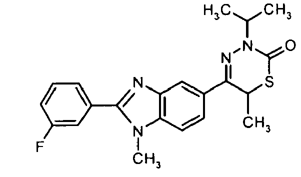 | 7 |
| **26-4** | 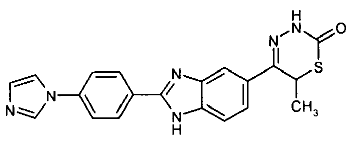 | 3,6 |
| **26-3** | 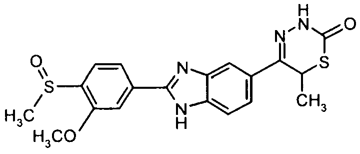 | 6,4 |
| **12-7** | 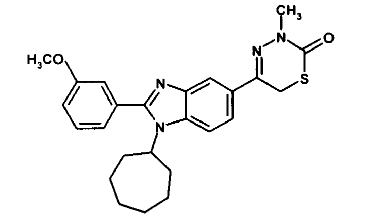 | 36 |

### Effets des différents composés préalablement décrits sur la production hépatique de glucose dans des cultures primaires d'hépatocytes de rat

Les hépatocytes sont isolés à partir de foie de rat Wistar par la technique de perfusion à la collagénase décrite par Seglen (Méthods Cell Biol. 1975 ; 13,29-83). La viabilité cellulaire est confirmée par le test d'exclusion au bleu trypan. Les hépatocytes isolés sont mis en suspension dans un milieu de William supplémenté avec du sérum de veau foetal (10%) dans des plaques 6 puits. Après une période d'attachement de 4 heures, le milieu est aspiré pour éliminer les débris cellulaires et remplacé par un milieu MEM sans sérum et sans glucose. Les hépatocytes sont ensuite cultivés pendant 16 à 18 heures (37°C; 5% CO₂). A la fin de la culture et après avoir aspiré le milieu, la production de glucose est mesurée en incubant les hépatocytes pendant 3 heures dans un tampon Krebs supplémenté avec de la Dihydroxyacetone (DHA) comme substrat de la néoglucogenèse en présence ou non des différents composés précédemment décrits. La quantité de glucose dans le milieu est mesurée dans chaque puits de culture par un test à la Glucose Oxydase (GOD). Le contenu en protéines cellulaires est évalué par la méthode de Lowry. Les résultats sont exprimés en nanomoles de glucose produit par mg de proteine cellulaire. L'activité des composés testés est exprimée en % du contrôle (production de glucose en présence de DHA mais sans composés).

### Inhibition de la production hépatique de glucose

| **Exemple** | **Structure** | **Production** hépatique de glucose. % du control à 30 µM |
|---|---|---|
| **26** | 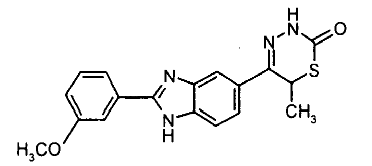 | 38 |
| **26-3** | 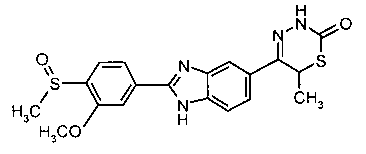 | 34 |
| **26-4** | 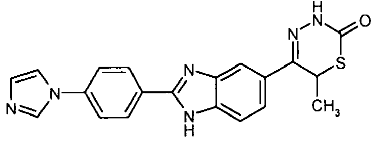 | 28 |

## Revendications

1. Composés de formule générale (I): 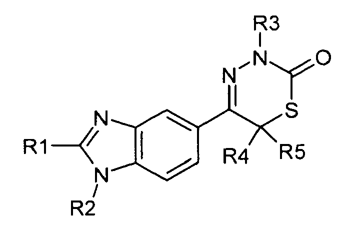 dans laquelle
R1 représente un groupe choisi indifférement parmi :
- Alkyle-, alcoxyalkyle-, alcényle-, alcynyle-, chacun de ces groupes pouvant être éventuellement substitué par un ou plusieurs groupes choisis parmi Y,
- Aryle-, arylalkyle-, aryloxyalkyle-, arylalkyloxyalkyle-, arylthioalkyle-, arylalkylthioalkyle-,
- Hétéroaryle-, hétéroarylalkyle-, hétéroaryloxyalkyle-, hétéroarylalkyloxyalkyle-, hétéroarylthioalkyle-, hétéroarylalkylthioalkyle-,
- Cycloalkyle-, cycloalkylalkyle-, cycloalkyloxyalkyle-, cycloalkylalkyloxyalkyle-, cycloalkylthioalkyle-, cycloalkylalkylthioalkyle-,
- Hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
chacun des groupes aryle-, hétéroaryle-, cycloalkyle-, hétérocycloalkyle- pouvant être éventuellement substitué par un ou plusieurs groupes choisis indifféremment parmi Y ;
R1 peut également représenter un groupe A-B- constitué de deux cycles A et B reliés par une liaison et dans lequel A et B représentent indépendamment un groupe choisi indifférement parmi :
- Aryle-, hétéroaryle-, cycloalkyle-, hétérocycloalkyle-,
chacun des groupes aryle-, hétéroaryle-, cycloalkyle-, hétérocycloalkyle- pouvant être éventuellement substitués par un ou plusieurs groupes choisis indifférement parmi Y.
R2 représente un groupe choisi indifféremment parmi :
- H,
- Alkyle-, alcoxyalkyle-, alcényle-, alcynyle-, chacun de ces groupes pouvant être éventuellement substitué par un ou plusieurs groupes choisis parmi Y,
- Aryle-, arylalkyle-, aryloxyalkyle-, arylalkyloxyalkyle-, arylthioalkyle-, arylalkylthioalkyle-,
- Hétéroaryle-, hétéroarylalkyle-, hétéroaryloxyalkyle-, hétéroarylalkyloxyalkyle-, hétéroarylthioalkyle-, hétéroarylalkylthioalkyle-,
- Cycloalkyle-, cycloalkylalkyle-, cycloalkyloxyalkyle-, cycloalkylalkyloxyalkyle-, cycloalkylthioalkyle-, cycloalkylalkylthioalkyle-,
- Hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
chacun des groupes aryle-, hétéroaryle-, cycloalkyle-, hétérocycloalkyle- pouvant être éventuellement substitués par un ou plusieurs groupes choisis indifféremment parmi Y ;
R3 représente un groupe choisi indifféremment parmi :
- H,
- Alkyle-, alcoxyalkyle-, hydroxyalkyl-, alcényle-, alcynyle-, chacun de ces groupes pouvant être éventuellement substitué par un ou plusieurs groupes choisis parmi
- Y,
- Aryle-, arylalkyle-, aryloxyalkyle-, arylalkyloxyalkyle-, arylthioalkyle-, arylalkylthioalkyle-,
- Hétéroaryle-, hétéroarylalkyle-, hétéroaryloxyalkyle-, hétéroarylalkyloxyalkyle-, hétéroarylthioalkyle-, hétéroarylalkylthioalkyle-,
- Cycloalkyle-, cycloalkylalkyle-, cycloalkyloxyalkyle-, cycloalkylalkyloxyalkyle-, cycloalkylthioalkyle-, cycloalkylalkylthioalkyle-,
- Hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
chacun des groupes aryle-, hétéroaryle-, cycloalkyle-, hétérocycloalkyle- pouvant être éventuellement substitués par un ou plusieurs groupes choisis indifférement parmi Y ;
R4 et R5 indépendamment représentent un groupe choisi indifférement parmi :
- H,
- W ;
Y représente un groupe choisi indifféremment parmi :
Hydroxy- ; thio- ; halogène- ; trifluorométhoxy- ; trifluorométhyle- ; alkyloxy- ; carboxyle- ; alcoxycarbonyle- ; carbamoyle- ; sulfamoyle- ; nitro- ; guanidino- ; amidino-; aryle- ; hétéroaryle- ; amino- ; 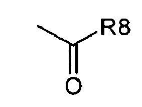
dans lequel R8 représente un groupe choisi indifféremment parmi W; 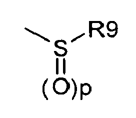 dans lequel R9 représente un groupe choisi indifférement parmi W et p=0, 1 ou 2 ;
-(CH₂)n-O-R10
dans lequel R10 représente un groupe choisi indifférement parmi
- H,
- W,
et n est un entier compris entre 0 à 8 ; 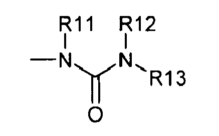 dans lequel R11, R12, R13 représentent indépendamment un groupe choisi indifférement parmi :
- H,
- W,
étant entendu que R12 et R13 peuvent former un système mono- ou bicyclique saturé ou non de 3 à 10 atomes comportant de 1 à 3 hétéroatomes; 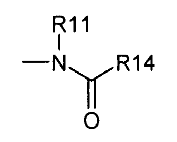
dans lequel R11 représente un groupe choisi indifféremment parmi :
- H,
- W,
et dans lequel R14 représente un groupe choisi indifféremment parmi W ; 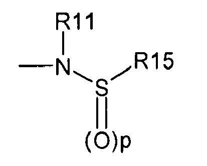
dans lequel R11 représente un groupe choisi indifféremment parmi :
- H,
- W,
et dans lequel R15 représente un groupe choisi indifféremment parmi W et p = 0,1,2 ;
où :
Amino désigne un groupe 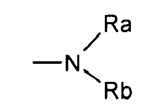
dans lequel Ra et Rb sont choisis indifférement parmi
- H,
- W,
étant entendu que Ra et Rb peuvent former un système mono- ou bicyclique saturé ou non de 3 à 10 atomes comportant de 1 à 3 hétéroatomes,
W représente un groupe choisi indifféremment parmi :
- Alkyle-, alcényle-, alcynyle-, aryle-, arylalkyle-, aryloxyalkyle-, arylalkyloxyalkyle-, arylthioalkyle-, arylalkylthioalkyle-, hétéroaryle-, hétéroarylalkyle-, hétéroaryloxyalkyle-, hétéroarylalkyloxyalkyle-, hétéroarylthioalkyle-, hétéroarylalkylthioalkyle-, cycloalkyle-, cycloalkylalkyle-, cycloalkyloxyalkyle-, cycloalkylalkyloxyalkyle-, cycloalkylthioalkyle-, cycloalkylalkylthioalkyle-, hétérocycloalkyle-, hétérocycloalkylalkyle-, hétérocycloalkyloxyalkyle-, hétérocycloalkylalkyloxyalkyle-, hétérocycloalkylthioalkyle-, hétérocycloalkylalkylthioalkyle-,
chacun de ces groupes pouvant être éventuellement substitué par un ou plusieurs groupes choisis indifférement parmi
hydroxy-, thio-, halogène-, trifluorométhoxy-, trifluorométhyle-, alkyloxy-, carboxyle-, alcoxycarbonyle-, carbamoyle-, sulfamoyle-, nitro-, guanidino-, amidino-, aryle-, hétéroaryle-, amino- qui a la même signification que ci dessus, acétyle- ;
ou leurs formes tautomères, énantiomères, diastéréoisomères, épimères, ainsi que les sels pharmaceutiquement acceptables, et les formes cristallines, ou leurs mélanges.

2. Composés selon la revendication 1 tels que dans la formule générale (I), R1 représente un groupe aryle éventuellement substitué par un à quatre groupes identiques ou différents indépendamment choisis parmi halogène, -OR, perhalogénoalkyle-, -S(O)ₚ-R où p est égal à 0, 1 ou 2, -NRR', -S-CH₂-CN ou hétéroaryle; ou un groupe -hétéroaryle, éventuellement substitué par un ou plusieurs groupes -alkyle ;
où R, R', identiques ou différents, sont indépendamment choisis parmi H, -alkyle, -aryle, -hétéroaryle, -cycloalkyle, -hétérocycloalkyle, étant entendu que R et R' peuvent former un système mono- ou bicyclique saturé ou non de 3 à 10 atomes comportant de 1 à 3 hétéroatomes,.

3. Composés selon la revendication 1 ou 2 tels que dans la formule générale (I), R4 et R5, identiques ou différents, sont indépendamment choisis parmi un atome d'hydrogène et un groupe -alkyle.

4. Composé selon l'une quelconque des revendications précédentes tels que dans la formule générale (I):
R1 représente un groupe aryle éventuellement substitué par un à quatre groupes identiques ou différents indépendamment choisis parmi halogène, -OR, perhalogénoalkyle-, -S(O)ₚ-R, -NRR', -S-CH₂-CN ou hétéroaryle; ou un groupe -hétéroaryle, éventuellement substitué par un ou plusieurs groupes -alkyle ;
R2 représente un atome d'hydrogène ; un groupe -cycloalkyle ; un groupe -alkyle éventuellement substitué par un groupe -cycloalkyle ou -aryle ;
R4 et R5, identiques ou différents, sont indépendamment choisis parmi un atome d'hydrogène et un groupe -alkyle ;
R3 représente :
- un atome d'hydrogène ;
- un groupe -alkyle, éventuellement substitué par un ou plusieurs groupes choisis parmi:
-OR, -OHétérocycloalkyle,
-cycloalkyle,
-hétérocycloalkyle,
-COOR,
-CONRR',
-NRR',
-NRCOalkyle, -NRCOalkyle-aryle, -NRCO-cycloalkyle, -NRCOaryle, -NRCOhétéroaryle, le groupe aryle étant éventuellement substitué par un atome d'halogène ou un groupe alkyle,
-NRCOOAlkyle,
-NRCO-NR-Alkyle, -NRCO-NR-Aryle ou -NRCO-NR-Alkyle-aryle, où le groupe aryle est éventuellement substitué par un atome d'halogène ou un groupe -COR ;
-NRCO-NR-Cycloalkyle,
-NRS(O)ₚ-aryle, -NRS(O)ₚ-alkyle,
-Aryle ou -OAryle, chaque groupe aryle étant éventuellement substitué par un ou plusieurs substituants choisis parmi les atomes d'halogène et les groupes -alkyle, -OR, perhalogénoalkyle-, perhalogénoalkyloxy-, -S(O)ₚ-R ;
p = 0, 1 ou 2 ;
R, R', identiques ou différents, sont indépendamment choisis parmi H, -alkyle, -aryle, -hétéroaryle, -cycloalkyle, -hétérocycloalkyle, étant entendu que R et R' peuvent former un système mono- ou bicyclique saturé ou non de 3 à 10 atomes comportant de 1 à 3 hétéroatomes,
ou leurs formes tautomères, énantiomères, diastéréoisomères, épimères, ainsi que les sels pharmaceutiquement acceptables, et les formes cristallines, ou leurs mélanges.

5. Composés selon l'une quelconque des revendications précédentes tels que dans la formule générale (I) :
R1 représente un groupe aryle éventuellement substitué par un à quatre groupes identiques ou différents indépendamment choisis parmi halogène, -Oalkyle, -S(O)p-alkyle où p=0, 1 ou 2 ;
R2 représente un atome d'hydrogène ; un groupe -cycloalkyle ; un groupe -alkyle;
R4 et R5, identiques ou différents, sont indépendamment choisis parmi un atome d'hydrogène et un groupe -alkyle ;
R3 représente :
- un atome d'hydrogène ;
- un groupe -alkyle,
et leurs formes tautomères, énantiomères, diastéréoisomères, épimères, ainsi que les sels pharmaceutiquement acceptables, et les formes cristallines, ou leurs mélanges.

6. Composés selon l'une quelconque des revendications précdentes choisis parmi :
- 5-[2-(4-méthoxyphényl)-1*H*-benzimidazol-5-yl]-3,6-dihydro-2*H*-1,3,4-thiadiazin-2-one,
- 6-méthyl-5-[2-(1*H*-pyrazol-3-yl)-1*H*-benzimidazol-5-yl]-3,6-dihydro-2*H*-1,3,4-thiadiazin-2-one,
- 6-méthyl-5-[2-(2-thienyl)-1*H*-benzimidazol-5-yl]-3,6-dihydro-2*H*-1,3,4-thiadiazin-2-one,
- 5-[2-(3-méthoxyphényl)-1*H*-benzimidazol-5-yl]-6-méthyl-3,6-dihydro-2*H*-1,3,4-thiadiazin-2-one,
- 6-méthyl-5-(2-pyridin-4-yl-1*H*-benzimidazol-5-yl)-3,6-dihydro-2*H*-1,3,4-thiadiazin-2-one,
- 5-{2-[3-méthoxy-4-(méthylthio)phényl]-1*H*-benzimidazol-5-yl}-6-méthyl-3,6-dihydro-2*H*-1,3,4-thiadiazin-2-one,
- 5-{2-[2-méthoxy-4-(méthylthio)phényl]-1*H*-benzimidazol-5-yl}-6-méthyl-3,6-dihydro-2*H*-1,3,4-thiadiazin-2-one,
- 6-méthyl-5-[2-(1,3-thiazol-4-yl)-1*H*-benzimidazol-5-yl]-3,6-dihydro-2*H*-1,3,4-thiadiazin-2-one,
- 5-{2-[3-méthoxy-4-(méthylsulfinyl)phényl]-1*H*-benzimidazol-5-yl}-6-méthyl-3,6-dihydro-2*H*-1,3,4-thiadiazin-2-one,
- 5-[2-(1*H*-imidazol-1-yl)-1*H*-benzimidazol-5-yl]-6-méthyl-3,6-dihydro-2*H*-1,3,4-thiadiazin-2-one,
- 5-{2-[4-(diméthylamino)phényl]-1*H*-benzimidazol-5-yl}-6-méthyl-3,6-dihydro-2*H*-1,3,4-thiadiazin-2-one,
- 6-méthyl-5-{2-[4-(méthylthio)phényl]-1*H*-benzimidazol-5-yl}-3,6-dihydro-2*H-*1,3,4-thiadiazin-2-one,
- 6-méthyl-5-{2-[4-(méthylsulfinyl)phényl]-1*H*-benzimidazol-5-yl}-3,6-dihydro-2*H-*1,3,4-thiadiazin-2-one,
- 5-[2-(2-méthoxyphényl)-1*H*-benzimidazol-5-yl]-6-méthyl-3,6-dihydro-2*H*-1,3,4-thiadiazin-2-one,
- 5-[2-(3-méthoxyphényl)-1*H*-benzimidazol-5-yl]-6-méthyl-3,6-dihydro-2*H*-1,3,4-thiadiazin-2-one,
- 3-méthoxy-4-[5-(6-méthyl-2-oxo-3,6-dihydro-2*H*-1,3,4-thiadiazin-5-yl)-1*H-*benzimidazol-2-yl]phényl thiocyanate,
- 5-{2-[2-chloro-4-(méthylthio)phényl]-1*H*-benzimidazol-5-yl}-6-méthyl-3,6-dihydro-2*H*-1,3,4-thiadiazin-2-one,
- 6-méthyl-5-{2-[4-(pyridin-4-ylamino)phényl]-1*H*-benzimidazol-5-yl}-3,6-dihydro-2*H*-1,3,4-thiadiazin-2-one,
- 5-[2-(4-méthoxyphényl)-1*H*-benzimidazol-5-yl]-3,6-diméthyl-3,6-dihydro-2*H-*1,3,4-thiadiazin-2-one,
- 3,6-diméthyl-5-[2-(1*H*-pyrazol-3-yl)-1*H*-benzimidazol-5-yl]-3,6-dihydro-2*H-*1,3,4-thiadiazin-2-one,
- 3,6-diméthyl-5-[2-(1,3-thiazol-4-yl)-1*H*-benzimidazol-5-yl]-3,6-dihydro-2*H-*1,3,4-thiadiazin-2-one,
- 3,6-diméthyl-5-(2-pyridin-4-yl-1*H*-benzimidazol-5-yl)-3,6-dihydro-2*H*-1,3,4-thiadiazin-2-one,
- 3-éthyl-6-méthyl-5-[2-(1*H*-pyrazol-3-yl)-1*H*-benzimidazol-5-yl]-3,6-dihydro-2*H-*1,3,4-thiadiazin-2-one,
- 3-éthyl-6-méthyl-5-[2-(1,3-thiazol-4-yl)-1*H*-benzimidazol-5-yl]-3,6-dihydro-2*H-*1,3,4-thiadiazin-2-one,
- 3-éthyl-5-[2-(4-méthoxyphényl)-1*H*-benzimidazol-5-yl]-6-méthyl-3,6-dihydro-2*H*-1,3,4-thiadiazin-2-one,
- 5-[1-Cycloheptyl-2-(3-méthoxy-phényl)-1*H*-benzimidazol-5-yl]-3,6-dihydro-2*H-*1,3,4-thiadiazin-2-one,
- 5-[1-Isobutyl-2-(3-méthoxy-phényl)-1*H*-benzimidazol-5-yl]-3,6-dihydro-2*H-*1,3,4-thiadiazin-2-one,
- 5-[1-Isobutyl-2-(3-méthoxy-phényl)-1*H*-benzimidazol-5-yl]-3-(4-méthyl-benzyl)-3,6-dihydro-2*H*-1,3,4-thiadiazin-2-one,
- 3-(4-tert-Butyl-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 5-[1-Cycloheptyl-2-(3-méthoxy-phényl)-1*H*-benzimidazol-5-yl]-3-méthyl]-3,6-dihydro-2*H*-1,3,4-thiadiazin-2-one,
- 5-[1-Cycloheptyl-2-(3-méthoxy-phényl)-1*H*-benzimidazol-5-yl]-3-propyl]-3,6-dihydro-2*H*-1,3,4-thiadiazin-2-one,
- 5-[1-Cycloheptyl-2-(3-méthoxy-phényl)-1*H*-benzimidazol-5-yl]-3-isobutyl]-3,6-dihydro-2*H*-1,3,4-thiadiazin-2-one,
- 5-[1-Cycloheptyl-2-(3-méthoxy-phényl)-1*H*-benzimidazol-5-yl]-3-(2-méthoxy-éthyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-méthyl-benzyl)]-3,6-dihydro-2*H*-1,3,4-thiadiazin-2-one,
- 3-(3-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-Benzyl-5-[1-isobutyl-2-(3-méthoxy-phényl)-1*H*-benzimidazol-5-yl]-3,6-dihydro-2*H*-1,3,4-thiadiazin-2-one,
- 3-(4-Fluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3-Chloro-benzyl)-5-[1-isobutyl-2-(3-méthoxy-phényl)-1*H*-benzimidazol-5-yl]-3,6-dihydro-2*H*-1,3,4-thiadiazin-2-one,
- 5-[1-Isobutyl-2-(3-méthoxy-phényl)-1*H*-benzimidazol-5-yl]-3-(3-méthoxy-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(4-trifluorométhoxy-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-trifluorométhyl-benzyl)]-3,6-dihydro-2*H*-1,3,4-thiadiazin-2-one,
- 5-[1-Isobutyl-2-(3-méthoxy-phényl)-1*H*-benzimidazol-5-yl]-3-(4-trifluorométhyl-benzyl)]-3,6-dihydro-2*H*-1,3,4-thiadiazin-2-one,
- 3-(2,3-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,4-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,5-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(2,6-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3,4-Difluoro-benzyl)-5-[1-isobutyl-2-(3-méthoxy-phényl)-1*H*-benzimidazol-5-yl]-3,6-dihydro-2*H*-1,3,4-thiadiazin-2-one,
- 3-(3,5-Diméthoxy-benzyl)-5-[1-isobutyl-2-(3-méthoxy-phényl)-1*H-*benzimidazol-5-yl]-3,6-dihydro-2*H*-1,3,4-thiadiazin-2-one,
- 3-Cyclopropylméthyl-5-[1-isobutyl-2-(3-méthoxy-phényl)-1*H*-benzimidazol-5-yl]-3,6-dihydro-2*H*-1,3,4-thiadiazin-2-one,
- 3-(2-Hydroxy-éthyl)-5-[1-isobutyl-2-(3-méthoxy-phényl)-1*H*-benzimidazol-5-yl]-3,6-dihydro-2*H*-1,3,4-thiadiazin-2-one,
- 3-Éthyl-5-[1-isobutyl-2-(3-méthoxy-phényl)-1*H*-benzimidazol-5-yl]-3,6-dihydro-2*H*-1,3,4-thiadiazin-2-one,
- 5-[1-Isobutyl-2-(3-méthoxy-phényl)-1*H*-benzimidazol-5-yl]-3-propyl]-3,6-dihydro-2*H*-1,3,4-thiadiazin-2-one,
- 5-[1-Isobutyl-2-(3-méthoxy-phényl)-1*H*-benzimidazol-5-yl]-3-isopropyl]-3,6-dihydro-2*H*-1,3,4-thiadiazin-2-one,
- 2-{5-[1-Isobutyl-2-(3-méthoxy-phényl)-1*H*-benzimidazol-5-yl]-2-oxo-6H-[1,3,4]thiadiazin-3-yl}-acetamide,
- 5-[1-Isobutyl-2-(3-méthoxy-phényl)-1*H*-benzimidazol-5-yl]-3-(2-méthoxy-éthyl)]-3,6-dihydro-2H-1,3,4-thiadiazin-2-one,
- {5-[1-Isobutyl-2-(3-méthoxy-phényl)-1*H*-benzimidazol-5-yl]-2-oxo-6H-[1,3,4]thiadiazin-3-yl}-acetic acid,
- 5-[1-Isobutyl-2-(3-méthoxy-phényl)-1*H*-benzimidazol-5-yl]-6-méthyl]-3,6-dihydro-2*H*-1,3,4-thiadiazin-2-one,
- 3-(2-Hydroxy-éthyl)-5-[1-isobutyl-2-(3-méthoxy-phényl)-1*H*-benzimidazol-5-yl]-6-méthyl]-3,6-dihydro-2*H*-1,3,4-thiadiazin-2-one,
- 5-[1-Isobutyl-2-(3-méthoxy-phényl)-1*H*-benzimidazol-5-yl]-3-propyl]-3,6-dihydro-2*H*-1,3,4-thiadiazin-2-one,
- 5-[1-Isobutyl-2-(3-méthoxy-phényl)-1*H*-benzimidazol-5-yl]-3,6-diméthyl]-3,6-dihydro-2*H*-1,3,4-thiadiazin-2-one,
- 3-Éthyl-5-[1-isobutyl-2-(3-méthoxy-phényl)-1*H*-benzimidazol-5-yl]-6-méthyl]-3,6-dihydro-2*H*-1,3,4-thiadiazin-2-one,
- 5-[1-Isobutyl-2-(3-méthoxy-phényl)-1*H*-benzimidazol-5-yl]-3-isopropyl-6-méthyl]-3,6-dihydro-2*H*-1,3,4-thiadiazin-2-one,
- 3-Cyclopropylméthyl-5-[1-isobutyl-2-(3-méthoxy-phényl)-1*H*-benzimidazol-5-yl]-6-méthyl]-3,6-dihydro-2*H*-1,3,4-thiadiazin-2-one,
- 3-Éthyl-5-[2-(3-méthoxy-phényl)-1-méthyl-1*H*-benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-3-propyl]-3,6-dihydro-2*H*-1,3,4-thiadiazin-2-one; hydrochloride,
- 3-Cyclohexylméthyl-5-[2-(3-méthoxy-phényl)-1-méthyl-1*H*-benzimidazol-5-yl]-3,6-dihydro-2*H*-1,3,4-thiadiazin-2-one,
- 3-[2-(4-Fluoro-phénoxy)-éthyl]-5-[2-(3-méthoxy-phényl)-1-méthyl-1*H-*benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-3-méthyl]-3,6-dihydro-2*H*-1,3,4-thiadiazin-2-one,
- 3-Isopropyl-5-[2-(3-méthoxy-phényl)-1-méthyl-1*H*-benzimidazol-5-yl]-3,6-dihydro-2*H*-1,3,4-thiadiazin-2-one,
- 3-Isobutyl-5-[2-(3-méthoxy-phényl)-1-méthyl-1*H*-benzimidazol-5-yl]-3,6-dihydro-2*H*-1,3,4-thiadiazin-2-one,
- 3-Cyclopropylméthyl-5-[2-(3-méthoxy-phényl)-1-méthyl-1*H*-benzimidazol-5-yl]-3,6-dihydro-2*H*-1,3,4-thiadiazin-2-one,
- 5-[2-(3-Méthoxy-phényl)-1-méthyl-1*H*-benzimidazol-5-yl]-6-méthyl]-3,6-dihydro-2*H*-1,3,4-thiadiazin-2-one,
- 3-Éthyl-5-[2-(3-méthoxy-phényl)-1-méthyl-1*H*-benzimidazol-5-yl]-6-méthyl]-3,6-dihydro-2*H*-1,3,4-thiadiazin-2-one,
- 5-[2-(3-Méthoxy-phényl)-1-méthyl-1*H*-benzimidazol-5-yl]-6-méthyl-3-propyl]-3,6-dihydro-2*H*-1,3,4-thiadiazin-2-one,
- 5-[2-(3-Méthoxy-phényl)-1-méthyl-1*H*-benzimidazol-5-yl]-6-méthyl-3-(2-piperidin-1-yl-éthyl)]-3,6-dihydro-2*H*-1,3,4-thiadiazin-2-one,
- 3-Isobutyl-5-[2-(3-méthoxy-phényl)-1-méthyl-1*H*-benzimidazol-5-yl]-6-méthyl]-3,6-dihydro-2*H*-1,3,4-thiadiazin-2-one,
- 5-[2-(3-Méthoxy-phényl)-1-méthyl-1*H*-benzimidazol-5-yl]-3,6-diméthyl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3,6-dihydro-2*H*-1,3,4-thiadiazin-2-one,
- 3-Benzyl-6-méthyl-5-[1-méthyl-2-(3-trifluorométhyl-phényl)-1*H*-benzimidazol-5-yl]-3,6-dihydro-2*H*-1,3,4-thiadiazin-2-one,
- 3-Cyclopropylméthyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 3-Isopropyl-6-méthyl-5-[1-méthyl-2-(3-trifluorométhyl-phényl)-1*H-*benzim idazol-5-yl]-3,6-d ihydro-2*H*-1,3,4-thiad iazin-2-one,
- 3,6-Diméthyl-5-[1-méthyl-2-(3-trifluorométhyl-phényl)-1*H*-benzimidazol-5-yl]-3,6-dihydro-2*H*-1,3,4-thiadiazin-2-one,
- 3-Éthyl-6-méthyl-5-[1-méthyl-2-(3-trifluorométhyl-phényl)-1*H*-benzimidazol-5-yl]-3,6-dihydro-2*H*-1,3,4-thiadiazin-2-one,
- 5-[1-Benzyl-2-(1-méthyl-1*H*-pyrrol-2-yl)-1*H*-benzimidazol-5-yl]-6-méthyl]-3,6-dihydro-2*H*-1,3,4-thiadiazin-2-one,
- 5-[1-Benzyl-2-(1-méthyl-1*H*-imidazol-2-yl)-1*H*-benzimidazol-5-yl]-6-méthyl]-3,6-dihydro-2*H*-1,3,4-thiadiazin-2-one,
- 5-(1-Benzyl-2-thiophé-2-yl-1*H*-benzimidazol-5-yl)-6-méthyl]-3,6-dihydro-2*H-*1,3,4-thiadiazin-2-one,
- 5-[1-Benzyl-2-(3-méthoxy-phényl)-1*H*-benzimidazol-5-yl]-6-méthyl]-3,6-dihydro-2*H*-1,3,4-thiadiazin-2-one,
- 5-[1-Benzyl-2-(4-méthoxy-phényl)-1*H*-benzimidazol-5-yl]-6-méthyl]-3,6-dihydro-2*H*-1,3,4-thiadiazin-2-one,
- 5-[1-Benzyl-2-(3-hydroxy-4-méthoxy-phényl)-1*H*-benzimidazol-5-yl]-6-méthyl]-3,6-dihydro-2*H*-1,3,4-thiadiazin-2-one,
- 5-[1-Benzyl-2-(2,4-dihydroxy-phényl)-1*H*-benzimidazol-5-yl]-6-méthyl]-3,6-dihydro-2*H*-1,3,4-thiadiazin-2-one,
- 5-[1-Benzyl-2-(3,4-dihydroxy-phényl)-1*H*-benzimidazol-5-yl]-6-méthyl]-3,6-dihydro-2*H*-1,3,4-thiadiazin-2-one,
- 5-[1-Benzyl-2-(3,4,5-trihydroxy-phényl)-1*H*-benzimidazol-5-yl]-6-méthyl]-3,6-dihydro-2*H*-1,3,4-thiadiazin-2-one,
- 6-Méthyl-5-[1-méthyl-2-(1-méthyl-1*H*-pyrrol-2-yl)-1H-benzimidazol-5-yl]-3,6-dihydro-2*H*-1,3,4-thiadiazin-2-one,
- 5-[2-(1*H*-Imidazol-4-yl)-1-méthyl-1*H*-benzimidazol-5-yl]-6-méthyl]-3,6-dihydro-2*H*-1,3,4-thiadiazin-2-one,
- 5-[2-(1*H*-Imidazol-2-yl)-1-méthyl-1*H*-benzimidazol-5-yl]-6-méthyl]-3,6-dihydro-2*H*-1,3,4-thiadiazin-2-one,
- 6-Méthyl-5-[1-méthyl-2-(5-méthyl-3*H*-imidazol-4-yl)-1 H-benzimidazol-5-yl]-3,6-dihydro-2*H*-1,3,4-thiadiazin-2-one,
- 6-Méthyl-5-[1-méthyl-2-(1-méthyl-1*H*-imidazol-2-yl)-1 H-benzimidazol-5-yl]-3,6-dihydro-2*H*-1,3,4-thiadiazin-2-one,
- 6-Méthyl-5-(1-méthyl-2-thiophé-2-yl-1*H*-benzimidazol-5-yl)]-3,6-dihydro-2*H-*1,3,4-thiadiazin-2-one,
- 6-Méthyl-5-(1-méthyl-2-thiophé-3-yl-1*H*-benzimidazol-5-yl)]-3,6-dihydro-2*H-*1,3,4-thiadiazin-2-one,
- 5-[2-(2-Méthoxy-phényl)-1-méthyl-1*H*-benzimidazol-5-yl]-6-méthyl]-3,6-dihydro-2*H*-1,3,4-thiadiazin-2-one,
- 5-[2-(4-Méthoxy-phényl)-1-méthyl-1*H*-benzimidazol-5-yl]-6-méthyl]-3,6-dihydro-2*H*-1,3,4-thiadiazin-2-one,
- 5-[2-(3-Hydroxy-4-méthoxy-phényl)-1-méthyl-1*H*-benzimidazol-5-yl]-6-méthyl]-3,6-dihydro-2*H*-1,3,4-thiadiazin-2-one,
- 5-[2-(3,4-Dihydroxy-phényl)-1-méthyl-1*H*-benzimidazol-5-yl]-6-méthyl]-3,6-dihydro-2*H*-1,3,4-thiadiazin-2-one,
- 6-Méthyl-5-[1-méthyl-2-(3,4,5-trihydroxy-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(3-méthyl-benzyl)]-3,6-dihydro-2*H*-1,3,4-thiadiazin-2-one,
- 5-[1-Éthyl-2-(3-fluoro-phényl)-1*H*-benzimidazol-5-yl]-3-(4-méthyl-benzyl)]-3,6-dihydro-2*H*-1,3,4-thiadiazin-2-one,
- 3-(4-tert-Butyl-benzyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(2-fluoro-benzyl)]-3,6-dihydro-2*H*-1,3,4-thiadiazin-2-one,
- 5-[1-Éthyl-2-(3-fluoro-phényl)-1*H*-benzimidazol-5-yl]-3-(3-fluoro-benzyl)]-3,6-dihydro-2*H*-1,3,4-thiadiazin-2-one,
- 3-Benzyl-5-[1-éthyl-2-(3-fluoro-phényl)-1*H*-benzimidazol-5-yl]-3,6-dihydro-2*H-*1,3,4-thiadiazin-2-one,
- 3-(3-Chloro-benzyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-méthyl]-3,6-dihydro-2*H-*1,3,4-thiadiazin-2-one,
- 3-Cyclohexylméthyl-5-[1-éthyl-2-(3-fluoro-phényl)-1*H*-benzimidazol-5-yl]-3,6-dihydro-2*H*-1,3,4-thiadiazin-2-one,
- 3-(2-Diméthylamino-éthyl)-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(2-pyrrolidin-1-yl-éthyl)]-3,6-dihydro-2*H*-1,3,4-thiadiazin-2-one,
- 5-[1-Isobutyl-2-(3-méthoxy-phényl)-1*H*-benzimidazol-5-yl]-3-(3-méthyl-benzyl)]-3,6-dihydro-2*H*-1,3,4-thiadiazin-2-one,
- 5-[2-(4-Méthoxy-phényl)-1*H*-benzimidazol-5-yl]-6-méthyl]-3,6-dihydro-2*H-*1,3,4-thiadiazin-2-one,
- 3-(2-Diméthylamino-éthyl)-6-méthyl-5-[1-méthyl-2-(3-trifluorométhyl-phényl)-1*H*-benzimidazol-5-yl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-piperidin-1-yl-éthyl)]-3,6-dihydro-2*H*-1,3,4-thiadiazin-2-one,
- 3-Cyclohexylméthyl-6-méthyl-5-[1-méthyl-2-(3-trifluorométhyl-phényl)-1*H-*benzimidazol-5-yl]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-morpholin-4-yl-éthyl)]-3,6-dihydro-2*H*-1,3,4-thiadiazin-2-one,
- 6-Méthyl-5-[1-méthyl-2-(3-trifluorométhyl-phényl)-1*H*-benzimidazol-5-yl]-3-(2-pyrrolidin-1-yl-éthyl)]-3,6-dihydro-2*H*-1,3,4-thiadiazin-2-one,
- 2-{6-Méthyl-5-[1-méthyl-2-(3-trifluorométhyl-phényl)-1*H*-benzimidazol-5-yl]-2-oxo-6*H*-[1,3,4]thiadiazin-3-yl}-acetamide,
- 3-(2-Méthoxy-éthyl)-6-méthyl-5-[1-méthyl-2-(3-trifluorométhyl-phényl)-1*H-*benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-(2-hydroxy-éthyl)-6-méthyl]-3,6-dihydro-2*H*-1,3,4-thiadiazin-2-one,
- 3-Cyclohexylméthyl-5-[2-(3-fluoro-phényl)-1-méthyl-1*H*-benzimidazol-5-yl]-6-méthyl]-3,6-dihydro-2*H*-1,3,4-thiadiazin-2-one,
- 2-{5-[2-(3-Fluoro-phényl)-1-méthyl-1*H*-benzimidazol-5-yl]-6-méthyl-2-oxo-6H-[1,3,4]thiadiazin-3-yl}-acetamide,
- 3-[2-(4-Fluoro-phénoxy)-éthyl]-5-[2-(3-fluoro-phényl)-1-méthyl-1*H-*benzimidazol-5-yl]-6-méthyl]-3,6-dihydro-2*H*-1,3,4-thiadiazin-2-one,
- 5-[2-(3-Fluoro-phényl)-1-méthyl-1*H*-benzimidazol-5-yl]-3-isopropyl-6-méthyl]-3,6-dihydro-2*H*-1,3,4-thiadiazin-2-one,
- 5-[2-(3-Fluoro-phényl)-1-méthyl-1*H*-benzimidazol-5-yl]-3-isobutyl-6-méthyl]-3,6-dihydro-2*H*-1,3,4-thiadiazin-2-one,
- 4-{5-[2-(3-Fluoro-phényl)-1-méthyl-1*H*-benzimidazol-5-yl]-6-méthyl-2-oxo-6H-[1,3,4]thiadiazin-3-yl}-butyric acid éthyl ester,
- 3-{5-[2-(3-Fluoro-phényl)-1-méthyl-1*H*-benzimidazol-5-yl]-6-méthyl-2-oxo-6*H-*[1,3,4]thiadiazin-3-yl}-propionic acid éthyl ester,
- 3-Cyclopropylméthyl-5-[2-(3-fluoro-phényl)-1-méthyl-1*H*-benzimidazol-5-yl]-6-méthyl]-3,6-dihydro-2*H*-1,3,4-thiadiazin-2-one,
- 5-[2-(3-Fluoro-phényl)-1-méthyl-1*H*-benzimidazol-5-yl]-3-(2-méthoxy-éthyl)-6-méthyl]-3,6-dihydro-2*H*-1,3,4-thiadiazin-2-one,
- 3-Benzyl-5-[2-(3-fluoro-phényl)-1-méthyl-1*H*-benzimidazol-5-yl]-6-méthyl]-3,6-dihydro-2*H*-1,3,4-thiadiazin-2-one,
- {5-[2-(3-Fluoro-phényl)-1-méthyl-1*H*-benzimidazol-5-yl]-6-méthyl-2-oxo-6*H-*[1,3,4]thiadiazin-3-yl}-acetic acid éthyl ester,
- 5-[2-(3-Fluoro-phényl)-1-méthyl-1*H*-benzimidazol-5-yl]-6-méthyl-3-propyl]-3,6-dihydro-2*H*-1,3,4-thiadiazin-2-one,
- 5-[2-(3-Fluoro-phényl)-1-méthyl-1*H*-benzimidazol-5-yl]-6-méthyl-3-(2-morpholin-4-yl-éthyl)]-3,6-dihydro-2*H*-1,3,4-thiadiazin-2-one
- 5-[2-(3-Fluoro-phényl)-1-méthyl-1*H*-benzimidazol-5-yl]-6-méthyl-3-(2-piperidin-1-yl-éthyl)]-3,6-dihydro-2*H*-1,3,4-thiadiazin-2-one,
- 5-[2-(3-Fluoro-phényl)-1-méthyl-1*H*-benzimidazol-5-yl]-6-méthyl]-3,6-dihydro-2*H*-1,3,4-thiadiazin-2-one,
- 3-Cyclohexylméthyl-5-[2-(3-fluoro-phényl)-1-méthyl-1*H*-benzimidazol-5-yl]-3,6-dihydro-2*H*-1,3,4-thiadiazin-2-one,
- 2-{5-[2-(3-Fluoro-phényl)-1-méthyl-1*H*-benzimidazol-5-yl]-2-oxo-6*H-*[1,3,4]thiadiazin-3-yl}-acetamide,
- 3-[2-(4-Fluoro-phénoxy)-éthyl]-5-[2-(3-fluoro-phényl)-1-méthyl-1*H-*benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-isopropyl]-3,6-dihydro-2*H*-1,3,4-thiadiazin-2-one,
- 5-[2-(3-Fluoro-phényl)-1-méthyl-1*H*-benzimidazol-5-yl]-3-isobutyl]-3,6-dihydro-2*H*-1,3,4-thiadiazin-2-one,
- 3-Cyclopropylméthyl-5-[2-(3-fluoro-phényl)-1-méthyl-1*H*-benzimidazol-5-yl]-3,6-dihydro-2*H*-1,3,4-thiadiazin-2-one
- 5-[2-(3-Fluoro-phényl)-1-méthyl-1*H*-benzimidazol-5-yl]-3-(2-méthoxy-éthyl)]-3,6-dihydro-2*H*-1,3,4-thiadiazin-2-one,
- 3-Benzyl-5-[2-(3-fluoro-phényl)-1-méthyl-1*H*-benzimidazol-5-yl]-3,6-dihydro-2*H*-1,3,4-thiadiazin-2-one,
- {5-[2-(3-Fluoro-phényl)-1-méthyl-1*H*-benzimidazol-5-yl]-2-oxo-6H-[1,3,4]thiadiazin-3-yl}-acetic acid éthyl ester,
- 5-[2-(3-Fluoro-phényl)-1-méthyl-1*H*-benzimidazol-5-yl]-3-(2-piperidin-1-yl-éthyl)]-3,6-dihydro-2*H*-1,3,4-thiadiazin-2-one,
- 5-[2-(3-Fluoro-phényl)-1-méthyl-1*H*-benzimidazol-5-yl]-3,6-dihydro-2*H*-1,3,4-thiadiazin-2-one,
- 5-[2-(3-Fluoro-phényl)-1-méthyl-1*H*-benzimidazol-5-yl]-3-propyl]-3,6-dihydro-2*H*-1,3,4-thiadiazin-2-one,
- 5-(1-Benzyl-2-thiophé-3-yl-1*H*-benzimidazol-5-yl)-6-méthyl]-3,6-dihydro-2*H-*1,3,4-thiadiazin-2-one,
- 5-[1-Benzyl-2-(2-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(3-méthoxy-phényl)-1*H*-benzimidazol-5-yl]-6-méthyl]-3,6-dihydro-2*H*-1,3,4-thiadiazin-2-one,
- 3-Cyclohexylméthyl-5-(2-furan-2-yl-1-méthyl-1*H*-benzimidazol-5-yl)]-3,6-dihydro-2*H*-1,3,4-thiadiazin-2-one,
- 3-Benzyl-5-(2-furan-2-yl-1-méthyl-1*H*-benzimidazol-5-yl)]-3,6-dihydro-2*H-*1,3,4-thiadiazin-2-one,
- 3-Cyclopropylméthyl-5-(2-furan-2-yl-1-méthyl-1*H*-benzimidazol-5-yl)]-3,6-dihydro-2*H*-1,3,4-thiadiazin-2-one,
- 5-(2-Furan-2-yl-1-méthyl-1*H*-benzimidazol-5-yl)-3-méthyl]-3,6-dihydro-2*H-*1,3,4-thiadiazin-2-one,
- 3-Éthyl-5-(2-furan-2-yl-1-méthyl-1*H*-benzimidazol-5-yl)]-3,6-dihydro-2*H*-1,3,4-thiadiazin-2-one,
- 5-(2-Furan-2-yl-1-méthyl-1*H*-benzimidazol-5-yl)-3-(2-piperidin-1-yl-éthyl)]-3,6-dihydro-2*H*-1,3,4-thiadiazin-2-one,
- 5-(2-Furan-2-yl-1-méthyl-1*H*-benzimidazol-5-yl)-3-(2-pyrrolidin-1-yl-éthyl)]-3,6-dihydro-2*H*-1,3,4-thiadiazin-2-one,
- 5-(2-Furan-2-yl-1-méthyl-1*H*-benzimidazol-5-yl)-3-isobutyl]-3,6-dihydro-2*H-*1,3,4-thiadiazin-2-one,
- 5-(2-Furan-2-yl-1-méthyl-1*H*-benzimidazol-5-yl)]-3,6-dihydro-2*H*-1,3,4-thiadiazin-2-one,
- 2-{5-[2-(3-Méthoxy-phényl)-1-méthyl-1*H*-benzimidazol-5-yl]-6-méthyl-2-oxo-6*H*-[1,3,4]thiadiazin-3-yl}-acetamide,
- 3-Isopropyl-5-[2-(3-méthoxy-phényl)-1-méthyl-1*H*-benzimidazol-5-yl]-6-méthyl]-3,6-dihydro-2*H*-1,3,4-thiadiazin-2-one,
- 3-(4-tert-Butyl-benzyl)-5-[1-méthyl-2-(3-trifluorométhyl-phényl)-1*H-*benzimidazol-5-yl]-3,6-dihydro-2*H*-1,3,4-thiadiazin-2-one,
- 3-(4-Fluoro-benzyl)-5-[1-méthyl-2-(3-trifluorométhyl-phényl)-1*H*-benzimidazol-5-yl]-3,6-dihydro-2*H*-1,3,4-thiadiazin-2-one,
- 5-[1-Méthyl-2-(3-trifluorométhyl-phényl)-1*H*-benzimidazol-5-yl]-3-(4-trifluorométhoxy-benzyl)]-3,6-dihydro-2*H*-1,3,4-thiadiazin-2-one,
- 5-[1-Méthyl-2-(3-trifluorométhyl-phényl)-1*H*-benzimidazol-5-yl]-3-(4-trifluorométhyl-benzyl)]-3,6-dihydro-2*H*-1,3,4-thiadiazin-2-one,
- 3-{5-[1-Méthyl-2-(3-trifluorométhyl-phényl)-1*H*-benzimidazol-5-yl]-2-oxo-6H-[1,3,4]thiadiazin-3-ylméthyl}-benzonitrile,
- 3-(4-Méthanesulfonyl-benzyl)-5-[1-méthyl-2-(3-trifluorométhyl-phényl)-1*H-*benzimidazol-5-yl]-3,6-dihydro-2*H*-1,3,4-thiadiazin-2-one,
- 3-(3,5-Diméthoxy-benzyl)-5-[1-méthyl-2-(3-trifluorométhyl-phényl)-1*H-*benzimidazol-5-yl]-3,6-dihydro-2*H*-1,3,4-thiadiazin-2-one,
- 5-[1-Méthyl-2-(3-trifluorométhyl-phényl)-1*H*-benzimidazol-5-yl]-3,6-dihydro-2*H-*1,3,4-thiadiazin-2-one,
- 5-[1-Éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-(4-fluoro-benzyl)]-3,6-dihydro-2*H*-1,3,4-thiadiazin-2-one,
- 5-[1-Éthyl-2-(3-fluoro-phényl)-1*H*-benzimidazol-5-yl]-3-(3-méthoxy-benzyl)]-3,6-dihydro-2*H*-1,3,4-thiadiazin-2-one,
- 5-[1-Éthyl-2-(3-fluoro-phényl)-1*H*-benzimidazol-5-yl]-3-(4-trifluorométhoxy-benzyl)]-3,6-dihydro-2*H*-1,3,4-thiadiazin-2-one,
- 2-{5-[1-Éthyl-2-(3-fluoro-phényl)-1*H*-benzimidazol-5-yl]-2-oxo-6H-[1,3,4]thiadiazin-3-yl}-acetamide,
- 3-Cyclopropylméthyl-5-[1-éthyl-2-(3-fluoro-phényl)-1*H*-benzimidazol-5-yl]-3,6-dihydro-2*H*-1,3,4-thiadiazin-2-one,
- 5-[1-Éthyl-2-(3-fluoro-phényl)-1*H*-benzimidazol-5-yl]-3-isopropyl]-3,6-dihydro-2*H*-1,3,4-thiadiazin-2-one,
- 3-Éthyl-5-[1-éthyl-2-(3-fluoro-phényl)-1*H*-benzimidazol-5-yl]-3,6-dihydro-2*H-*1,3,4-thiadiazin-2-one,
- 5-[1-Éthyl-2-(3-fluoro-phényl)-1*H*-benzimidazol-5-yl]-3-propyl]-3,6-dihydro-2*H-*1,3,4-thiadiazin-2-one,
- 5-[1-Éthyl-2-(3-fluoro-phényl)-1*H*-benzimidazol-5-yl]-3-(2-méthoxy-éthyl)]-3,6-dihydro-2*H*-1,3,4-thiadiazin-2-one,
- {5-[1-Éthyl-2-(3-fluoro-phényl)-1*H*-benzimidazol-5-yl]-2-oxo-6*H-*[1,3,4]thiadiazin-3-yl}-acetic acid,
- 5-[1-Éthyl-2-(3-fluoro-phényl)-1*H*-benzimidazol-5-yl]-3-(2-hydroxy-éthyl)]-3,6-dihydro-2*H*-1,3,4-thiadiazin-2-one,
- 5-[1-Éthyl-2-(3-fluoro-phényl)-1*H*-benzimidazol-5-yl]-3-(3-hydroxy-propyl)]-3,6-dihydro-2*H*-1,3,4-thiadiazin-2-one,
- 3-(2-Amino-éthyl)-5-[1-éthyl-2-(3-fluoro-phényl)-1*H*-benzimidazol-5-yl]-3,6-dihydro-2*H*-1,3,4-thiadiazin-2-one,
- 3-(3-Amino-propyl)-5-[1-éthyl-2-(3-fluoro-phényl)-1*H*-benzimidazol-5-yl]-3,6-dihydro-2*H*-1,3,4-thiadiazin-2-one,
- 3-Benzyl-5-[1-éthyl-2-(3-méthoxy-phényl)-1*H*-benzimidazol-5-yl]-3,6-dihydro-2*H*-1,3,4-thiadiazin-2-one,
- 5-[1-Éthyl-2-(3-méthoxy-phényl)-1*H*-benzimidazol-5-yl]-3-(4-méthyl-benzyl)]-3,6-dihydro-2*H*-1,3,4-thiadiazin-2-one,
- 5-[1-Éthyl-2-(3-méthoxy-phényl)-1*H*-benzimidazol-5-yl]-3-(2-fluoro-benzyl)]-3,6-dihydro-2*H*-1,3,4-thiadiazin-2-one,
- 3-Benzyl-5-[1-méthyl-2-(3-trifluorométhyl-phényl)-1*H*-benzimidazol-5-yl]-3,6-dihydro-2*H*-1,3,4-thiadiazin-2-one,
- 3-Méthyl-5-[1-méthyl-2-(3-trifluorométhyl-phényl)-1*H*-benzimidazol-5-yl]-3,6-dihydro-2*H*-1,3,4-thiadiazin-2-one,
- 3-Cyclohexylméthyl-5-[1-méthyl-2-(3-trifluorométhyl-phényl)-1*H*-benzimidazol-5-yl]-3,6-dihydro-2*H*-1,3,4-thiadiazin-2-one,
- 3-(3-Amino-propyl)-5-[1-méthyl-2-(3-trifluorométhyl-phényl)-1*H*-benzimidazol-5-yl]-3,6-dihydro-2*H*-1,3,4-thiadiazin-2-one,
- 3-(2-Amino-éthyl)-5-[1-méthyl-2-(3-trifluorométhyl-phényl)-1*H*-benzimidazol-5-yl]-3,6-dihydro-2*H*-1,3,4-thiadiazin-2-one,
- 3-(2-Amino-éthyl)-5-[1-éthyl-2-(3-méthoxy-phényl)-1*H*-benzimidazol-5-yl]-3,6-dihydro-2*H*-1,3,4-thiadiazin-2-one,
- 3-(3-Amino-propyl)-5-[1-éthyl-2-(3-méthoxy-phényl)-1*H*-benzimidazol-5-yl]-3,6-dihydro-2*H*-1,3,4-thiadiazin-2-one,
- 5-[1-Éthyl-2-(3-méthoxy-phényl)-1*H*-benzimidazol-5-yl]-3-(2-hydroxy-éthyl)]-3,6-dihydro-2*H*-1,3,4-thiadiazin-2-one
- 5-[1-Éthyl-2-(3-méthoxy-phényl)-1*H*-benzimidazol-5-yl]-3-(3-hydroxy-propyl)]-3,6-dihydro-2*H*-1,3,4-thiadiazin-2-one,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-acetamide,
- Furan-2-carboxylic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-(2-{5-[1-Méthyl-2-(3-trifluorométhyl-phényl)-1*H*-benzimidazol-5-yl]-2-oxo-6H-[1,3,4]thiadiazin-3-yl}-éthyl)-isobutyramide,
- Cyclopentanecarboxylic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-benzamide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-méthanesulfonamide,
- N-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-benzenesulfonamide,
- 5-[1-Méthyl-2-(3-trifluorométhyl-phényl)-1*H*-benzimidazol-5-yl]-6-propyl]-3,6-dihydro-2*H*-1,3,4-thiadiazin-2-one,
- 3-Éthyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-3,6-dipropyl]-3,6-dihydro-2*H*-1,3,4-thiadiazin-2-one,
- 3-Benzyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Cyclopropylméthyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Isopropyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 3-Isobutyl-5-[1-méthyl-2-(3-trifluorométhyl-phényl)-1*H*-benzimidazol-5-yl]-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-3-(2-piperidin-1-yl-éthyl)-6-propyl]-3,6-dihydro-2*H*-1,3,4-thiadiazin-2-one,
- 5-[1-Méthyl-2-(3-trifluorométhyl-phényl)-1*H*-benzimidazol-5-yl]-6-propyl-3-(2-pyrrolidin-1-yl-éthyl)]-3,6-dihydro-2*H*-1,3,4-thiadiazin-2-one,
- 1-(2-{5-[1-Méthyl-2-(3-trifluorométhyl-phényl)-1*H*-benzimidazol-5-yl]-2-oxo-6*H-*[1,3,4]thiadiazin-3-yl}-éthyl)-3-phényl-urée,
- 1-Éthyl-3-(2-{5-[1-méthyl-2-(3-trifluorométhyl-phényl)-1*H*-benzimidazol-5-yl]-2-oxo-6*H*-[1,3,4]thiadiazin-3-yl}-éthyl)-urée,
- 5-[1-Éthyl-2-(3-méthoxy-phényl)-1*H*-benzimidazol-5-yl]-3,6-dihydro-2*H*-1,3,4-thiadiazin-2-one,
- 3-Cyclopropylméthyl-5-[1-méthyl-2-(3-trifluorométhyl-phényl)-1*H*-benzimidazol-5-yl]-3,6-dihydro-2*H*-1,3,4-thiadiazin-2-one,
- Butane-1-sulfonic acid (2-{5-[1-méthyl-2-(3-trifluorométhyl-phényl)-1*H-*benzimidazol-5-yl]-2-oxo-6*H*-[1,3,4]thiadiazin-3-yl}-éthyl)-amide,
- N-{2-[5-(2-Furan-2-yl-1-méthyl-1*H*-benzimidazol-5-yl)-2-oxo-6*H-*[1,3,4]thiadiazin-3-yl]-éthyl}-acetamide,
- N-{2-[5-(2-Furan-2-yl-1-méthyl-1*H*-benzimidazol-5-yl)-2-oxo-6H-[1,3,4]thiadiazin-3-yl]-éthyl}-isobutyramide,
- Cyclopentanecarboxylic acid {2-[5-(2-furan-2-yl-1-méthyl-1*H*-benzimidazol-5-yl)-2-oxo-6*H*-[1,3,4]thiadiazin-3-yl]-éthyl}-amide,
- N-{2-[5-(2-Furan-2-yl-1-méthyl-1*H*-benzimidazol-5-yl)-2-oxo-6*H-*[1,3,4]thiadiazin-3-yl]-éthyl}-benzamide,
- N-{2-[5-(2-Furan-2-yl-1-méthyl-1*H*-benzimidazol-5-yl)-2-oxo-6*H-*[1,3,4]thiadiazin-3-yl]-éthyl}-nicotinamide,
- Cyclopropanecarboxylic acid {2-[5-(2-furan-2-yl-1-méthyl-1*H*-benzimidazol-5-yl)-2-oxo-6*H*-[1,3,4]thiadiazin-3-yl]-éthyl}-amide,
- 3-Fluoro-N-{2-[5-(2-furan-2-yl-1-méthyl-1*H*-benzimidazol-5-yl)-2-oxo-6*H-*[1,3,4]thiadiazin-3-yl]-éthyl}-benzamide,
- N-{2-[5-(2-Furan-2-yl-1-méthyl-1*H*-benzimidazol-5-yl)-2-oxo-6*H-*[1,3,4]thiadiazin-3-yl]-éthyl}-4-méthyl-benzamide,
- 3-(2-Amino-éthyl)-5-(2-furan-2-yl-1-méthyl-1*H*-benzimidazol-5-yl)]-3,6-dihydro-2*H*-1,3,4-thiadiazin-2-one,
- N-{2-[5-(2-Furan-2-yl-1-méthyl-1*H*-benzimidazol-5-yl)-2-oxo-6*H-*[1,3,4]thiadiazin-3-yl]-éthyl}-benzenesulfonamide,
- Butane-1-sulfonic acid {2-[5-(2-furan-2-yl-1-méthyl-1*H*-benzimidazol-5-yl)-2-oxo-6*H*-[1,3,4]thiadiazin-3-yl]-éthyl}-amide,
- 1-{2-[5-(2-Furan-2-yl-1-méthyl-1*H*-benzimidazol-5-yl)-2-oxo-6*H-*[1,3,4]thiadiazin-3-yl]-éthyl}-3-isopropyl-urée,
- 1-{2-[5-(2-Furan-2-yl-1-méthyl-1*H*-benzimidazol-5-yl)-2-oxo-6*H-*[1,3,4]thiadiazin-3-yl]-éthyl}-3-phényl-urée,
- 1-Cyclopentyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-Éthyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-Benzyl-3-{2-[5-(2-furan-2-yl-1-méthyl-1*H*-benzimidazol-5-yl)-2-oxo-6*H-*[1,3,4]thiadiazin-3-yl]-éthyl}-urée,
- 1-(4-Fluoro-benzyl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-(4-Acetyl-phényl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-{2-[5-(2-Furan-2-yl-1-méthyl-1*H*-benzimidazol-5-yl)-2-oxo-6*H-*[1,3,4]thiadiazin-3-yl]-éthyl}-3-p-tolyl-urée,
- 1-Butyl-3-{2-[5-(2-furan-2-yl-1-méthyl-1*H*-benzimidazol-5-yl)-2-oxo-6*H-*[1,3,4]thiadiazin-3-yl]-éthyl}-urée,
- N-{3-[5-(2-Furan-2-yl-1-méthyl-1*H*-benzimidazol-5-yl)-2-oxo-6*H-*[1,3,4]thiadiazin-3-yl]-propyl}-benzamide,
- Cyclopropanecarboxylic acid {3-[5-(2-furan-2-yl-1-méthyl-1*H*-benzimidazol-5-yl)-2-oxo-6*H*-[1,3,4]thiadiazin-3-yl]-propyl}-amide,
- 1-(4-Chloro-phényl)-3-{2-[5-(2-furan-2-yl-1-méthyl-1*H*-benzimidazol-5-yl)-2-oxo-6*H*-[1,3,4]thiadiazin-3-yl]-éthyl}-urée,
- 1-(4-Chloro-phényl)-3-{3-[5-(2-furan-2-yl-1-méthyl-1*H*-benzimidazol-5-yl)-2-oxo-6*H*-[1,3,4]thiadiazin-3-yl]-propyl}-urée,
- 1-Butyl-3-{3-[5-(2-furan-2-yl-1-méthyl-1*H*-benzimidazol-5-yl)-2-oxo-6*H-*[1,3,4]thiadiazin-3-yl]-propyl}-urée,
- 1-Benzyl-3-{3-[5-(2-furan-2-yl-1-méthyl-1*H*-benzimidazol-5-yl)-2-oxo-6*H-*[1,3,4]thiadiazin-3-yl]-propyl}-urée,
- 1-(3-Fluoro-phényl)-3-{3-[5-(2-furan-2-yl-1-méthyl-1*H*-benzimidazol-5-yl)-2-oxo-6*H*-[1,3,4]thiadiazin-3-yl]-propyl}-urée
- N-{2-[5-(2-Furan-2-yl-1-méthyl-1*H*-benzimidazol-5-yl)-2-oxo-6*H-*[1,3,4]thiadiazin-3-yl]-éthyl}-méthanesulfonamide,
- 5-(2-Furan-2-yl-1-méthyl-1*H*-benzimidazol-5-yl)-3-(2-méthoxy-éthyl)]-3,6-dihydro-2*H*-1,3,4-thiadiazin-2-one,
- 5-(2-Furan-2-yl-1-méthyl-1*H*-benzimidazol-5-yl)-3-(3-hydroxy-propyl)]-3,6-dihydro-2*H*-1,3,4-thiadiazin-2-one,
- 5-(2-Furan-2-yl-1-méthyl-1*H*-benzimidazol-5-yl)-3-(2-hydroxy-éthyl)]-3,6-dihydro-2*H*-1,3,4-thiadiazin-2-one,
- 3-(3-Amino-propyl)-5-(2-furan-2-yl-1-méthyl-1*H*-benzimidazol-5-yl)]-3,6-dihydro-2*H*-1,3,4-thiadiazin-2-one,
- 3-(2-Amino-éthyl)-5-[2-(3-fluoro-phényl)-1-méthyl-1*H*-benzimidazol-5-yl]-6-méthyl]-3,6-dihydro-2*H*-1,3,4-thiadiazin-2-one,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-2-phényl-acetamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-4-méthyl-benzamide,
- Cyclopropanecarboxylic acid (2-{5-[2-(3-fluoro-phényl)-1-méthyl-1*H-*benzimidazol-5-yl]-6-méthyl-2-oxo-6*H*-[1,3,4]thiadiazin-3-yl}-éthyl)-amide,
- Cyclopentanecarboxylic acid (2-{5-[2-(3-fluoro-phényl)-1-méthyl-1*H-*benzimidazol-5-yl]-6-méthyl-2-oxo-6*H*-[1,3,4]thiadiazin-3-yl}-éthyl)-amide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-2-méthoxy-acetamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-nicotinamide,
- *N*-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-benzamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-acétamide,
- N-(2-{5-[2-(3-Fluoro-phényl)-1-méthyl-1*H*-benzimidazol-5-yl]-6-méthyl-2-oxo-6*H*-[1,3,4]thiadiazin-3-yl}-éthyl)-isobutyramide,
- (S)-5-{2-[4-((S)-Méthanesulfinyl)-3-méthoxy-phényl]-1*H*-benzimidazol-5-yl}-6-méthyl]-3,6-dihydro-2*H*-1,3,4-thiadiazin-2-one,
- (R)-5-{2-[4-((S)-Méthanesulfinyl)-3-méthoxy-phényl]-1*H*-benzimidazol-5-yl}-6-méthyl]-3,6-dihydro-2*H*-1,3,4-thiadiazin-2-one,
- (S)-5-{2-[4-((R)-Méthanesulfinyl)-3-méthoxy-phényl]-1*H*-benzimidazol-5-yl}-6-méthyl]-3,6-dihydro-2*H*-1,3,4-thiadiazin-2-one,
- (R)-5-{2-[4-((R)-Méthanesulfinyl)-3-méthoxy-phényl]-1*H*-benzimidazol-5-yl}-6-méthyl]-3,6-dihydro-2*H*-1,3,4-thiadiazin-2-one,
- 1-(4-Acetyl-phényl)-3-(2-{5-[2-(3-fluoro-phényl)-1-méthyl-1*H*-benzimidazol-5-yl]-6-méthyl-2-oxo-6*H*-[1,3,4]thiadiazin-3-yl}-éthyl)-urée,
- 1-(3-Fluoro-phényl)-3-(2-{5-[2-(3-fluoro-phényl)-1-méthyl-1*H*-benzimidazol-5-yl]-6-méthyl-2-oxo-6*H*-[1,3,4]thiadiazin-3-yl}-éthyl)-urée,
- 5-[1-Cyclopropylméthyl-2-(1-méthyl-1*H*-pyrrol-2-yl)-1*H*-benzimidazol-5-yl]-6-méthyl]-3,6-dihydro-2*H*-1,3,4-thiadiazin-2-one,
- 5-[1-Cyclopropylméthyl-2-(1-méthyl-1*H*-imidazol-2-yl)-1*H*-benzimidazol-5-yl]-6-méthyl]-3,6-dihydro-2*H*-1,3,4-thiadiazin-2-one,
- 5-(1-Cyclopropylméthyl-2-thiophé-3-yl-1*H*-benzimidazol-5-yl)-6-méthyl]-3,6-dihydro-2*H*-1,3,4-thiadiazin-2-one,
- 5-[1-Cyclopropylméthyl-2-(2-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(4-méthoxy-phényl)-1*H*-benzimidazol-5-yl]-6-méthyl]-3,6-dihydro-2*H*-1,3,4-thiadiazin-2-one,
- 5-[1-Cyclopropylméthyl-2-(3-hydroxy-4-méthoxy-phényl)-1*H*-benzimidazol-5-yl]-6-méthyl]-3,6-dihydro-2*H*-1,3,4-thiadiazin-2-one,
ou leurs formes tautomères, énantiomères, diastéréoisomères, épimères, ainsi que les sels pharmaceutiquement acceptables, et les formes cristallines, ou leurs mélanges.

7. Composés selon l'une quelconque des revendications précédentes choisis parmi:
- 5-[2-(3-Fluorophényl)-1-méthyl-1*H*-benzimidazol-5-yl]-3-isopropyl-6-méthyl]-3,6-dihydro-2*H*-1,3,4-thiadiazin-2-one,
- 5-[2-(3-Fluorophényl)-1-méthyl-1*H*-benzimidazol-5-yl]-6-méthyl-3-propyl]-3,6-dihydro-2*H*-1,3,4-thiadiazin-2-one,
- 5-{2-[4-(1H-imidazol-1-yl)phényl]-1*H*-benzimidazol-5-yl}-6-méthyl-3,6-dihydro-2*H*-1,3,4-thiadiazin-2-one dichlorhydrate,
- 5-[1-Cycloheptyl-2-(3-méthoxy-phényl)-1*H*-benzimidazol-5-yl]-3-méthyl]-3,6-dihydro-2*H*-1,3,4-thiadiazin-2-one,
- 6-méthyl-5-{2-[3-(méthoxy)phényl]-1*H*-benzimidazol-5-yl}-3,6-dihydro-2*H-*1,3,4-thiadiazin-2-one,
- 5-{2-[3-méthoxy-4-(méthylsulfinyl)phényl]-1*H*-benzimidazol-5-yl}-6-méthyl-3,6-dihydro-2*H*-1,3,4-thiadiazin-2-one, chlorhydrate,
ou leurs formes tautomères, énantiomères, diastéréoisomères, épimères, ainsi que les formes libres ou les sels pharmaceutiquement acceptables, et les formes cristallines, ou leurs mélanges.

8. Procédé de préparation d'un composé de formule (I) dans laquelle R3 est différent d'un atome d'hydrogène selon l'une quelconque des revendications précédentes comprenant l'étape consistant à substituer le composé correspondant de formule (I) dans laquelle R3 est égal à un hydrogène, c'est-à-dire le composé de formule (F) ci-dessous: 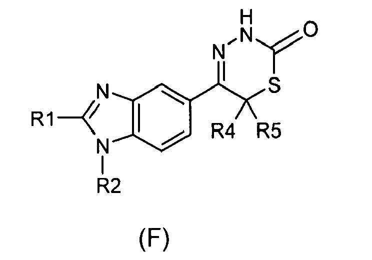 dans laquelle R1, R2, R4, R5 sont tels que définis en formule (I) au moyen d'un réactif approprié, selon la valeur du groupe R3 désiré, suivi éventuellement d'une ou plusieurs étape(s) de fonctionnalisation appropriée(s).

9. Procédé selon la revendication 8 tel que ladite étape de substitution correspond à : 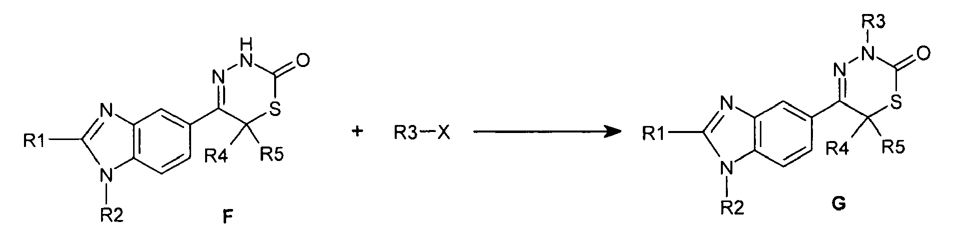 dans lesquels R1, R2, R4 et R5 sont tels que définis selon l'une quelconque des revendications 1 à 8, R3 est différent de H et X représente un atome d'halogène.

10. Procédé selon la revendication 8 tel que ladite étape de substitution correspond aux réactions suivantes : 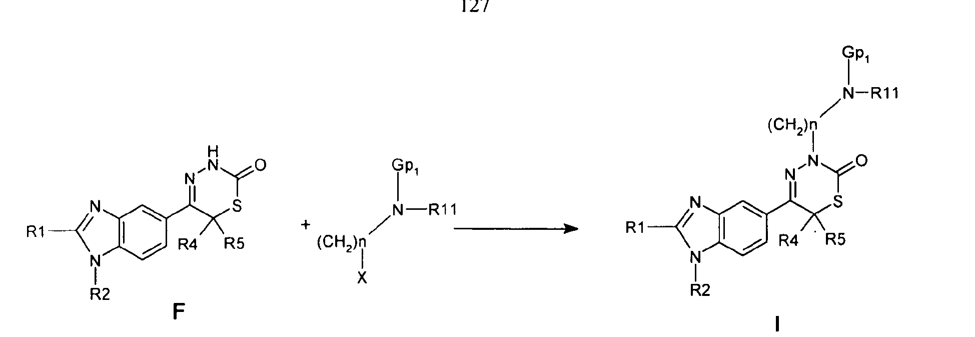 puis 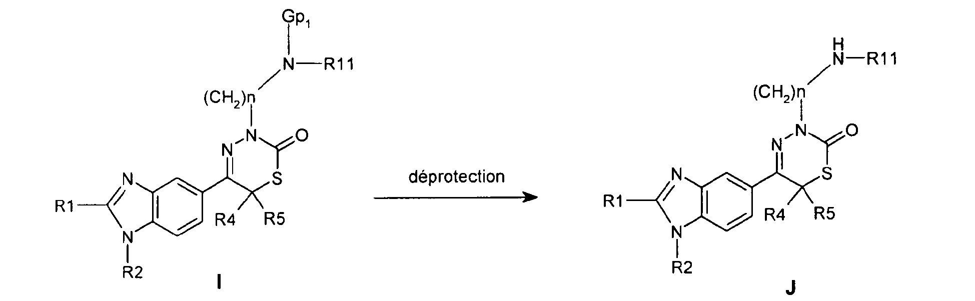 dans lesquels R1, R2, R4, R5 et R11 sont tels que définis selon l'une quelconque des revendications 1 à 7, X représente un atome d'halogène, n représente un entier compris entre 1 et 10 et où Gp1 est un groupe protecteur d'amines.

11. Procédé selon l'une quelconque des revendications 8 à 10 tel que ladite étape de fonctionnalisation correspond à : 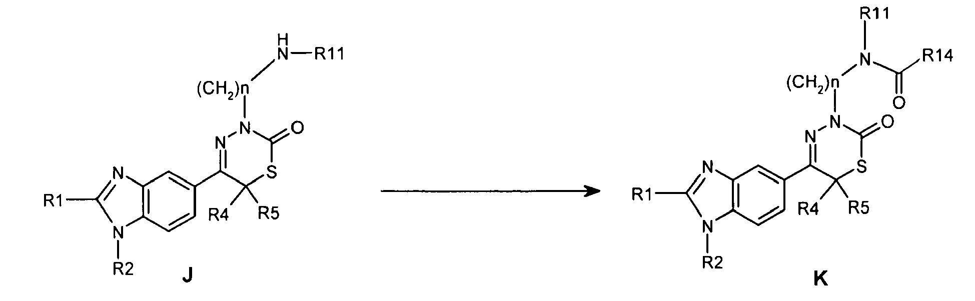 par action d'un halogénure d'acide convenablement choisi de formule
Hal-CO-R14
dans lesquels R1, R2, R4, R5, R11, R14 et n sont tels que définis selon l'une quelconque des revendications 1 à 7, 10 et Hal représente un atome d'halogène.

12. Procédé selon l'une quelconque des revendications 8 à 10 tel que ladite étape de fonctionnalisation correspond à : 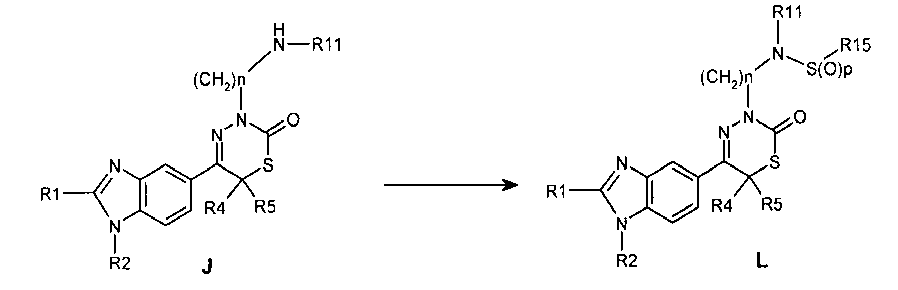 par action d'un halogénure de sulfonyle convenablement choisi de formule
Hal-S(O)ₚ-R15
dans lesquels R1, R2, R4, R5, R11, R14, R15 et p sont tels que définis selon l'une quelconque des revendications 1 à 7 et Hal représente un atome d'halogène.

13. Procédé selon l'une quelconque des revendications 8 à 10 tel que ladite étape de fonctionnalisation correspond à : 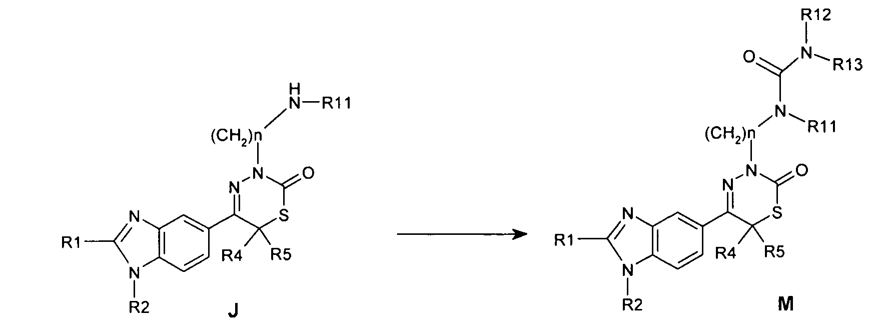 dans lesquels R1, R2, R4, R5, R11, R12, R13 et n sont tels que définis selon l'une quelconque des revendications 1 à 7, 10, au moyen d'un isothiocyanate correspondant de formule
R13-N=C=O
lorsque R12 est un hydrogène et R13 est différent d'un hydrogène.

14. Procédé selon l'une quelconque des revendications 8 à 10 tel que ladite étape de fonctionnalisation correspond à : 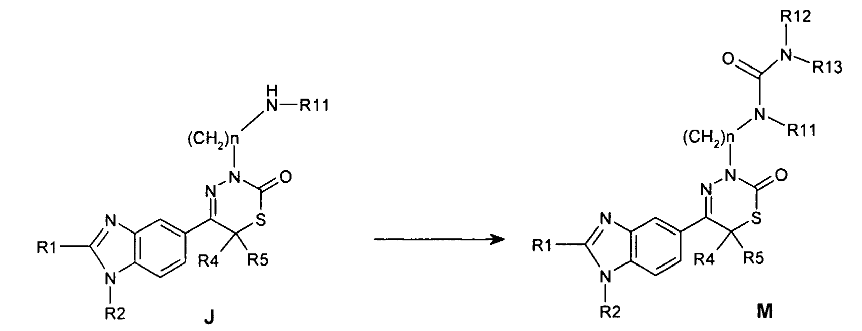 dans lesquels R1, R2, R4, R5, R11, R12, R13 et n sont tels que définis selon l'une quelconque des revendications 1 à 7, 10,
au moyen de carbonyldiimidazole puis d'une amine NHR12R13 correspondante lorsque R12 et R13 sont tous les deux différents d'un atome d'hydrogène.

15. Procédé selon l'une quelconque des revendications 8 à 10 tel que ladite étape de fonctionnalisation correspond à : 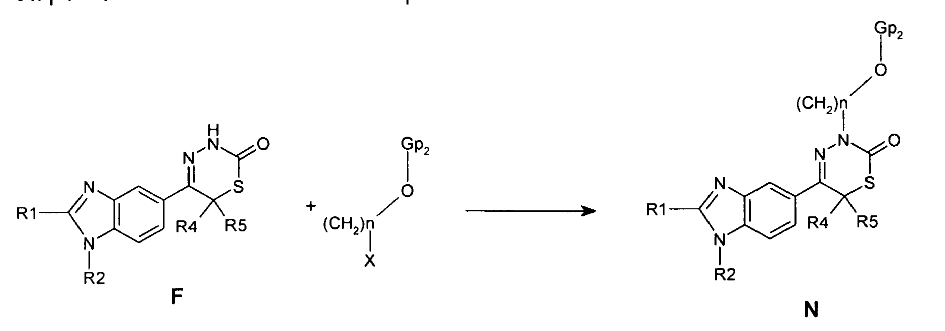 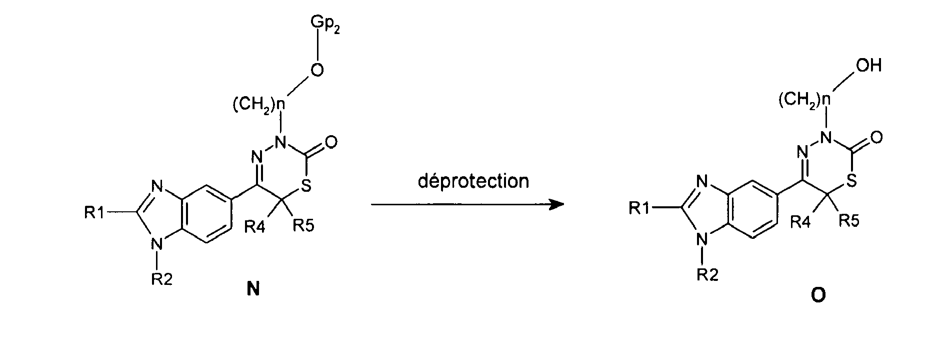 dans lesquels R1, R2, R4, R5, n sont tels que définis selon l'une quelconque des revendications 1 à 7, 10, X représente un atome d'halogène, et Gp2 représente un groupe protecteur d'alcool.

16. Procédé selon l'une quelconque des revendications 8 à 15, tel que le composé de formule (F) est obtenu selon l'étape 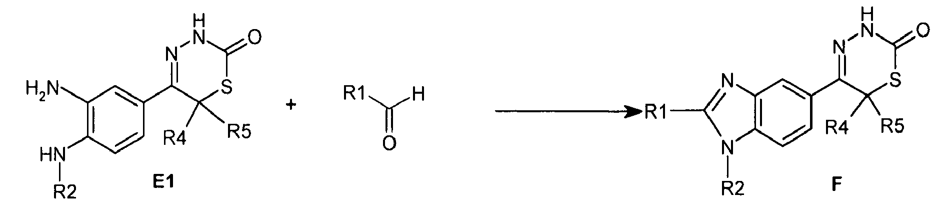 dans lesquels R1, R2, R4 et R5 sont tels que définis selon l'une quelconque des revendications 1 à 7.

17. Procédé selon l'une quelconque des revendications 8 à 16, tel que le composé de formule (F) est obtenu selon l'étape 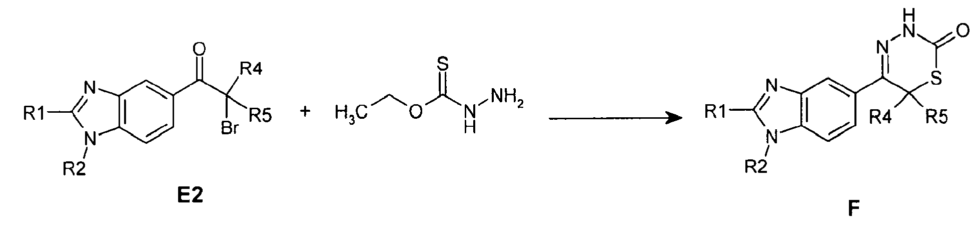 dans lesquels R1, R2, R4 et R5 sont tels que définis selon l'une quelconque des revendications 1 à 7.

18. Procédé selon l'une quelconque des revendications 8 à 17, comprenant en outre l'étape consistant à isoler le produit obtenu.

19. Composition pharmaceutique comprenant au moins un composé de formule générale (I) selon l'une quelconque des revendications 1 à 7.

20. Utilisation d'un composé de formule générale (I) selon l'une quelconque des revendications 1 à 7 pour la préparation d'un médicament destiné à prévenir ou traiter les pathologies associées au syndrôme d'insulino résistance.

21. Utilisation selon la revendication 20 pour le traitement et/ou la prévention du diabète.

22. Utilisation selon la revendication 21 telle que le diabète est le diabète non insulinodépendant (diabète de type II ou NIDDM).

23. Utilisation selon la revendication 20 pour la prévention et/ou le traitement de la dyslipidémie, l'obésité, l'hypertension artérielle, l'athérosclérose, les rétinopathies et les neuropathies, l'accident ischémique du myocarde, l'hypercholestérolémie, l'hyperlipidémie, la néphropathie.

24. Utilisation selon la revendication 20 pour la prévention et/ou le traitement de la dyslipidémie, l'hyperlipidémie, l'hypercholestérolémie.

25. Utilisation selon la revendication 20 pour la prévention et/ou le traitement de l'obésité.

26. Utilisation selon la revendication 20 pour la prévention et/ou le traitement de la rétinopathie, neuropathie et néphropathie.

27. Utilisation selon la revendication 20 pour la prévention et/ou le traitement de l'hypertension artérielle, l'accident ischémique du myocarde.

28. Utilisation d'un composé de formule générale (I) selon l'une quelconque des revendications 1 à 7 pour la fabrication d'un médicament pour inhiber ou limiter la production hépatique de glucose.

29. Composé selon l'une quelconque des revendications précédentes pour prévenir ou traiter le diabète.
